# EUROPEAN PATENT APPLICATION

(11) **EP 3 889 153 A1**
(43) Date of publication of application: **06.10.2021**
(21) Application number: 19890755.2
(22) Date of filing: 29.11.2019
(51) Int. Cl.: C07D 471/04, A61K 35/00, A61P 35/00

(54) **PYRIMIDINE AND FIVE-MEMBERED NITROGEN HETEROCYCLE DERIVATIVE, PREPARATION METHOD THEREFOR, AND MEDICAL USES THEREOF**

(30) Priority: 30.11.2018 CN 201811452514; 28.06.2019 CN 201910577816
(71) Applicant: Tuojie Biotech (Shanghai) Co., Ltd., Shanghai 201203 (CN)
(72) Inventor: ZOU, Hao, Shanghai 201203 (CN); LI, Zhengtao, Shanghai 201203 (CN); WANG, Yuanhao, Shanghai 201203 (CN); YU, Jian, Shanghai 201203 (CN); ZHU, Wei, Shanghai 201203 (CN)
(74) Representative: Viganò, Elena
(86) International application number: PCT/CN2019/121844
(87) International publication number: WO 2020/108590

(57) **Abstract**

The present invention relates to a pyrimidine and a five-membered nitrogen heterocycle derivative, a preparation method therefor, and the medical uses thereof. Particularly, the present invention relates to a pyrimidine and a five-membered nitrogen heterocycle derivative represented by the general formula (I), a preparation method thereof, a pharmaceutical composition containing the derivative, and the uses thereof as a SHP2 inhibitor for use in the prevention and/or treatment of tumor or cancer, wherein each substituent in the general formula (I) is as defined in the description.

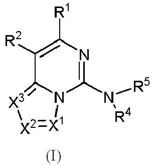

## Description

### FIELD OF THE INVENTION

The present invention belongs to the field of medicine, and relates to a pyrimido five-membered nitrogen-containing heterocycle derivative, a method for preparing the same, and a use thereof in medicine. In particular, the present invention relates to a pyrimido five-membered nitrogen-containing heterocycle derivative of formula (I), a method for preparing the same, a pharmaceutical composition comprising the same, a use thereof as a SHP2 inhibitor, and a use thereof in the preparation of a medicament for preventing and/or treating tumor or cancer.

### BACKGROUND OF THE INVENTION

Src homology domain 2 containing tyrosine phosphatase-2 (SHP2) is an evolutionarily conserved non-receptor protein tyrosine phosphatase (PTP) encoded by the PTPN11 gene. SHP2 is mainly composed of two SH2 domains (N-SH2 and C-SH2) and one PTP catalytic domain. SHP2 is widely expressed in various human tissues, and plays an important role in maintaining tissue development, cell homeostasis and the like. SHP2 is related to signals through the Ras-mitogen-activated protein kinase, JAK-STAT or phosphoinositide 3-kinase AKT pathway. Mutations in the PTPN11 gene and subsequent mutations in SHP2 have been identified in a variety of human diseases, such as Noonan syndrome, Leopard syndrome, juvenile myelomonocytic leukemia, neuroblastoma, melanoma, acute myeloid leukemia, breast cancer, lung cancer, and colon cancer (same as Claim19). Therefore, SHP2 represents a highly attractive target for the development of new therapies for treating various diseases.

Published patent applications related to the SHP2 target include WO2018136264A, WO2015003094A, WO2018160731A, WO2018130928A1, WO2018136265A, WO2018172984A, WO2018081091, WO2016203405, WO2017211303A, WO2018013597A and the like. At present, the SHP2 inhibitor TNO155 developed by Novartis and the SHP2 inhibitor JAB-3068 developed by JACOBIO are both in the phase I clinical trial, and there is no marketed product on this target. Therefore, it is still necessary to continue to develop novel SHP2 inhibitors with higher effcacy in order to provide patients with new and effective anti-cancer drugs.

### SUMMARY OF THE INVENTION

The present invention provides a compound of formula (I) or a tautomer, mesomer, racemate, enantiomer, diastereomer, atropisomer thereof, or mixture thereof, or a pharmaceutically acceptable salt thereof, 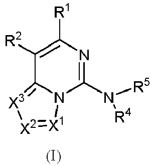 wherein:
R¹ is selected from the group consisting of hydrogen atom, deuterium atom, hydroxy, cyano, nitro, halogen, carboxy, alkyl, alkoxy, haloalkyl, haloalkoxy, amino, alkenyl and hydroxyalkyl;
R² is 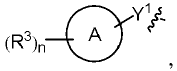
Y¹ is selected from the group consisting of -S-, -NH-, -S(O)₂-, -S(O)₂-NH-, -C(=CH₂)-, -S(O)- and a bond;
ring A is selected from the group consisting of cycloalkyl, heterocyclyl, aryl and heteroaryl, wherein the cycloalkyl, heterocyclyl, aryl and heteroaryl are each independently a 5 to 12 membered monocycle or polycycle;
each R³ is independently selected from the group consisting of hydrogen atom, deuterium atom, halogen, alkyl, alkoxy, cyano, amino, nitro, carboxy, hydroxy, hydroxyalkyl, C₃₋₈ cycloalkyl, 3 to 12 membered heterocyclyl, aryl, heteroaryl, C₂₋₆ alkenyl, C₄₋₈ cycloalkenyl, C₂₋₆ alkynyl, -CHR^{a}R^{b}, -NR^{a}R^{b}, -alkenyl-NR^{a}R^{b}, -alkenyl-O-R^{a}, -alkenyl-C(O)₂R^{a}, -alkenyl-R^{a}, -alkenyl-CO-NR^{a}R^{b}, -alkenyl-NR^{a}-CO-NR^{a}R^{b}, -alkenyl-NR^{a}-C(O)R^{b}, -C(O)NR^{a}R^{b}, -C(O)R^{a}, -CO-alkenyl-NR^{a}R^{b}, -NR^{a} C(O)R^{b}, -C(O)₂R^{a}, -O-alkenyl-CO-OR^{a}, -O-alkenyl-CO-NR^{a}R^{b}, -O-alkenyl-NR^{a}R^{b}, -OR^{a}, -SR^{a}, -NR^{a}-CO-NR^{a}R^{b}, -NR^{a}-alkenyl-NR^{a}R^{b}, -NR^{a}-alkenyl-R^{b}, -NR^{a}S(O)₂R^{b}, -NR^{a}S(O)R^{b}, -NR^{a}S(O)₂NR^{a}R^{b}, -NR^{a}S(O)NR^{a}R^{b}, -S(O)₂ NR^{a}R^{b}, -S(O)NR^{a}R^{b}, -S(O)R^{a}, -S(O)₂R^{a}, -P(O)R^{a}R^{b}, -N(S(O)R^{a}R^{b}) and -S(O)(NR^{a})R^{b}, wherein the alkyl, alkoxy, aryl and heteroaryl are each independently optionally further substituted by one or more substituents selected from the group consisting of halogen, hydrogen atom, deuterium atom, cyano, amino, nitro, carboxy, hydroxy, hydroxyalkyl, alkyl, alkoxy, haloalkyl and haloalkoxy;
n is selected from the group consisting of 0, 1, 2, 3, 4 and 5;
X¹, X² and X³ are each independently selected from the group consisting of CR^{c} and N, wherein at least one of them is N, and preferably X¹ is CR^{c};
R^{c} is selected from the group consisting of hydrogen atom, deuterium atom, C₁₋₆ alkyl, C₁₋₆ alkoxy, C₁₋₆ alkylthio, amino, nitro, hydroxy, carbonyl, carboxy, halogen and cyano;
R⁴ is selected from the group consisting of hydrogen, C₁₋₆ alkyl, 3 to 12 membered monocyclic heterocyclyl or polycyclic heterocyclyl and C₃₋₈ cycloalkyl, wherein the alkyl, heterocyclyl and cycloalkyl are each independently optionally substituted by one or more substituents selected from the group consisting of halogen, hydroxy, C₁₋₃ alkyl, amino, alkylamino, hydroxyalkyl and alkoxy;
R⁵ is selected from the group consisting of hydrogen, hydroxy, C₁₋₆ alkyl and C₃₋₈ cycloalkyl, wherein the alkyl or cycloalkyl is optionally substituted by one or more amino; or
R⁴ and R⁵ together with the nitrogen atom to which they are attached form a 3 to 12 membered monocyclic heterocycle or polycyclic heterocycle, wherein the monocyclic heterocycle or polycyclic heterocycle is optionally substituted by one or more substituents selected from the group consisting of halogen, hydroxy, halogen-substituted or unsubstituted C₁₋₆ alkyl, amino, alkoxy, hydroxyalkyl, aryl, heteroaryl, heterocyclyl, alkylamino, halogen-substituted or unsubstituted alkoxy and -NR^{a}S(O)NR^{a}R^{b}, and the polycyclic heterocycle includes, but is not limited to, bridged heterocycle and spiro heterocycle;
exemplary rings formed by R⁴ and R⁵ together with the nitrogen atom to which they are attached include, but are not limited to: 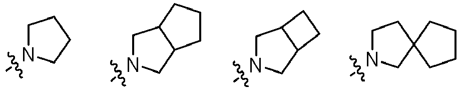 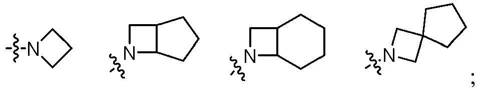
or R⁴ and R⁵ together with the nitrogen atom to which they are attached form a structure of 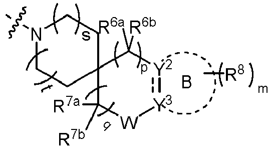
wherein s and t are each independently selected from the group consisting of 0 and 1;
R^{6a} and R^{6b} are each independently selected from the group consisting of hydrogen atom, deuterium atom, fluorine atom, amino, hydroxy, cyano, nitro, carboxy, fluorine-substituted or unsubstituted alkyl and fluorine-substituted or unsubstituted alkoxy; or R^{6a} and R^{6b} together with the carbon atom to which they are attached form a CO, C=NH, C=N-OH, 3 to 12 membered heterocyclyl or C₃₋₈ cycloalkyl;
p is selected from the group consisting of 0, 1, 2, 3 and 4;
R^{7a} and R^{7b} are each independently selected from the group consisting of hydrogen atom, deuterium atom, fluorine atom, amino, hydroxy, cyano, nitro, carboxy, fluorine-substituted or unsubstituted alkyl, fluorine-substituted or unsubstituted alkoxy and -NR^{a}S(O)NR^{a}R^{b};
or R^{7a} and R^{7b} together with the carbon atom to which they are attached form a 3 to 12 membered heterocyclyl, 5 to 10 membered heteroaryl, C₃₋₈ cycloalkyl and C=NR^{7c}, wherein the rings are optionally substituted;
R^{7c} is selected from the group consisting of hydrogen atom, deuterium atom and C₁₋₆ alkyl;
q is selected from the group consisting of 0, 1, 2, 3 and 4;
W is absent or is selected from the group consisting of -O-, -S- and -NR^{w-};
R^{w} is selected from the group consisting of hydrogen atom, halogen, amino, hydroxy, cyano, nitro, carboxy, -C(O)C₁₋₆ alkyl, -C(O)₂C₁₋₆ alkyl, C₁₋₆ alkyl ether, halogen-substituted or unsubstituted C₁₋₆ alkyl and halogen-substituted or unsubstituted C₁₋₆ alkoxy;
ring B is absent or is a 3 to 10 membered ring;
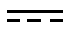 is a single bond or double bond;
when ring B is absent, then Y² is CR^{2a}R^{2b}, NR^{2a} or O, Y³ is CR^{3a}R^{3b}, NR^{3a} or O;
when ring B is a 3 to 10 membered ring, then
   1) Y² is CR^{2a} or N, Y³ is CR^{3a} or N, 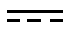 is a single bond; or
   2) Y² is C and Y³ is C, 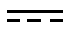 is a double bond;
R^{2a}, R^{2b}, R^{3a} and R^{3b} are each independently selected from the group consisting of hydrogen atom, deuterium atom, halogen, cyano, amino, nitro, carboxy, hydroxy, hydroxyalkyl, C₃₋₈ cycloalkyl, 3 to 12 membered heterocyclyl, aryl, heteroaryl, C₂₋₆ alkenyl, C₄₋₈ cycloalkenyl, C₂₋₆ alkynyl, -NR^{a}R^{b}, -alkenyl-NR^{a}R^{b}, -alkenyl-O-R^{a}, -alkenyl-C(O)₂R^{a}, -alkenyl-R^{a}, -alkenyl-CO-NR^{a}R^{b}, -alkenyl-NR^{a}-CO-NR^{a}R^{b}, -alkenyl-NR^{a}-C(O)R^{b}, -C(O)NR^{a}R^{b}, -C(O)R^{a}, -CO-alkenyl-NR^{a}R^{b}, -NR^{a}C(O)R^{b}, -C(O)2R^{a}, -O-alkenyl-CO-OR^{a}, -O-alkenyl-CO-NR^{a}R^{b}, -O-alkenyl-NR^{a}R^{b}, -OR^{a}, -SR^{a}, -NR^{a}-CO-NR^{a}R^{b}, -NR^{a}-alkenyl-NR^{a}R^{b}, -NR^{a}-alkenyl-R^{b}, -NR^{a}S(O)₂R^{b}, -NR^{a}S(O)R^{b}, -NR^{a}S(O)₂NR^{a}R^{b}, -NR^{a}S(O)NR^{a}R^{b}, -S(O)₂NR^{a}R^{b}, -S(O)NR^{a}R^{b}, -S(O)R^{a}, -S(O)₂R^{a}, -P(O)R^{a}R^{b}, -N(S(O)R^{a}R^{b}) and -S(O)(NR^{a})R^{b}, wherein the aryl and heteroaryl are each independently optionally further substituted by one or more substituents selected from the group consisting of halogen, hydrogen atom, deuterium atom, cyano, amino, nitro, carboxy, hydroxy, hydroxyalkyl, alkyl, alkoxy, haloalkyl and haloalkoxy;
R^{a} and R^{b} are each independently selected from the group consisting of hydrogen, deuterium atom, halogen, amino, hydroxy, cyano, nitro, carboxy, alkyl, alkoxy, haloalkyl, haloalkoxy, C₃₋₈ cycloalkyl, 5 to 10 membered heteroaryl and aryl, wherein the aryl and heteroaryl are each independently optionally further substituted by one or more substituents selected from the group consisting of halogen, hydrogen atom, deuterium atom, cyano, amino, nitro, carboxy, hydroxy, hydroxyalkyl, alkyl, alkoxy, haloalkyl and haloalkoxy;
m is selected from the group consisting of 0, 1, 2, 3 and 4; and
each R⁸ is independently selected from the group consisting of hydrogen atom, deuterium atom, halogen, amino, hydroxy, cyano, nitro, carboxy, C₁₋₆ alkyl and C₁₋₆ alkoxy;
or two R⁸ are attached together to form a phenyl, 5 membered heteroaryl, 6 membered heteroaryl or 3 to 6 membered heterocyclyl, wherein each ring is optionally substituted by one or more substituents selected from the group consisting of halogen, amino, hydroxy, cyano, nitro and C₁₋₆ alkyl.

In a preferred embodiment of the present invention, in the compound of formula (I) or the tautomer, mesomer, racemate, enantiomer, diastereomer, atropisomer thereof, or mixture thereof, or the pharmaceutically acceptable salt thereof, R⁴ and R⁵ together with the nitrogen atom to which they are attached form a structure of 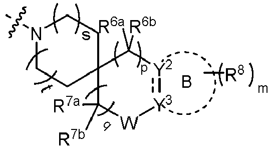
wherein s and t are each independently selected from the group consisting of 0 and 1;
R^{6a} and R^{6b} are each independently selected from the group consisting of hydrogen atom, deuterium atom, C₁₋₆ alkyl and C₁₋₆ alkoxy; or R^{6a} and R^{6b} together with the carbon atom to which they are attached form a 3 to 12 membered heterocyclyl or C₃₋₈ cycloalkyl;
p is selected from the group consisting of 0, 1 and 2;
R^{7a} and R^{7b} are each independently selected from the group consisting of hydrogen atom, deuterium atom, amino, C₁₋₆ alkyl and -NR^{a}S(O)NR^{a}R^{b}, wherein R^{a} and R^{b} are as defined in the above formula (I);
q is 1 or 2;
W is absent;
ring B is absent or is a 3 to 10 membered ring;
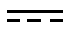 is a single bond or double bond;
when ring B is absent, then Y² is CR^{2a}R^{2b} or O, Y³ is CR^{3a}R^{3b}; or
when ring B is a 3 to 10 membered ring, then
Y² is CR^{2a} or N, Y³ is CR^{3a} or N, 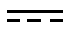 is a single bond; or
Y² is C and Y³ is C, 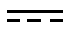 is a double bond;
R^{2a}, R^{2b} and R^{3a} are each independently selected from the group consisting of hydrogen atom, deuterium atom and C₁₋₆ alkyl;
m is selected from the group consisting of 0, 1, 2, 3 and 4; and
each R⁸ is independently selected from the group consisting of hydrogen atom, deuterium atom, halogen, amino, hydroxy, cyano, nitro, carboxy, C₁₋₆ alkyl and C₁₋₆ alkoxy;
or two R⁸ are attached together to form a phenyl, 5 membered heteroaryl, 6 membered heteroaryl or 3 to 6 membered heterocyclyl, wherein each ring is optionally substituted by one or more substituents selected from the group consisting of halogen, amino, hydroxy, cyano, nitro and C₁₋₆ alkyl.

In a preferred embodiment of the present invention, in the compound of formula (I) or the tautomer, mesomer, racemate, enantiomer, diastereomer, atropisomer thereof, or mixture thereof, or the pharmaceutically acceptable salt thereof, R⁴ and R⁵ together with the nitrogen atom to which they are attached form a structure of 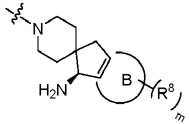 wherein:
ring B is selected from the group consisting of benzene ring, 5 membered heteroaromatic ring and 6 membered heteroaromatic ring, preferably a benzene ring or pyridine ring;
each R⁸ is independently selected from the group consisting of hydrogen atom, deuterium atom, halogen, cyano, C₁₋₆ alkyl and C₁₋₆ alkoxy; and
m is selected from the group consisting of 0, 1, 2, 3 and 4.

In a preferred embodiment of the present invention, in the compound of formula (I) or the tautomer, mesomer, racemate, enantiomer, diastereomer, atropisomer thereof, or mixture thereof, or the pharmaceutically acceptable salt thereof, R⁴ and R⁵ together with the nitrogen atom to which they are attached form a structure of 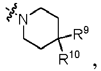
wherein R⁹ and R¹⁰ are each independently selected from the group consisting of hydrogen atom, deuterium atom, hydroxy, C₁₋₆ alkyl, C₁₋₆ alkoxy, halogen, C₁₋₆ hydroxyalkyl, aryl, heteroaryl, heterocyclyl, amino, C₁₋₆ alkylamino and -NR^{a}S(O)NR^{a}R^{b}, preferably selected from the group consisting of hydrogen atom, deuterium atom, C₁₋₆ alkyl, amino and -NR^{a}S(O)NR^{a}R^{b}; or
R^{a} and R^{b} are as defined in the above formula (I).

In a preferred embodiment of the present invention, in the compound of formula (I) or the tautomer, mesomer, racemate, enantiomer, diastereomer, atropisomer thereof, or mixture thereof, or the pharmaceutically acceptable salt thereof,
Y¹ is -S- or a bond;
ring A is an aryl or heteroaryl;
each R³ is independently selected from the group consisting of hydrogen atom, deuterium atom, halogen, C₁₋₆ alkyl, haloC₁₋₆ alkyl, haloC₁₋₆ alkoxy, C₁₋₆ alkoxy, cyano, amino, nitro, carboxy, hydroxy and phenyl, wherein the phenyl is optionally further substituted by one or more substituents selected from the group consisting of halogen, hydrogen atom, deuterium atom, cyano, amino, nitro, carboxy, hydroxy, hydroxyalkyl, alkyl, alkoxy, haloalkyl and haloalkoxy; each R³ is preferably selected from the group consisting of hydrogen atom, deuterium atom, halogen, haloC₁₋₆ alkyl, C₁₋₆ alkyl, C₁₋₆ alkoxy, haloC₁₋₆ alkoxy and phenyl, wherein the phenyl is optionally further substituted by one or more substituents selected from the group consisting of halogen, hydrogen atom, deuterium atom, cyano, amino, nitro, carboxy, hydroxy, hydroxyalkyl, alkyl, alkoxy, haloalkyl and haloalkoxy; and
n is selected from the group consisting of 0, 1, 2, 3, 4 and 5.

In a preferred embodiment of the present invention, in the compound of formula (I) or the tautomer, mesomer, racemate, enantiomer, diastereomer, atropisomer thereof, or mixture thereof, or the pharmaceutically acceptable salt thereof, X¹, X² and X³ are each independently selected from the group consisting of CR^{c} and N, wherein at least one of them is N, preferably X¹ is CR^{c}, and R^{c} is a hydrogen atom.

In a preferred embodiment of the present invention, in the compound of formula (I) or the tautomer, mesomer, racemate, enantiomer, diastereomer, atropisomer thereof, or mixture thereof, or the pharmaceutically acceptable salt thereof, X¹ and X² are both CR^{c} and X³ is N, or X¹ is CR^{c} and X² and X³ are both N, and R^{c} is a hydrogen atom.

In a preferred embodiment of the present invention, in the compound of formula (I) or the tautomer, mesomer, racemate, enantiomer, diastereomer, atropisomer thereof, or mixture thereof, or the pharmaceutically acceptable salt thereof, R¹ is selected from the group consisting of hydrogen atom, deuterium atom, C₁₋₆ alkyl, C₁₋₆ alkoxy, amino and hydroxy.

In a preferred embodiment of the present invention, in the compound of formula (I) or the tautomer, mesomer, racemate, enantiomer, diastereomer, atropisomer thereof, or mixture thereof, or the pharmaceutically acceptable salt thereof,

R¹ is selected from the group consisting of hydrogen atom, deuterium atom, C₁₋₆ alkyl and amino;
Y¹ is -S- or a bond;
ring A is an aryl or heteroaryl;
each R³ is independently selected from the group consisting of hydrogen atom, deuterium atom, halogen, haloC₁₋₆ alkyl, C₁₋₆ alkyl, C₁₋₆ alkoxy, haloC₁₋₆ alkoxy and substituted phenyl;
n is selected from the group consisting of 0, 1, 2, 3, 4 and 5;
X¹, X² and X³ are each independently selected from the group consisting of CR^{c} and N, wherein at least one of them is N, preferably X¹ is CR^{c}, and R^{c} is a hydrogen atom;
R⁴ and R⁵ together with the nitrogen atom to which they are attached form a structure of 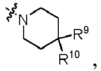 and
R⁹ and R¹⁰ are each independently selected from the group consisting of hydrogen atom, deuterium atom, C₁₋₆ alkyl, amino and -NR^{a}S(O)NR^{a}R^{b}, wherein R^{a} and R^{b} are as defined in the above formula (I).

In a preferred embodiment of the present invention, in the compound of formula (I) or the tautomer, mesomer, racemate, enantiomer, diastereomer, atropisomer thereof, or mixture thereof, or the pharmaceutically acceptable salt thereof,
R¹ is selected from the group consisting of hydrogen atom, deuterium atom, C₁₋₆ alkyl and amino;
Y¹ is -S- or a bond;
ring A is an aryl or heteroaryl;
each R³ is independently selected from the group consisting of hydrogen atom, deuterium atom, halogen, haloC₁₋₆ alkyl, C₁₋₆ alkyl, C₁₋₆ alkoxy, haloC₁₋₆ alkoxy and substituted phenyl;
n is selected from the group consisting of 0, 1, 2, 3, 4 and 5;
X¹, X² and X³ are each independently selected from the group consisting of CR^{c} and N, wherein at least one of them is N, preferably X¹ is CR^{c}, and R^{c} is a hydrogen atom;
R^{6a} and R^{6b} are each independently selected from the group consisting of hydrogen atom, deuterium atom, C₁₋₆ alkyl and C₁₋₆ alkoxy; or R^{6a} and R^{6b} together with the carbon atom to which they are attached form a 3 to 12 membered heterocyclyl or C₃₋₈ cycloalkyl;
p is 1 or 2;
R^{7a} and R^{7b} are each independently selected from the group consisting of hydrogen atom, deuterium atom, amino, C₁₋₆ alkyl and -NR^{a}S(O)NR^{a}R^{b}, wherein R^{a} and R^{b} are as defined in the above formula (I);
q is 1 or 2;
W is absent;
ring B is absent, Y² is CR^{2a}R^{2b} or O, Y³ is CR^{3a}R^{3b}; and
R^{2a}, R^{2b}, R^{3a} and R^{3b} are each independently selected from the group consisting of hydrogen atom, deuterium atom and C₁₋₆ alkyl.
In a preferred embodiment of the present invention, in the compound of formula (I) or the tautomer, mesomer, racemate, enantiomer, diastereomer, atropisomer thereof, or mixture thereof, or the pharmaceutically acceptable salt thereof,
R¹ is selected from the group consisting of hydrogen atom, deuterium atom, C₁₋₆ alkyl and amino;
Y¹ is -S- or a bond;
ring A is an aryl or heteroaryl;
each R³ is independently selected from the group consisting of hydrogen atom, deuterium atom, halogen, haloC₁₋₆ alkyl, C₁₋₆ alkyl, C₁₋₆ alkoxy, haloC₁₋₆ alkoxy and substituted phenyl;
n is selected from the group consisting of 0, 1, 2, 3, 4 and 5;
X¹, X² and X³ are each independently selected from the group consisting of CR^{c} and N, wherein at least one of them is N, preferably X¹ is CR^{c}, and R^{c} is a hydrogen atom;
R^{6a} and R^{6b} are each independently selected from the group consisting of hydrogen atom, deuterium atom, C₁₋₆ alkyl and C₁₋₆ alkoxy;
p is 1 or 2;
R^{7a} and R^{7b} are each independently selected from the group consisting of hydrogen atom, deuterium atom, amino, C₁₋₆ alkyl and -NR^{a}S(O)NR^{a}R^{b}, wherein R^{a} and R^{b} are as defined in the above formula (I);
q is 1 or 2;
W is absent;
ring B is selected from the group consisting of phenyl, 5 membered heteroaryl and 6 membered heteroaryl;
Y² is C and Y³ is C, 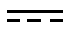 is a double bond;
each R⁸ is independently selected from the group consisting of hydrogen atom, deuterium atom, halogen, amino, hydroxy, cyano, nitro, carboxy, C₁₋₆ alkyl and C₁₋₆ alkoxy; and
m is selected from the group consisting of 0, 1, 2, 3 and 4.

In a preferred embodiment of the present invention, in the compound of formula (I) or the tautomer, mesomer, racemate, enantiomer, diastereomer, atropisomer thereof, or mixture thereof, or the pharmaceutically acceptable salt thereof, R⁴ and R⁵ together with the nitrogen atom to which they are attached form a structure of 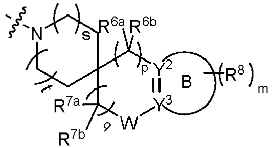
R¹ is selected from the group consisting of hydrogen atom, C₁₋₆ alkyl and amino;
Y¹ is -S- or a bond;
ring A is an aryl or heteroaryl, preferably phenyl or pyridyl;
each R³ is independently selected from the group consisting of hydrogen atom, deuterium atom, halogen, cyano, amino, haloC₁₋₆ alkyl, C₁₋₆ alkyl, C₁₋₆ alkoxy, haloC₁₋₆ alkoxy, C₁₋₆ alkylamino, haloC₁₋₆ alkylamino, C₃₋₈ cycloalkyl, 3 to 12 membered heterocyclyl, -OR^{a}, -CHR^{a}R^{b} and -NR^{a}R;
R^{a} and R^{b} are each independently selected from the group consisting of hydrogen, deuterium atom, hydroxy, C₁₋₆ alkyl and C₃₋₈ cycloalkyl, wherein the alkyl, heterocyclyl and cycloalkyl are each independently optionally further substituted by one or more substituents selected from the group consisting of halogen, deuterium atom, cyano, amino and hydroxy;
n is selected from the group consisting of 0, 1, 2, 3, 4 and 5;
X³ is N, X¹ and X² are each independently CR^{c}, and R^{c} is a hydrogen atom;
s and t are each independently selected from the group consisting of 0 and 1;
R^{6a} and R^{6b} are each independently selected from the group consisting of hydrogen atom, deuterium atom, C₁₋₆ alkyl and C₁₋₆ alkoxy;
p is 1;
R^{7a} and R^{7b} are each independently selected from the group consisting of hydrogen atom, deuterium atom, amino and C₁₋₆ alkyl;
q is 1;
W is absent;
ring B is selected from the group consisting of benzene ring, 5 membered heteroaromatic ring and 6 membered heteroaromatic ring, preferably a benzene ring or pyridine ring;
Y² is C and Y³ is C;
each R⁸ is independently selected from the group consisting of hydrogen atom, deuterium atom, halogen, amino, hydroxy, cyano, nitro, carboxy, C₁₋₆ alkyl and C₁₋₆ alkoxy; and
m is selected from the group consisting of 0, 1, 2, 3 and 4.

In a preferred embodiment of the present invention, the compound of formula (I) or the tautomer, mesomer, racemate, enantiomer, diastereomer, atropisomer thereof, or mixture thereof, or the pharmaceutically acceptable salt thereof is a compound of formula (II) or a tautomer, mesomer, racemate, enantiomer, diastereomer, atropisomer thereof, or mixture thereof, or a pharmaceutically acceptable salt thereof, 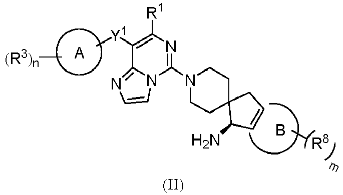 wherein:
R¹ is selected from the group consisting of hydrogen atom, C₁₋₆ alkyl, haloalkyl and amino;
Y¹ is -S- or a bond;
ring A is an aryl or heteroaryl, preferably phenyl or pyridyl;
each R³ is independently selected from the group consisting of hydrogen atom, deuterium atom, halogen, cyano, amino, C₁₋₆ alkyl, C₁₋₆ alkoxy, haloC₁₋₆ alkyl, haloC₁₋₆ alkoxy, C₃₋₈ cycloalkyl, 3 to 12 membered heterocyclyl, -OR^{a}, -CHR^{a}R^{b} and -NR^{a}R^{b};
R^{a} and R^{b} are each independently selected from the group consisting of hydrogen, deuterium atom, hydroxy, C₁₋₆ alkyl and C₃₋₈ cycloalkyl, wherein the alkyl, heterocyclyl and cycloalkyl are each independently optionally further substituted by one or more substituents selected from the group consisting of halogen, deuterium atom, cyano, amino and hydroxy;
ring B is selected from the group consisting of benzene ring, 5 membered heteroaromatic ring and 6 membered heteroaromatic ring, preferably a benzene ring or pyridine ring;
each R⁸ is independently selected from the group consisting of hydrogen atom, deuterium atom, halogen, cyano, C₁₋₆ alkyl and C₁₋₆ alkoxy;
m is selected from the group consisting of 0, 1, 2, 3 and 4; and
n is selected from the group consisting of 1, 2, 3 and 4.

In the present invention, when Y¹ is a bond, then the compound provided by the present invention may exist as a mixture of atropisomers due to the restriction of rotation around the bond, and the enantiomeric excess thereof is from 0 to 98%. When the compound is a pure atropisomer, the stereochemistry of each chiral center can be specified by aR or aS. These terms can also be used for a mixture that is rich in one atropisomer. The aR and aS atropisomers can be resolved by chiral chromatography.

A further description of atropisomerism and axial chirality can be found in Eliel, E.L. & Wilen, S. H. 'Stereochemistry of Organic Compounds' John Wiley and Sons, Inc. 1994.

Typical compounds of formula (I) of the present invention include, but are not limited to:

| Compound No. | Chemical structure and name |
|---|---|
| 1 | 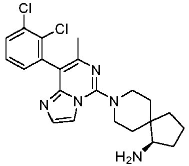 |
| | (R)-8-(8-(2,3-Dichlorophenyl)-7-methylimidazo[1,2-c]pyrimidin-5-yl)-8-a zaspiro[4.5]decan-1-amine |
| 2 | 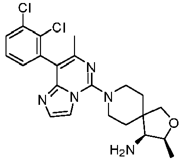 |
| | (3S,4S)-8-(8-(2,3-Dichlorophenyl)-7-methylimidazo[1,2-c]pyrimidin-5-yl) -3-methyl-2-oxa-8-azaspiro[4.5]decan-4-amine |
| 3 | 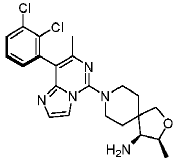 |
| | a(*R*)-(3S,4S)-8-(8-(2,3-Dichlorophenyl)-7-methylimidazo[1,2-c]pyrimidin-5-yl)-3-methyl-2-oxa-8-azaspiro[4.5]decan-4-amine atropisomer **1** |
| 4 | 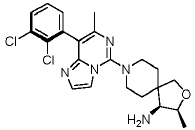 |
| | a(*S*)-(3S,4S)-8-(8-(2,3-Dichlorophenyl)-7-methylimidazo[1,2-c]pyrimidin-5-yl)-3-methyl-2-oxa-8-azaspiro[4.5]decan-4-amine atropisomer **2** |
| 5 | 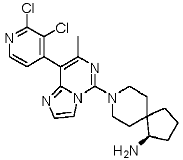 |
| | (R)-8-(8-(2,3-Dichloropyridin-4-yl)-7-methylimidazo[1,2-c]pyrimidin-5-yl )-8-azaspiro[4.5]decan-1-amine |
| 6 | 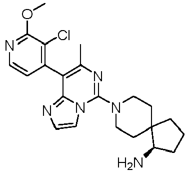 |
| | (R)-8-(8-(3-Chloro-2-methoxypyridin-4-yl)-7-methylimidazo[1,2-c]pyrimi din-5-yl)-8-azaspiro[4.5]decan-1-amine |
| 7 | 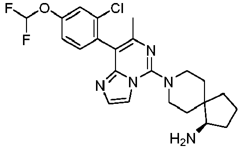 |
| | (R)-8-(8-(2-Chloro-4-(difluoromethoxy)phenyl)-7-methylimidazo[1,2-c]py rimidin-5-yl)-8-azaspiro[4.5]decan-1-amine |
| 8 | 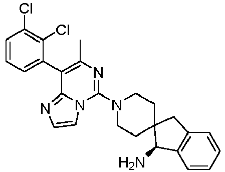 |
| | (S)-1'-(8-(2,3-Dichlorophenyl)-7-methylimidazo[1,2-c]pyrimidin-5-yl)-1,3 -dihydrospiro[indene-2,4'-piperidin]-1-amine |
| 9 | 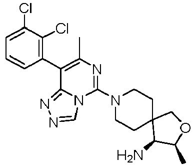 |
| | (3S,4S)-8-(8-(2,3-Dichlorophenyl)-7-methyl-[1,2,4]triazolo[4,3-c]pyrimidi n-5-yl)-3-methyl-2-oxa-8-azaspiro[4.5]decan-4-amine |
| 10 | 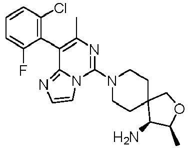 |
| | (3S,4S)-8-(8-(2-Chloro-6-fluorophenyl)-7-methylimidazo[1,2-c]pyrimidin-5-yl)-3-methyl-2-oxa-8-azaspiro[4.5]decan-4-amine |
| 11 | 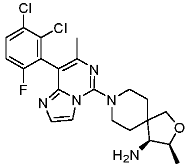 |
| | (3S,4S)-8-(8-(2,3-Dichloro-6-fluorophenyl)-7-methylimidazo[1,2-c]pyrimi din-5-yl)-3-methyl-2-oxa-8-azaspiro[4.5]decan-4-amine |
| 12 | 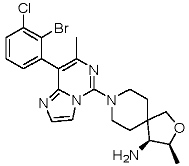 |
| | (3S,4S)-8-(8-(2-Bromo-3-chlorophenyl)-7-methylimidazo[1,2-c]pyrimidin-5-yl)-3-methyl-2-oxa-8-azaspiro[4.5]decan-4-amine |
| 13 | 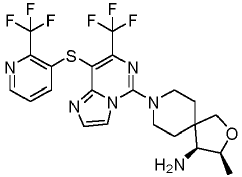 |
| | (3S,4S)-3-Methyl-8-(7-(trifluoromethyl)-8-((2-(trifluoromethyl)pyridin-3-y 1)thio)imidazo[1,2-c]pyrimidin-5-yl)-2-oxa-8-azaspiro[4.5]decan-4-amine |
| 14 | 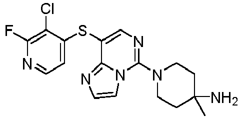 |
| | 1-(8-((3-Chloro-2-fluoropyridin-4-yl)thio)imidazo[1,2-c]pyrimidin-5-yl)-4 -meth ylpiperidin-4-amine |
| 15 | 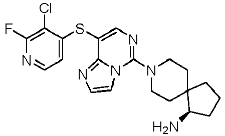 |
| | (R)-8-(8-((3-Chloro-2-fluoropyridin-4-yl)thio)imidazo[1,2-c]pyrimidi n-5-yl)-8-azaspiro[4.5]decan-1-amine |
| 16 | 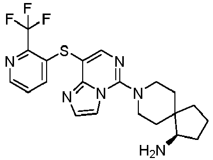 |
| | (R)-8-(8-((2-(Trifluoromethyl)pyridin-3-yl)thio)imidazo[1,2-c]pyrimidin-5 -yl)-8-azaspiro[4.5]decan-1-amine |
| 17 | 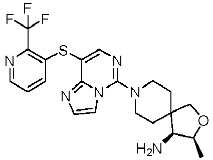 |
| | (3S,4S)-3-Methyl-8-(8-((2-(trifluoromethyl)pyridin-3-yl)thio)imidazo[1,2-c]pyrimidin-5-yl)-2-oxa-8-azaspiro[4.5]decan-4-amine |
| 18 | 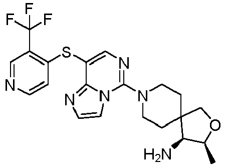 |
| | (3S,4S)-3-Methyl-8-(8-((3-(trifluoromethyl)pyridin-4-yl)thio)imidazo[1,2-c]pyrimidin-5-yl)-2-oxa-8-azaspiro[4.5]decan-4-amine |
| 19 | 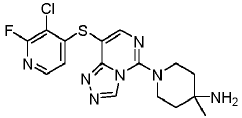 |
| | 1-(8-((3-Chloro-2-fluoropyridin-4-yl)thio)-[1,2,4]triazolo[4,3-c]pyrimidin-5-yl)-4-methylpiperidin-4-amine |
| 20 | 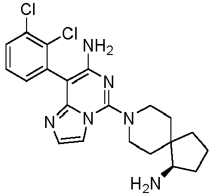 |
| | (R)-8-(7-Amino-8-(2,3-dichlorophenyl)imidazo[1,2-c]pyrimidin-5-yl)-8-az aspiro[4.5]decan-1-amine |
| 21 | 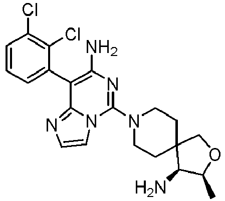 |
| | (3S,4S)-8-(7-Amino-8-(2,3-dichlorophenyl)imidazo[1,2-c]pyrimidin-5-yl)-3-methyl-2-oxa-8-azaspiro[4.5]decan-4-amine |
| 22 | 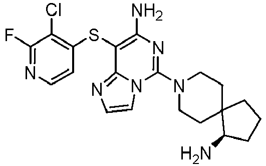 |
| | (R)-8-(7-Amino-8-((3-chloro-2-fluoropyridin-4-yl)thio)imidazo[1,2-c]pyri midin-5-yl)-8-azaspiro[4.5]decan-1-amine |
| 23 | 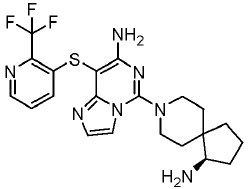 |
| | (R)-8-(7-Amino-8-((2-(trifluoromethyl)pyridin-3-yl)thio)imidazo[1,2-c]pyr imidin-5-yl)-8-azaspiro[4.5]decan-1-amine |
| 24 | 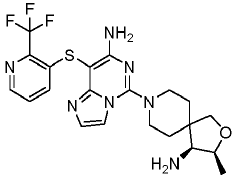 |
| | (3S,4S)-8-(7-Amino-8-((2-(trifluoromethyl)pyridin-3-yl)thio)imidazo[1,2-c ]pyrimidin-5-yl)-3-methyl-2-oxa-8-azaspiro[4.5]decan-4-amine |
| 25 | 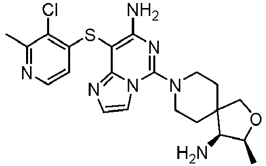 |
| | (3S,4S)-8-(7-Amino-8-((3-chloro-2-methylpyridin-4-yl)thio)imidazo[1,2-c ]pyrimidin-5-yl)-3-methyl-2-oxa-8-azaspiro[4.5]decan-4-amine |
| 26 | 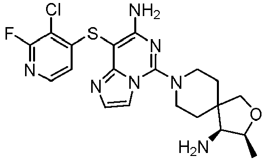 |
| | (3S,4S)-8-(7-Amino-8-((3-chloro-2-fluoropyridin-4-yl)thio)imidazo[1,2-c] pyrimidin-5-yl)-3-methyl-2-oxa-8-azaspiro[4.5]decan-4-amine |
| 27 | 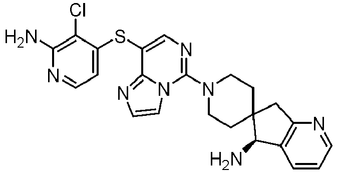 |
| | (S)-1'-(8-((2-Amino-3-chloropyridin-4-yl)thio)imidazo[1,2-c]pyrimidin-5-yl)-5,7-dihydrospiro[cyclopenta[b]pyridine-6,4'-piperidin]-5-amine |
| 28 | 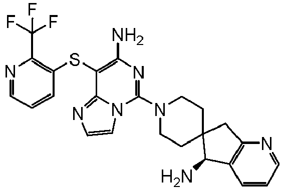 |
| | (S)-1'-(7-Amino-8-((2-(trifluoromethyl)pyridin-3-yl)thio)imidazo[1,2-c]py rimidin-5-yl)-5,7-dihydrospiro[cyclopenta[b]pyridine-6,4'-piperidin]-5-ami ne |
| 29 | 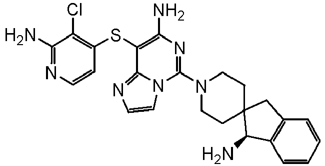 |
| | (S)-1'-(7-Amino-8-((2-amino-3-chloropyridin-4-yl)thio)imidazo[1,2-c]pyri midin-5-yl)-1,3-dihydrospiro[indene-2,4'-piperidin]-1-amine |
| 30 | 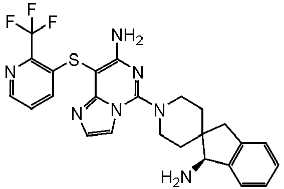 |
| | (S)-1'-(7-Amino-8-((2-(trifluoromethyl)pyridin-3-yl)thio)imidazo[1,2-c]py rimidin-5-yl)-1,3-dihydrospiro[indene-2,4'-piperidin]-1-amine |
| 31 | 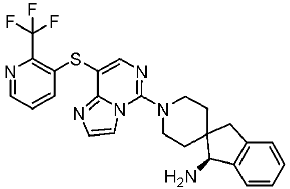 |
| | (S)-1'-(8-((2-(Trifluoromethyl)pyridin-3-yl)thio)imidazo[1,2-c]pyrimidin-5 -yl)-1,3-dihydrospiro[indene-2,4'-piperidin]-1-amine |
| 32 | 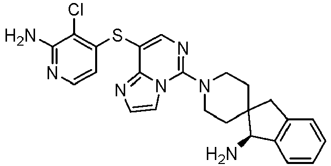 |
| | (S)-1'-(8-((2-Amino-3-chloropyridin-4-yl)thio)imidazo[1,2-c]pyrimidin-5-yl)-1,3-dihydrospiro[indene-2,4'-piperidin]-1-amine |
| 33 | 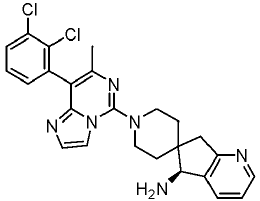 |
| | (S)-1'-(8-(2,3-Dichlorophenyl)-7-methylimidazo[1,2-c]pyrimidin-5-yl)-5,7 -dihydrospiro[cyclopenta[b]pyridine-6,4'-piperidin]-5-amine |
| 34 | 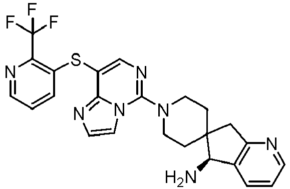 |
| | (S)-1'-(8-((2-(Trifluoromethyl)pyridin-3-yl)thio)imidazo[1,2-c]pyrimidin-5 -yl)-5,7-dihydrospiro[cyclopenta[b]pyridine-6,4'-piperidin]-5-amine |
| 35 | 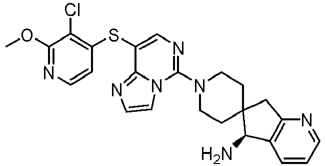 |
| | (S)-1'-(8-((3-Chloro-2-methoxypyridin-4-yl)thio)imidazo[1,2-c]pyrimidin-5-yl)-5,7-dihydrospiro[cyclopenta[b]pyridine-6,4'-piperidin]-5-amine |
| 36 | 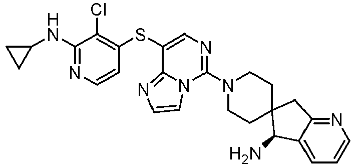 |
| | (S)-1'-(8-((3-Chloro-2-(cyclopropylamino)pyridin-4-yl)thio)imidazo[1,2-c] pyrimidin-5-yl)-5,7-dihydrospiro[cyclopenta[b]pyridine-6,4'-piperidin]-5-a mine |
| 37 | 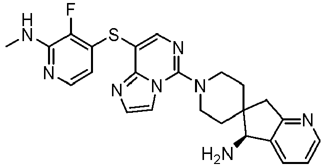 |
| | (S)-1'-(8-((3-Fluoro-2-(methylamino)pyridin-4-yl)thio)imidazo[1,2-c]pyri midin-5-yl)-5,7-dihydrospiro[cyclopenta[b]pyridine-6,4'-piperidin]-5-amin e |
| 38 | 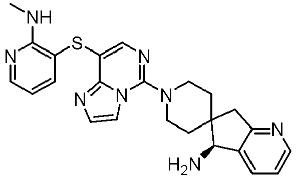 |
| | (S)-1'-(8-((2-(Methylamino)pyridin-3-yl)thio)imidazo[1,2-c]pyrimidin-5-yl )-5,7-dihydrospiro[cyclopenta[b]pyridine-6,4'-piperidin]-5-amine |
| 39 | 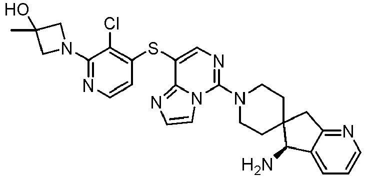 |
| | (S)-1-(4-((5-(5-Amino-5,7-dihydrospiro[cyclopenta[b]pyridine-6,4'-piperid in]-1'-yl)imidazo[1,2-c]pyrimidin-8-yl)thio)-3-chloropyridin-2-yl)-3-methy lazetidin-3-ol |
| 40 | 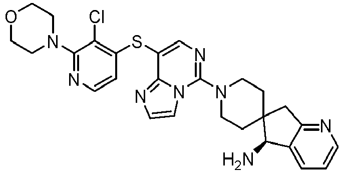 |
| | (S)-1'-(8-((3-Chloro-2-morpholinopyridin-4-yl)thio)imidazo[1,2-c]pyrimid in-5-yl)-5,7-dihydrospiro[cyclopenta[b]pyridine-6,4'-piperidin]-5-amine |
| 41 | 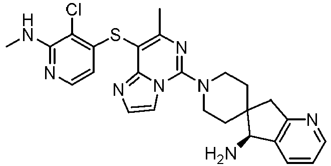 |
| | (S)-1'-(8-((3-Chloro-2-(methylamino)pyridin-4-yl)thio)-7-methylimidazo[1 ,2-c]pyrimidin-5-yl)-5,7-dihydrospiro[cyclopenta[b]pyridine-6,4'-piperidin ]-5-amine |
| 42 | 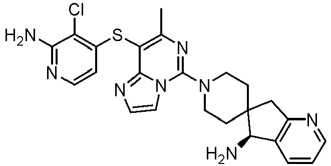 |
| | (S)-1'-(8-((2-Amino-3-chloropyridin-4-yl)thio)-7-methylimidazo[1,2-c]pyr imidin-5-yl)-5,7-dihydrospiro[cyclopenta[b]pyridine-6,4'-piperidin]-5-ami ne |
| 43 | 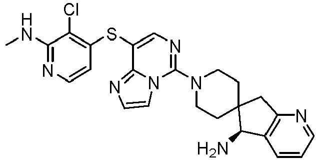 |
| | (S)-1'-(8-((3-Chloro-2-(methylamino)pyridin-4-yl)thio)imidazo[1,2-c]pyri midin-5-yl)-5,7-dihydrospiro[cyclopenta[b]pyridine-6,4'-piperidin]-5-amin e |
| 44 | 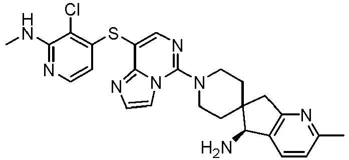 |
| | (S)-1'-(8-((3-Chloro-2-(methylamino)pyridin-4-yl)thio)imidazo[1,2-c]pyri midin-5-yl)-2-methyl-5,7-dihydrospiro[cyclopenta[b]pyridine-6,4'-piperidi n]-5-amine |

or a tautomer, mesomer, racemate, enantiomer, diastereomer, atropisomer thereof, or mixture thereof, or a pharmaceutically acceptable salt thereof.

The present invention provides a method for preparing the compound of formula (I), wherein the compound of formula (I) is a compound of formula (I-A) or a compound of formula (I-B), characterized by comprising the steps of 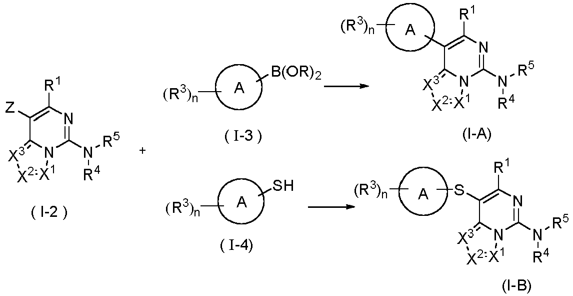
subjecting a compound of formula (1-2) and a compound of formula (1-3) to a Suzuki coupling reaction under an alkaline condition in the presence of a catalyst to obtain the compound of formula (I-A), the catalyst is selected from the group consisting of palladium on carbon, Raney nickel, tetrakis(triphenylphosphine)palladium, palladium dichloride, palladium acetate, [1,1'-bis(diphenylphosphino)ferrocene]palladium (II) dichloride, 1,1'-bis(dibenzylphosphino)dichloroferrocene palladium (II), tris(dibenzylideneacetone)dipalladium and 2-dicyclohexylphosphino-2',6'-dimethoxybiphenyl, and preferably [1,1'-bis(diphenylphosphino)ferrocene]palladium (II) dichloride and 2-dicyclohexylphosphino-2',6'-dimethoxybiphenyl; or
subjecting a compound of formula (1-2) and a compound of formula (1-4) to a C-S coupling reaction under an alkaline condition to obtain the compound of formula (I-B);
wherein the reagent that provides an alkaline condition includes organic bases and inorganic bases; the organic base is selected from the group consisting of triethylamine, *N,N*-diisopropylethylamine, n-butyllithium, lithium diisopropylamide, lithium bistrimethylsilylamide, potassium acetate, sodium *tert*-butoxide and potassium *tert*-butoxide; the inorganic base is selected from the group consisting of sodium hydride, potassium phosphate, sodium carbonate, potassium carbonate, potassium acetate, cesium carbonate, sodium hydroxide and lithium hydroxide;
B(OR)₂ is a borate or boric acid that includes, but is not limited to, 4,4,5,5-tetramethyl-1,3,2-dioxaborolane, 4,4,4',4',5,5,5',5'-octamethyl-2,2'-bis(1,3,2-dioxaborolane), bis(neopentyl glycolato)diboron, B(OBu-n)₃ and B(OPr-i)₃;
Z is selected from the group consisting of halogen and sulfonyl; and
R¹, X¹, X², X³, R³, R⁴ and R⁵ are as defined in the above formula (I).

In order to achieve the object of the present invention, the present invention can apply the following synthesis scheme of: 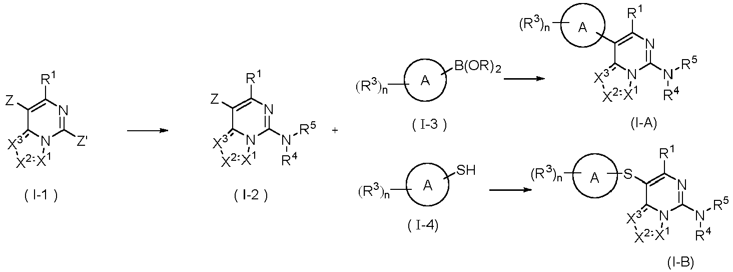 ammonifying a compound of formula (I-1) to obtain the compound of formula (1-2), wherein Z and Z' are each independently selected from the group consisting of halogen and sulfonyl, other substituents are as defined in the foregoing embodiment, the reaction solvent of the synthesis scheme of the present invention includes, but is not limited to, acetic acid, methanol, ethanol, toluene, tetrahydrofuran, dichloromethane, petroleum ether, ethyl acetate, n-hexane, dimethyl sulfoxide, 1,4-dioxane, water, *N,N*-dimethylformamide and mixtures thereof.

The present invention provides a method for preparing the compound of formula (II) or the tautomer, mesomer, racemate, enantiomer, diastereomer, atropisomer thereof, or mixture thereof, or the pharmaceutically acceptable salt thereof, wherein the compound of formula (II) is a compound of formula (II-A) or a compound of formula (II-B), comprising the following steps of: 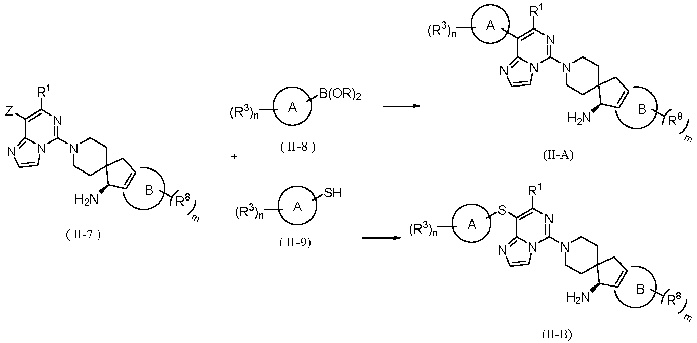
subjecting a compound of formula (II-7) and a compound of formula (II-8) to a Suzuki coupling reaction under an alkaline condition in the presence of a catalyst to obtain the compound of formula (II-A);
or subjecting a compound of formula (II-7) and a compound of formula (II-9) to a C-S coupling reaction under an alkaline condition to obtain the compound of formula (II-B);
wherein the catalyst is selected from the group consisting of palladium on carbon, Raney nickel, tetrakis(triphenylphosphine)palladium, palladium dichloride, palladium acetate, [1,1'-bis(diphenylphosphino)ferrocene]palladium (II) dichloride, 1,1'-bis(dibenzylphosphino)dichloroferrocene palladium (II), tris(dibenzylideneacetone)dipalladium and 2-dicyclohexylphosphino-2',6'-dimethoxybiphenyl, and preferably [1,1'-bis(diphenylphosphino)ferrocene]palladium (II) dichloride and 2-dicyclohexylphosphino-2',6'-dimethoxybiphenyl;
the reagent that provides an alkaline condition includes organic bases and inorganic bases; the organic base is selected from the group consisting of triethylamine, *N,N*-diisopropylethylamine, n-butyllithium, lithium diisopropylamide, lithium bistrimethylsilylamide, potassium acetate, sodium *tert*-butoxide and potassium *tert*-butoxide; the inorganic base is selected from the group consisting of sodium hydride, potassium phosphate, sodium carbonate, potassium carbonate, potassium acetate, cesium carbonate, sodium hydroxide and lithium hydroxide;
B(OR)₂ is a borate or boric acid that includes, but is not limited to, 4,4,5,5-tetramethyl-1,3,2-dioxaborolane, 4,4,4',4',5,5,5',5'-octamethyl-2,2'-bis(1,3,2-dioxaborolane), bis(neopentyl glycolato)diboron, B(OBu-n)₃ and B(OPr-i)₃;
Z is selected from the group consisting of halogen, sulfonyl and sulfinyl; and
ring A, ring B, R¹, R³, R⁸, B, m and n are as defined in the above formula (II).

The method for preparing the compound of formula (II) or the tautomer, mesomer, racemate, enantiomer, diastereomer, atropisomer thereof, or mixture thereof, or the pharmaceutically acceptable salt thereof provided by the present invention further comprises a step of reacting a compound of formula (II-5) with a compound of formula (II-6) under an alkaline condition to obtain the compound of formula (II-7), 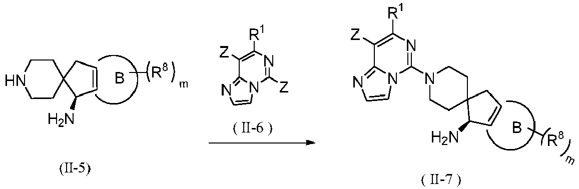
wherein the reagent that provides an alkaline condition includes organic bases and inorganic bases; the organic base is selected from the group consisting of triethylamine, *N,N*-diisopropylethylamine, n-butyllithium, lithium diisopropylamide, lithium bistrimethylsilylamide, potassium acetate, sodium *tert*-butoxide and potassium *tert*-butoxide; the inorganic base is selected from the group consisting of sodium hydride, potassium phosphate, sodium carbonate, potassium carbonate, potassium acetate, cesium carbonate, sodium hydroxide and lithium hydroxide; and
Z, R¹, R⁸, ring B and m are as defined in formula (II).

Optionally, the method for preparing the compound of formula (II) or the tautomer, mesomer, racemate, enantiomer, diastereomer, atropisomer thereof, or mixture thereof, or the pharmaceutically acceptable salt thereof provided by the present invention further comprises the following steps of: 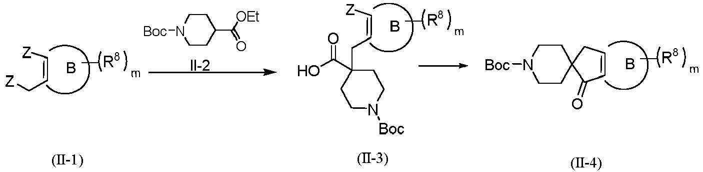
1) reacting a compound of formula (II-1) with a compound of formula (II-2) under an alkaline condition to obtain a compound of formula (II-3); 2) subjecting the compound of formula (II-3) to an intramolecular cyclization reaction in the presence of *n*-butyl lithium to obtain a compound of formula (II-4); subjecting the compound of formula (II-4) to a chiral selective reductive amination followed by removing the amino protecting group to obtain the compound of formula (II-5); wherein the reagent that provides an alkaline condition includes organic bases and inorganic bases; the organic base is selected from the group consisting of triethylamine, *N,N*-diisopropylethylamine, n-butyllithium, lithium diisopropylamide, lithium bistrimethylsilylamide, potassium acetate, sodium *tert*-butoxide and potassium *tert*-butoxide; the inorganic base is selected from the group consisting of sodium hydride, potassium phosphate, sodium carbonate, potassium carbonate, potassium acetate, cesium carbonate, sodium hydroxide and lithium hydroxide; and
Z, R⁸, ring B and m are as defined in formula (II).

The present invention provides a compound of formula (1-2) or a pharmaceutically acceptable salt thereof, 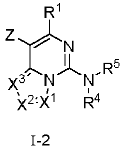
wherein R¹, X¹, X², X³, R⁴ and R⁵ are as defined in formula (I);
Z is selected from the group consisting of halogen and sulfonyl.

The present invention provides a compound of formula (I-1) or a pharmaceutically acceptable salt thereof 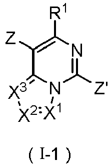
wherein R¹, X¹, X² and X³ are as defined in formula (I);
Z and Z' are each independently selected from the group consisting of halogen and sulfonyl.

The present invention provides a method for preparing the compound of formula (I) from the compound of formula (1-2) or the pharmaceutically acceptable salt thereof or the compound of formula (I-1) or the pharmaceutically acceptable salt thereof.

In another aspect, the present invention relates to a pharmaceutical composition comprising a therapeutically effective amount of the compound of formula (I) or formula (II) or the tautomer, mesomer, racemate, enantiomer, diastereomer, atropisomer thereof, or mixture thereof, or the pharmaceutically acceptable salt thereof, and one or more pharmaceutically acceptable carrier, diluent or excipient, and the therapeutically effective amount of the present invention can be from 0.1 to 2000 mg. The present invention also relates to a method for preparing the pharmaceutical composition comprising a step of mixing the compound of formula (I) or formula (II) or the tautomer, mesomer, racemate, enantiomer, diastereomer, atropisomer thereof, or mixture thereof, or the pharmaceutically acceptable salt thereof or the compound of formula (II) or the tautomer, mesomer, racemate, enantiomer, diastereomer, atropisomer thereof, or mixture thereof, or the pharmaceutically acceptable salt thereof with the pharmaceutically acceptable carrier, diluent or excipient.

The present invention further relates to a use of the compound of formula (I) or formula (II) or the tautomer, mesomer, racemate, enantiomer, diastereomer, atropisomer thereof, or mixture thereof, or the pharmaceutically acceptable salt thereof, or the pharmaceutical composition comprising the same in the preparation of a SHP2 inhibitor.

The present invention further relates to a use of the compound of formula (I) or formula (II) or the tautomer, mesomer, racemate, enantiomer, diastereomer, atropisomer thereof, or mixture thereof, or the pharmaceutically acceptable salt thereof, or the pharmaceutical composition comprising the same in the preparation of a medicament for treating a disease or condition mediated by SHP2 activity.

The present invention further relates to a use of the compound of formula (I) or formula (II) or the tautomer, mesomer, racemate, enantiomer, diastereomer, atropisomer thereof, or mixture thereof, or the pharmaceutically acceptable salt thereof, or the pharmaceutical composition comprising the same as a SHP2 inhibitor in the preparation of a medicament for preventing and/or treating tumor or cancer.

The present invention further relates to a use of the compound of formula (I) or formula (II) or the tautomer, mesomer, racemate, enantiomer, diastereomer, atropisomer thereof, or mixture thereof, or the pharmaceutically acceptable salt thereof, or the pharmaceutical composition comprising the same in the preparation of a medicament for preventing or treating Noonan syndrome, Leopard syndrome, juvenile myelomonocytic leukemia, neuroblastoma, melanoma, acute myelogenous leukemia, breast cancer, esophageal cancer, lung cancer, colon cancer, head cancer, pancreatic cancer, head and neck squamous cell carcinoma, stomach cancer, liver cancer, anaplastic large cell lymphoma or glioblastoma.

The present invention further relates to the compound of formula (I) or formula (II) or the tautomer, mesomer, racemate, enantiomer, diastereomer, atropisomer thereof, or mixture thereof, or the pharmaceutically acceptable salt thereof, or the pharmaceutical composition comprising the same, for use as a medicament.

The present invention also relates to the compound of formula (I) or formula (II) or the tautomer, mesomer, racemate, enantiomer, diastereomer, atropisomer thereof, or mixture thereof, or the pharmaceutically acceptable salt thereof, or the pharmaceutical composition comprising the same, for use as a SHP2 inhibitor.

The present invention also relates to the compound of formula (I) or formula (II) or the tautomer, mesomer, racemate, enantiomer, diastereomer, atropisomer thereof, or mixture thereof, or the pharmaceutically acceptable salt thereof, or the pharmaceutical composition comprising the same, for use as a SHP2 inhibitor in preventing and/or treating tumor or cancer.

The present invention also relates to the compound of formula (I) or formula (II) or the tautomer, mesomer, racemate, enantiomer, diastereomer, atropisomer thereof, or mixture thereof, or the pharmaceutically acceptable salt thereof, or the pharmaceutical composition comprising the same, for use in preventing or treating Noonan syndrome, Leopard syndrome, juvenile myelomonocytic leukemia, neuroblastoma, melanoma, acute myelogenous leukemia, breast cancer, esophageal cancer, lung cancer, colon cancer, head cancer, pancreatic cancer, head and neck squamous cell carcinoma, stomach cancer, liver cancer, anaplastic large cell lymphoma or glioblastoma.

The present invention also relates to a method for preventing and/or treating tumor or cancer, comprising a step of administering to a patient in need thereof a therapeutically effective dose of the compound of formula (I) or formula (II) or the tautomer, mesomer, racemate, enantiomer, diastereomer, atropisomer thereof, or mixture thereof, or the pharmaceutically acceptable salt thereof as a SHP2 inhibitor.

The present invention also relates to a method for preventing or treating Noonan syndrome, Leopard syndrome, juvenile myelomonocytic leukemia, neuroblastoma, melanoma, acute myelogenous leukemia, breast cancer, esophageal cancer, lung cancer, colon cancer, head cancer, pancreatic cancer, head and neck squamous cell carcinoma, stomach cancer, liver cancer, anaplastic large cell lymphoma or glioblastoma, comprising a step of administering to a patient in need thereof a therapeutically effective dose of the compound of formula (I) or formula (II) or the tautomer, mesomer, racemate, enantiomer, diastereomer, atropisomer thereof, or mixture thereof, or the pharmaceutically acceptable salt thereof as a SHP2 inhibitor.

The pharmaceutical composition containing the active ingredient can be in a form suitable for oral administration, for example, a tablet, troche, lozenge, aqueous or oily suspension, dispersible powder or granule, emulsion, hard or soft capsule, syrup or elixir. An oral composition can be prepared according to any known method in the art for the preparation of pharmaceutical composition. Such a composition can contain one or more ingredient(s) selected from the group consisting of sweeteners, flavoring agents, colorants and preservatives, in order to provide a pleasing and palatable pharmaceutical formulation. The tablet contains the active ingredient in admixture with nontoxic, pharmaceutically acceptable excipients suitable for the manufacture of tablets. These excipients can be inert excipients, granulating agents, disintegrating agents, binders and lubricants. The tablet can be uncoated or coated by means of a known technique to mask drug taste or delay the disintegration and absorption of the active ingredient in the gastrointestinal tract, thereby providing sustained release over a long period of time.

An oral formulation can also be provided as soft gelatin capsules in which the active ingredient is mixed with an inert solid diluent, or the active ingredient is mixed with a water-soluble carrier or an oil medium.

An aqueous suspension contains the active ingredient in admixture with excipients suitable for the manufacture of an aqueous suspension. Such excipients are suspending agents, dispersants or wetting agents. The aqueous suspension can also contain one or more preservatives, one or more colorants, one or more flavoring agents, and one or more sweeteners.

An oil suspension can be formulated by suspending the active ingredient in a vegetable oil or mineral oil. The oil suspension can contain a thickener. The aforementioned sweeteners and flavoring agents can be added to provide a palatable formulation. These compositions can be preserved by adding an antioxidant.

The pharmaceutical composition of the present invention can also be in the form of an oil-in-water emulsion. The oil phase can be a vegetable oil, or a mineral oil, or a mixture thereof. Suitable emulsifying agents can be naturally occurring phospholipids. The emulsion can also contain a sweetening agent, flavoring agent, preservative and antioxidant. Such a formulation can also contain a demulcent, preservative, colorant and antioxidant.

The pharmaceutical composition of the present invention can be in the form of a sterile injectable aqueous solution. Acceptable vehicles or solvents that can be used are water, Ringer's solution or isotonic sodium chloride solution. The sterile injectable formulation can be a sterile injectable oil-in-water micro-emulsion in which the active ingredient is dissolved in the oil phase. The injectable solution or micro-emulsion can be introduced into a patient's bloodstream by local bolus injection. Alternatively, the solution and micro-emulsion are preferably administered in a manner that maintains a constant circulating concentration of the compound of the present invention. In order to maintain this constant concentration, a continuous intravenous delivery device can be used. An example of such a device is Deltec CADD-PLUS. TM. 5400 intravenous injection pump.

The pharmaceutical composition of the present invention can be in the form of a sterile injectable aqueous or oily suspension for intramuscular and subcutaneous administration. Such a suspension can be formulated with suitable dispersants or wetting agents and suspending agents as described above according to known techniques. The sterile injectable formulation can also be a sterile injectable solution or suspension prepared in a nontoxic parenterally acceptable diluent or solvent. Moreover, sterile fixed oils can easily be used as a solvent or suspending medium. For this purpose, any blended fixed oil can be used. In addition, fatty acids can also be used to prepare injections.

The compound of the present invention can be administered in the form of a suppository for rectal administration. These pharmaceutical compositions can be prepared by mixing the drug with a suitable non-irritating excipient that is solid at ordinary temperatures, but liquid in the rectum, thereby melting in the rectum to release the drug.

It is well known to those skilled in the art that the dosage of a drug depends on a variety of factors including but not limited to, the following factors: activity of a specific compound, age of the patient, weight of the patient, general health of the patient, behavior of the patient, diet of the patient, administration time, administration route, excretion rate, drug combination and the like. In addition, the optimal treatment, such as treatment mode, daily dose of the compound of formula (I) or the type of pharmaceutically acceptable salt thereof can be verified by traditional therapeutic regimens.

### DEDINITIONS

Unless otherwise stated, the terms used in the specification and claims have the meanings described below.

The term "alkyl" refers to a saturated aliphatic hydrocarbon group, which is a straight or branched chain group comprising 1 to 20 carbon atoms, preferably an alkyl having 1 to 12 carbon atoms, and more preferably an alkyl having 1 to 6 carbon atoms. Non-limiting examples include methyl, ethyl, n-propyl, isopropyl, *n*-butyl, isobutyl, *tert-butyl, sec*-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof. More preferably, the alkyl group is a lower alkyl having 1 to 6 carbon atoms, and non-limiting examples include methyl, ethyl, n-propyl, isopropyl, *n*-butyl, isobutyl, *tert*-butyl, *sec*-butyl, n-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and the like. The alkyl group can be substituted or unsubstituted. When substituted, the substituent group(s) can be substituted at any available connection point. The substituent group(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and alkoxycarbonyl.

The term "cycloalkyl" refers to a saturated or partially unsaturated monocyclic or polycyclic hydrocarbon substituent group having 3 to 20 carbon atoms, preferably 3 to 12 carbon atoms, and more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl and the like. Polycyclic cycloalkyl includes a cycloalkyl having a spiro ring, fused ring or bridged ring.

The term "spiro cycloalkyl" refers to a 5 to 20 membered polycyclic group with individual rings connected through one shared carbon atom (called a spiro atom), wherein the rings can contain one or more double bonds, but none of the rings has a completely conjugated *π*-electron system. The spiro cycloalkyl is preferably a 6 to 14 membered spiro cycloalkyl, and more preferably a 7 to 10 membered spiro cycloalkyl. According to the number of the spiro atoms shared between the rings, the spiro cycloalkyl can be divided into a mono-spiro cycloalkyl, di-spiro cycloalkyl, or poly-spiro cycloalkyl, and the spiro cycloalkyl is preferably a mono-spiro cycloalkyl or di-spiro cycloalkyl, and more preferably a 4-membered/4-membered, 4-membered/5-membered, 4-membered/6-membered, 5-membered/5-membered, or 5-membered/6-membered mono-spiro cycloalkyl. Non-limiting examples of spiro cycloalkyl include:

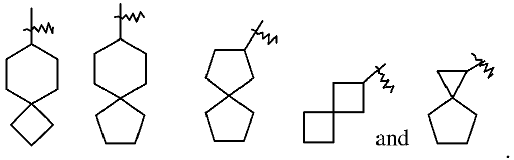

The term "heterocyclyl" refers to a 3 to 20 membered saturated or partially unsaturated monocyclic or polycyclic hydrocarbon substituent group, wherein one or more ring atoms are heteroatoms selected from the group consisting of N, O and S(O)ₘ (wherein m is an integer of 0 to 2), but excluding -O-O-, -O-S- or -S-S- in the ring, with the remaining ring atoms being carbon atoms. Preferably, the heterocyclyl has 3 to 12 ring atoms wherein 1 to 4 atoms are heteroatoms; most preferably, 3 to 8 ring atoms wherein 1 to 3 atoms are heteroatoms; and most preferably 3 to 6 ring atoms wherein 1 to 2 atoms are heteroatoms. Non-limiting examples of monocyclic heterocyclyl include azetidinyl, pyrrolidinyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, pyranyl and the like, and preferably azetidinyl, piperidinyl, piperazinyl or morpholinyl. Polycyclic heterocyclyl includes a heterocyclyl having a spiro ring, fused ring or bridged ring.

The heterocyclyl ring can be fused to the ring of aryl, heteroaryl or cycloalkyl, wherein the ring bound to the parent structure is the heterocyclyl. Non-limiting examples thereof include: 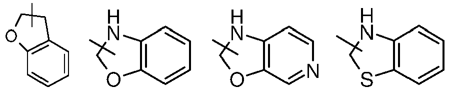 and the like.

The term "aryl" refers to a 6 to 14 membered all-carbon monocyclic ring or polycyclic fused ring (*i.e.* each ring in the system shares an adjacent pair of carbon atoms with another ring in the system) having a conjugated *π*-electron system, preferably a 6 to 10 membered aryl, for example, phenyl and naphthyl, and more preferably phenyl. The aryl ring can be fused to the ring of heteroaryl, heterocyclyl or cycloalkyl, wherein the ring bound to the parent structure is the aryl ring. Non-limiting examples thereof include:

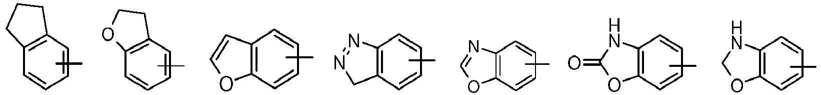

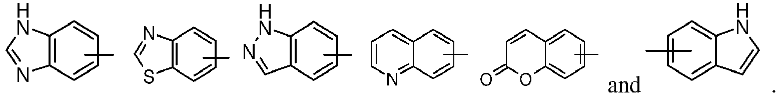

The aryl can be substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

The term "heteroaryl" refers to a 5 to 14 membered heteroaromatic system having 1 to 4 heteroatoms selected from the group consisting of O, S and N. The heteroaryl is preferably a 5 to 10 membered heteroaryl having 1 to 3 heteroatoms, more preferably a 5 or 6 membered heteroaryl having 1 to 2 heteroatoms; preferably for example, imidazolyl, furyl, thienyl, thiazolyl, pyrazolyl, oxazolyl, pyrrolyl, tetrazolyl, pyridyl, pyrimidinyl, thiadiazolyl, pyrazinyl and the like, preferably imidazolyl, tetrazolyl, pyridyl, thienyl, pyrazolyl, pyrimidinyl, thiazolyl, and more preferably pyridyl. The heteroaryl ring can be fused to the ring of aryl, heterocyclyl or cycloalkyl, wherein the ring bound to the parent structure is the heteroaryl ring. Non-limiting examples thereof include:

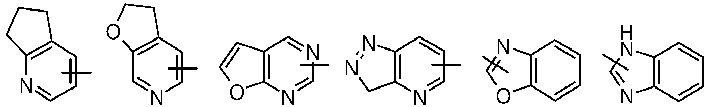

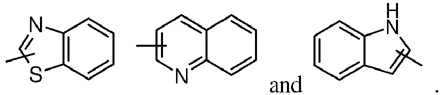

The heteroaryl can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

The term "halogen" refers to fluorine, chlorine, bromine or iodine.

The term "haloalkyl" refers to an alkyl group substituted by one or more halogen(s), wherein the alkyl is as defined above.

The term "haloalkoxy" refers to an alkoxy group substituted by one or more halogen(s), wherein the alkoxy is as defined above.

The term "hydroxyalkyl" refers to an alkyl group substituted by hydroxy(s), wherein the alkyl is as defined above.

The term "alkylamino" refers to an amino group substituted by one or two alkyl(s), wherein the alkyl is as defined above.

The term "hydroxy" refers to an -OH group.

The term "halogen" refers to fluorine, chlorine, bromine or iodine.

The term "amino" refers to a -NH₂ group.

The term "cyano" refers to a -CN group.

The term "nitro" refers to a -NO₂ group.

The term "oxo" refers to a =O group.

The term "carbonyl" refers to a C=O group.

The term "carboxy" refers to a -C(O)OH group.

The term "thio" refers to a -S- group.

The term "thiol" refers to a -SH group.

"Optional" or "optionally" means that the event or circumstance described subsequently can, but need not, occur, and such a description includes the situation in which the event or circumstance does or does not occur. For example, "the heterocyclyl optionally substituted by an alkyl" means that an alkyl group can be, but need not be, present, and such a description includes the situation of the heterocyclyl being substituted by an alkyl and the heterocyclyl being not substituted by an alkyl.

"Substituted" refers to one or more hydrogen atoms in a group, preferably up to 5, and more preferably 1 to 3 hydrogen atoms, independently substituted by a corresponding number of substituents. It goes without saying that the substituents only exist in their possible chemical position. The person skilled in the art is able to determine whether the substitution is possible or impossible by experiments or theory without excessive effort. For example, the combination of amino or hydroxy having free hydrogen and carbon atoms having unsaturated bonds (such as olefinic) may be unstable.

A "pharmaceutical composition" refers to a mixture of one or more of the compounds according to the present invention or physiologically/pharmaceutically acceptable salts or prodrugs thereof with other chemical components, and other components such as physiologically/pharmaceutically acceptable carriers and excipients. The purpose of the pharmaceutical composition is to facilitate administration of a compound to an organism, which is conducive to the absorption of the active ingredient so as to show biological activity.

A "pharmaceutically acceptable salt" refers to a salt of the compound of the present invention, which is safe and effective in mammals and has the desired biological activity.

### DETAILED DESCRIPTION OF THE INVENTION

The present invention will be further described with reference to the following examples, but the examples should not be considered as limiting the scope of the present invention.

### EXAMPLES

The structures of the compounds were identified by nuclear magnetic resonance (NMR) and/or mass spectrometry (MS). NMR shifts (δ) are given in 10⁻⁶ (ppm). NMR was determined by a Bruker AVANCE-400 machine. The solvents for determination were deuterated-dimethyl sulfoxide (DMSO-*d₆*), deuterated-chloroform (CDCl₃) and deuterated-methanol (CD₃OD), and the internal standard was tetramethylsilane (TMS).

MS was determined by a Shimadzu 2010 Mass spectrometer or Agilent 6110A MSD spectrometer.

High performance liquid chromatography (HPLC) was determined on a Shimadzu LC-20A systems, Shimadzu LC-2010HT series or Agilent 1200 LC high pressure liquid chromatograph (Ultimate XB-C18 3.0*150 mm column or Xtimate C18 2.1*30 mm column).

Chiral HPLC was determined on a Chiralpak IC-3 100×4.6 mm I.D., 3 µm, Chiralpak AD-3 150×4.6 mm I.D., 3 µm, Chiralpak AD-3 50×4.6 mm I.D., 3 µm, Chiralpak AS-3 150×4.6 mm I.D., 3 µm, Chiralpak AS-3 100×4.6 mm I.D., 3 µm, ChiralCel OD-3 150×4.6 mm I.D., 3 µm, Chiralcel OD-3 100×4.6 mm I.D., 3 µm, ChiralCel OJ-H 150×4.6 mm I.D., 5 µm, Chiralcel OJ-3 150×4.6 mm I.D., 3 µm column.

Yantai Huanghai HSGF254 or Qingdao GF254 silica gel plate was used as the thin-layer silica gel chromatography (TLC) plate. The dimension of the silica gel plates used in TLC was 0.15 mm to 0.2 mm, and the dimension of the silica gel plates used in product purification by thin-layer chromatography was 0.4 mm to 0.5 mm.

Yantai Huanghai 100 to 200 mesh, 200 to 300 mesh or 300 to 400 mesh silica gel was generally used as a carrier for column chromatography.

Chiral preparation column used was DAICEL CHIRALPAK IC (250mm*30mm, 10 µm) or Phenomenex-Amylose-1 (250mm*30mm, 5 µm).

CombiFlash rapid preparation instrument used was Combiflash Rf150 (TELEDYNE ISCO).

The average kinase inhibition rates and IC₅₀ values were determined by a NovoStar ELISA (BMG Co., Germany).

The known starting materials of the present invention can be prepared by the known methods in the art, or can be purchased from ABCR GmbH & Co. KG, Acros Organnics, Aldrich Chemical Company, Accela ChemBio Inc., Dari chemical Company etc.

Unless otherwise stated, the reactions were carried out under argon atmosphere or nitrogen atmosphere.

"Argon atmosphere" or "nitrogen atmosphere" means that a reaction flask is equipped with an argon or nitrogen balloon (about1 L).

"Hydrogen atmosphere" means that a reaction flask is equipped with a hydrogen balloon (about1 L).

Pressurized hydrogenation reactions were performed on a Parr 3916EKX hydrogenation instrument and a Qinglan QL-500 hydrogen generator or HC2-SS hydrogenation instrument.

In hydrogenation reactions, the reaction system was generally vacuumed and filled with hydrogen, and the above operation was repeated three times.

CEM Discover-S 908860 type microwave reactor was used in microwave reactions.

Unless otherwise stated, the solution refers to an aqueous solution.

Unless otherwise stated, the reaction temperature is room temperature from 20°C to 30°C.

The reaction process in the examples was monitored by thin layer chromatography (TLC). The developing solvent used in the reactions, the eluent system in column chromatography and the developing solvent system in thin layer chromatography for purification of the compounds included: A: dichloromethane/methanol system, B: *n*-hexane/ethyl acetate system, C: petroleum ether/ethyl acetate system, and D: petroleum ether/ethyl acetate/methanol system. The ratio of the volume of the solvent was adjusted according to the polarity of the compounds, and a small quantity of alkaline reagent such as triethylamine or acidic reagent such as acetic acid could also be added for adjustment.

### Example 1

### (R)-8-(8-(2,3-Dichlorophenyl)-7-methylimidazo[1,2-c]pyrimidin-5-yl)-8-azaspiro[4.5]d ecan-1-amine

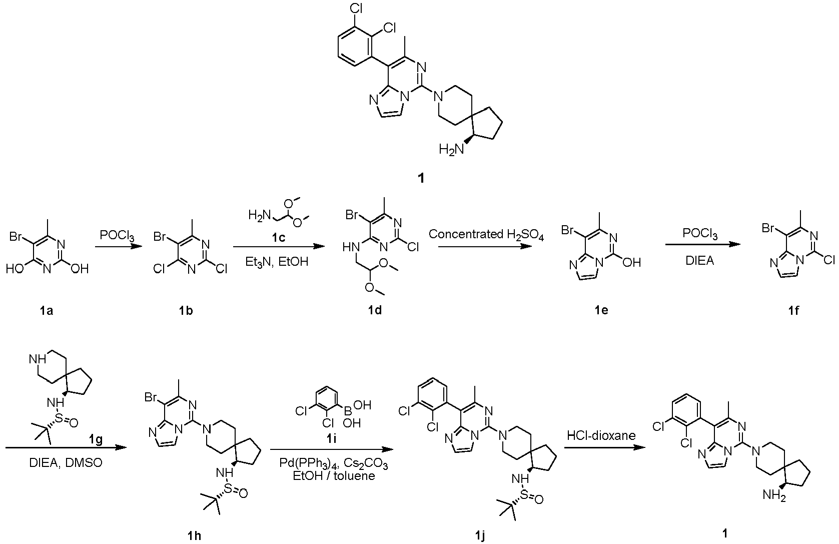

### Step 1

### 5-Bromo-2,4-dichloro-6-methylpyrimidine 1b

5-Bromo-6-methylpyrimidine-2,4-diol **1a** (1.5 g, 7.32 mmol) was dissolved in 8 mL of phosphorus oxychloride. 0.3 mL of N,N-dimethylformamide was added, and the reaction solution was warmed up to 115°C and stirred for 4 hours. After the reaction was completed, the reaction solution was concentrated under reduced pressure, and added to 20 mL of ice-water mixture. The reaction solution was extracted with ethyl acetate (10 mL×3). The organic phases were combined, washed with saturated sodium chloride solution (5 mL), dried over anhydrous sodium sulfate, and filtered. The filtrate was collected, and concentrated under reduced pressure to obtain the title compound **1b** (950 mg, yield: 54%) as a yellow solid.
MS(ESI) m/z 242.8 [M+H]⁺
¹H NMR (400MHz, CDCl₃) δ 2.72 (s, 3H).

### Step 2

### 5-Bromo-2-chloro-N-(2,2-dimethoxyethyl)-6-methylpyrimidin-4-amine 1d

Compound **1b** (940 mg, 3.89 mmol) and 2,2-dimethoxyethylamine **1c** (817 mg, 7.77 mmol) were dissolved in 15 mL of ethanol. 1.1 mL of triethylamine (785 mg, 7.77 mmol) was added at 0°C, and the reaction solution was stirred at room temperature for 12 hours. After the reaction was completed, the reaction solution was concentrated under reduced pressure. Water was added, and the reaction solution was extracted with ethyl acetate (10 mL×3). The organic phases were combined, dried over anhydrous sodium sulfate, and filtered. The filtrate was collected, and concentrated under reduced pressure. The residue was purified by silica gel column chromatography with petroleum ether and ethyl acetate as an eluent to obtain the title compound **1d** (790 mg, yield: 65%) as a white solid.
MS(ESI) m/z 311.8 [M+H]⁺
¹H NMR (400MHz, CDCl₃) δ 5.82 (s, 1H), 4.49 (t, J = 4.8 Hz, 1H), 3.66 (t, J = 5.6 Hz, 2H), 3.45 (s, 6H), 2.48 (s, 3H).

### Step 3

### 8-Bromo-7-imidazo[l,2-c]pyrimidin-5-ol le

Compound **1d** (780 mg, 2.51 mmol) was dissolved in 8 mL of concentrated sulfuric acid, and reacted at 65°C for 2 hours. After the reaction was completed, 100 mL of ice-water mixture was added, and saturated sodium hydroxide solution was added until pH=6. The reaction solution was extracted with a mixed solvent of dichloromethane and isopropanol (50 mL×3, volume ratio: 3:1). The organic phases were combined, washed with saturated sodium chloride solution (150 mL), dried over anhydrous sodium sulfate, and filtered. The filtrate was collected, and concentrated under reduced pressure to obtain the title compound **1e** (560 mg, yield: 92%) as a yellow solid.
MS(ESI) m/z 227.9, 229.9 [M+H]⁺
¹H NMR (400MHz, MeOH-*d*₄) δ 7.81 (d, J = 1.6 Hz, 1H), 7.36 (d, J = 1.6 Hz, 1H), 2.43 (s, 3H).

### Step 4

### 8-Bromo-5-chloro-7-methylimidazo[1,2-c]pyrimidine 1f

N,N-Diisopropylethylamine (5.6 mL, 5.57 mmol) was added to a suspension of compound **1e** (300 mg, 1.32 mmol) and phosphorus oxychloride (7.58 g). The reaction solution was reacted at 110°C for 3 hours. After the reaction was completed, the reaction solution was concentrated under reduced pressure. 50 mL of saturated sodium bicarbonate solution was added, and the reaction solution was extracted with ethyl acetate (50 mL×3). The organic phases were combined, washed with saturated sodium chloride solution (200 mL), dried over anhydrous sodium sulfate, and filtered. The filtrate was collected, and concentrated under reduced pressure to obtain the title compound **1f** (210 mg, yield: 36%) as a yellow solid.
MS(ESI) m/z 247.9 [M+H]⁺
¹H NMR (400MHz, DMSO-*d*₆) δ 8.10 (s, 1H), 7.75 (s, 1H), 2.56 (s, 3H).

### Step 5

### (R)-N-(R)-8-(8-Bromo-7-methylimidazo[1,2-c]pyrimidin-5-yl)-8-azaspiro[4.5]decan-1-yl)-2-methylpropane-2-sulfinamide 1h

Compound **1f** (180 mg, 0.73 mmol), (R)-2-methyl-N-((R)-8-azaspiro[4.5]decan-1-yl)propane-2-sulfinamide **1g** (268 mg, 0.73 mmol, prepared according to the method disclosed in the patent application "WO2016203406 A1") and N,N-diisopropylethylamine (0.36 mL, 2.19 mmol) were dissolved in 5 mL of dimethyl sulfoxide. The reaction solution was reacted at 90°C for 30 minutes. After the reaction was completed, 20 mL of ethyl acetate and 40 mL of water were added, and the reaction solution was extracted with ethyl acetate (20 mL×3). The organic phases were combined, washed with saturated sodium chloride solution (100 mL), dried over anhydrous sodium sulfate, and filtered. The filtrate was collected, and concentrated under reduced pressure. The residue was purified by silica gel chromatography with dichloromethane and methanol as an eluent to obtain the title compound **1h** (200 mg, yield: 56%) as a white solid.
MS(ESI) m/z 468.0, 470.0 [M+H]⁺
¹H NMR (400MHz, MeOH-*d*₄) δ 7.77 (d, *J* = 1.6 Hz, 1H), 7.54 (d, *J* = 1.6 Hz, 1H), 5.02 (d, *J* = 8.4 Hz, 1H), 3.84-3.77 (m, 2H), 3.37-3.33 (m, 1H), 3.17-3.03 (m, 2H), 2.55 (s, 3H), 2.21-2.15 (m, 1H), 2.09-1.89 (m, 3H), 1.81-1.64 (m, 3H), 1.62-1.54 (m, 1H), 1.53-1.39 (m, 2H), 1.26 (s, 9H).

### Step 6

### (R)-N-((R)-8-(8-(2,3-Dichlorophenyl)-7-methylimidazo[1,2-c]pyrimidin-5-yl)-8-azaspir o[4.5]decan-1-yl)-2-methylpropane-2-sulfinamide 1j

Compound **1h** (170 mg, 0.36 mmol), 2,3-dichlorophenylboronic acid **1i** (138 mg, 0.73 mmol), cesium carbonate (355 mg, 1.09 mmol) and tetrakis(triphenylphosphine)palladium (42 mg, 0.036 mmol) were dissolved successively in a mixed solution of 2 mL of toluene and 2 mL of ethanol under a nitrogen atmosphere. The reaction solution was reacted at 120°C for 1 hour. The reaction solution was cooled to room temperature. 10 mL of ethyl acetate and 10 mL of water were added, and the reaction solution was extracted with ethyl acetate (10 mL×3). The organic phases were combined, washed with saturated sodium chloride solution (50 mL), dried over anhydrous sodium sulfate, and filtered. The filtrate was collected, and concentrated under reduced pressure. The residue was purified by silica gel chromatography with dichloromethane and methanol as an eluent to obtain the title compound **1j** (37 mg, yield: 19%) as a yellow oil.
MS(ESI) m/z 534.4 [M+H]⁺
¹H NMR (400MHz, MeOH-*d*₄) δ 7.83-7.25 (m, 5H), 3.92-3.75 (m, 2H), 3.22-2.95 (m, 2H), 2.18 (s, 3H), 2.11-1.18 (m, 20H).

### Step 7

### (R)-8-(8-(2,3-Dichlorophenyl)-7-methylimidazo[1,2-c]pyrimidin-5-yl)-8-azaspiro[4.5]d ecan-1-amine 1

Compound **1j** (25 mg, 0.047 mmol) was dissolved in 2 mL of 1,4-dioxane. 1 mL of 4M solution of hydrogen chloride in 1,4-dioxane was added at 0°C, and the reaction solution was stirred at room temperature for 30 minutes. 10 mL of ethyl acetate was added, then the reaction solution was filtered, and washed with ethyl acetate (10 mL×3). The resulting solid was dissolved in 10 mL of water, and saturated sodium bicarbonate solution was added until pH = 9. The mixture was extracted with chloroform (20 mL×2). The organic phase was washed with saturated sodium bicarbonate solution (10 mL) and saturated sodium chloride solution (20 mL×2), dried over anhydrous sodium sulfate, and filtered. The filtrate was collected, and concentrated under reduced pressure to obtain the title compound **1** (9.3 mg, yield: 42%).
MS(ESI) m/z 430.1 [M+H]⁺
¹H NMR (400MHz, MeOH-*d*₄) δ 7.70-7.62 (m, 2H), 7.47-7.39 (m, 2H), 7.31 (d, *J* = 1.6 Hz, 1H), 3.97-3.81 (m, 2H), 3.29-3.18 (m, 2H), 3.11-3.04 (m, 1H), 2.19 (s, 3H), 2.17-2.10 (m, 1H), 2.06-1.90 (m, 3H), 1.88-1.82 (m, 1H), 1.81-1.70 (m, 2H), 1.66-1.50 (m, 3H).

### Example 2

### (3S,4S)-8-(8-(2,3-Dichlorophenyl)-7-methylimidazo[1,2-c]pyrimidin-5-yl)-3-methyl-2-oxa-8-azaspiro[4.5]decan-4-amine

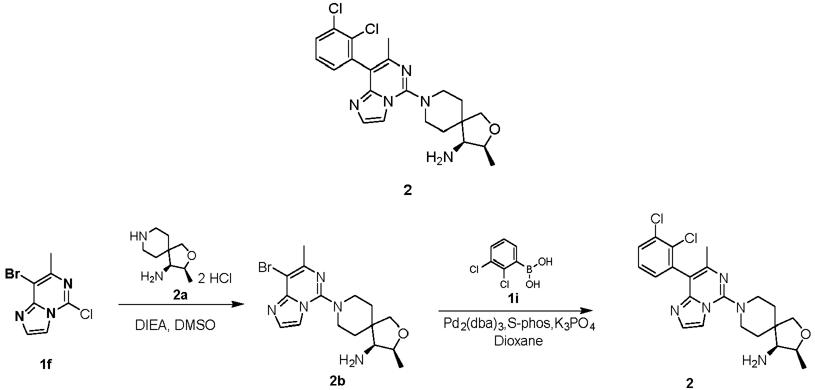

### Step 1

### (3S,4S)-8-(8-Bromo-7-methylimidazo[1,2-c]pyrimidin-5-yl)-3-methyl-2-oxa-8-azaspiro [4.5]decan-4-amine 2b

Compound **1f** (150 mg, 0.53 mmol), (3S,4S)-3-methyl-2-oxa-8-azaspiro[4.5]decan-4-amine 2a (154 mg, 0.63 mmol, prepared according to the method disclosed in the patent application " WO2015107495 A1") and N,N-diisopropylethylamine (31 mg, 1.06 mmol) were dissolved in 3 mL of dimethyl sulfoxide. The reaction solution was reacted at 90°C for 1 hour. After the reaction was completed, 15 mL of ethyl acetate and 30 mL of water were added, and the reaction solution was extracted with ethyl acetate (10 mL×3). The organic phases were combined, washed with saturated sodium chloride solution (10 mL×3), dried over anhydrous sodium sulfate, and filtered. The filtrate was collected, and concentrated under reduced pressure. The residue was purified by silica gel chromatography with dichloromethane and methanol as an eluent to obtain the title compound **2b** (150 mg, yield: 75%) as a yellow solid.
MS(ESI) m/z 380.1, 382.1 [M+H]⁺
¹H NMR (400MHz, CDCl₃) δ 7.59 (s, 1H), 7.42 (s, 1H), 4.23-4.16 (m, 1H), 3.83 (d, J = 8.8 Hz, 1H), 3.72 (d, J = 8.8 Hz, 1H), 3.64-3.55 (m, 2H), 3.30-3.22 (m, 1H), 3.20-3.14 (m, 1H), 3.04 (d, J = 4.4 Hz, 1H), 2.57 (s, 3H), 2.03-1.98 (m, 1H), 1.93-1.86 (m, 1H), 1.84-1.74 (m, 2H), 1.26 (d, J = 6.4 Hz, 3H).

### Step 2

### (3S,4S)-8-(8-(2,3-Dichlorophenyl)-7-methylimidazo[1,2-c]pyrimidin-5-yl)-3-methyl-2-oxa-8-azaspiro[4.5]decan-4-amine 2

Compound **2b** (50 mg, 0.131 mmol), 2,3-dichlorophenylboronic acid **1i** (30 mg, 0.158 mmol), potassium phosphate (55.6 mg, 0.262 mmol), tris(dibenzylideneacetone)dipalladium (5.95 mg, 0.007 mmol) and 2-dicyclohexylphosphino-2',6'-dimethoxybiphenyl were suspended successively in 1 mL of 1,4-dioxane under a nitrogen atmosphere. The reaction solution was reacted at 100°C for 1 hour. The reaction solution was cooled to room temperature. 5 mL of ethyl acetate and 4 mL of water were added, and the reaction solution was extracted with ethyl acetate (8 mL×3). The organic phases were combined, washed with saturated sodium chloride solution (8 mL), dried over anhydrous sodium sulfate, and filtered. The filtrate was collected, and concentrated under reduced pressure. The residue was purified by silica gel chromatography with ethyl acetate, methanol and ammonia as an eluent to obtain the title compound 2 (9.7 mg, yield: 16%).
MS(ESI) m/z 446.1 [M+H]⁺
¹H NMR (400MHz, CDCl₃) δ 7.57-7.50 (m, 2H), 7.41 (d, J = 1.2 Hz, 1H), 7.32 (t, J = 7.6 Hz, 1H), 7.28 (d, J = 1.6 Hz, 1H), 4.25-4.19 (m, 1H), 3.86 (d, J = 8.8 Hz, 1H), 3.76 (d, J = 8.8 Hz, 1H), 3.72-3.63 (m, 2H), 3.40-3.18 (m, 2H), 3.07 (d, J = 4.8 Hz, 1H), 2.23 (s, 3H), 2.10-2.01 (m, 1H), 1.98-1.77 (m, 3H), 1.27 (d, J = 6.4 Hz, 3H).

### Example 3

### a(R)-(3S,4S)-8-(8-(2,3-Dichlorophenyl)-7-methylimidazo[1,2-c]pyrimidin-5-yl)-3-meth yl-2-oxa-8-azaspiro[4.5]decan-4-amine atropisomer 1

### Example 4

### a(S)-(3S,4S)-8-(8-(2,3-Dichlorophenyl)-7-methylimidazo[1,2-c]pyrimidin-5-yl)-3-meth yl-2-oxa-8-azaspiro[4.5]decan-4-amine atropisomer 2

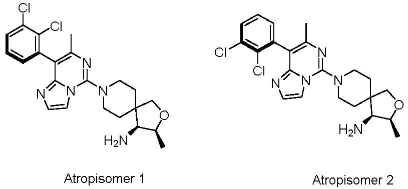

(3S,4S)-8-(8-(2,3-Dichlorophenyl)-7-methylimidazo[1,2-c]pyrimidin-5-yl)-3-meth yl-2-oxa-8-azaspiro[4.5]decan-4-amine (17 mg) obtained in Example **2** was resolved by supercritical fluid chromatography (mobile phase: 45% EtOH ⁺ 0.1% NH₃H₂O 155% scCO₂, flow rate: 80 ml/min) on a chiral column (DAICEL CHIRALPAK IC (250mm*30mm, 10 µm)). 5.2 mg of an atropisomer (d.e. 98.91%) was obtained from the first elution peak. ¹H NMR (400MHz, CDCl₃) δ 7.72-7.60 (m, 2H), 7.47-7.39 (m, 2H), 7.34-7.25 (m, 1H), 4.95-4.91 (m, 1H), 4.42-4.23 (m, 1H), 3.96-3.88 (m, 1H), 3.80-3.68 (m, 2H), 3.55-3.42 (m, 1H), 3.30-3.07 (m, 2H), 2.18 (s, 3H), 2.05-1.95 (m, 2H), 1.89-1.72 (m, 2H), 1.5 (d, J = 6.8 Hz, 3H). Chiral analysis method: Chiralpak IC-3 100 7.47-7.39 (m, 2H), mobile phase: A- supercritical carbon dioxide, B- EtOH ⁺ 0.05% DEA, flow rate: 2.8 ml/min, isocratic elution 40% B. Retention time (RT): 1.495 minutes.

4.4 mg of an atropisomer (d.e. 99.33%) was obtained from the second elution peak. ¹H NMR (400MHz, CDCl₃) δ 7.72-7.60 (m, 2H), 7.46-7.39 (m, 2H), 7.32-7.28 (m, 1H), 5.01-4.90 (m, 1H), 4.45-4.23 (m, 1H), 3.97-3.87 (m, 1H), 3.80-3.65 (m, 2H), 3.55-3.44 (m, 1H), 3.27-3.07 (m, 2H), 2.18 (s, 3H), 2.05-1.94 (m, 2H), 1.87-1.70 (m, 2H), 1.25 (d, J = 6.8 Hz, 3H). Chiral analysis method: Chiralpak IC-3 100 7.46-7.39 (m, 2H), mobile phase: A- supercritical carbon dioxide, B- EtOH ⁺ 0.05% DEA, flow rate: 2.8 ml/min, isocratic elution 40% B. Retention time (RT): 2.716 minutes.

### Example 5

### (R)-8-(8-(2,3-Dichloropyridin-4-yl)-7-methylimidazo[1,2-c]pyrimidin-5-yl)-8-azaspiro[ 4.5]decan-1-amine

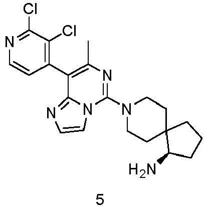

In accordance with the synthetic steps of Example 1, compound **1i** was replaced with compound (2,3-dichloropyridin-4-yl)boronic acid, accordingly, the compound of Example 5 was prepared.
MS(ESI) m/z 431.0 [M+H]⁺
¹H NMR (400MHz, MeOH-d4) δ 8.42 (d, J= 4.8 Hz, 1H), 7.72 (d, J=2.0 Hz, 1H), 7.46 (d, J=1.6 Hz, 1H), 7.42 (d, J=4.8 Hz, 1H), 3.97-3.84 (m, 2H), 3.29-3.15 (m, 2H), 2.89 (t, J=7.2 Hz, 1H), 2.22 (s, 3H), 2.13-2.04 (m, 1H), 2.01-1.87 (m, 3H), 1.83-1.74 (m, 1H),1.73-1.67 (m, 1H), 1.66-1.58 (m, 1H), 1.55-1.41 (m, 3H).

### Example 6

### (R)-8-(8-(3-Chloro-2-methoxypyridin-4-yl)-7-methylimidazo[1,2-c]pyrimidin-5-yl)-8-a zaspiro[4.5]decan-1-amine

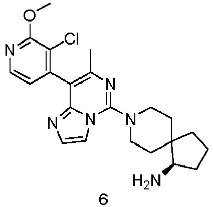

In accordance with the synthetic steps of Example 1, compound **1i** was replaced with compound (3-chloro-2-methoxypyridin-4-yl)boronic acid, accordingly, the compound of Example 6 was prepared.
MS(ESI) m/z 427.2 [M+H]⁺
¹H NMR (400MHz, MeOH-d4) δ 8.16 (d, J=5.2 Hz, 1H), 7.69 (s, 1H), 7.45 (s, 1H), 6.95 (d, J=5.2 Hz, 1H), 4.06 (s, 3H), 3.94-3.82 (m, 2H), 3.27-3.16 (m, 2H), 3.01 (t, J=7.2 Hz, 1H), 2.21 (s, 3H), 2.16-2.08 (m, 1H), 2.00-1.91 (m, 2H), 1.88-1.77 (m, 2H), 1.74-1.64 (m, 2H), 1.62-1.45 (m, 3H).

### Example 7

### (R)-8-(8-(2-Chloro-4-(difluoromethoxy)phenyl)-7-methylimidazo[1,2-clpyrimidin-5-yl) -8-azaspiro[4.5]decan-1-amine

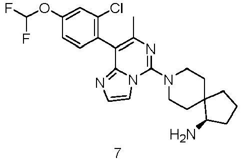

In accordance with the synthetic steps of Example 1, compound **1i** was replaced with compound (2-chloro-4-(difluoromethoxy)phenyl)boronic acid, accordingly, the compound of Example 7 was prepared.
MS(ESI) m/z 462.2 [M+H]⁺
¹H NMR (400MHz, MeOH-d4) δ 7.68 (d, J = 1.2 Hz, 1H), 7.44 (d, J = 1.2 Hz, 1H), 7.24 (dd, J = 2.0, 8.4 Hz, 1H), 6.97 (t, J = 73.6 Hz, 1H), 3.90-3.80 (m, 2H), 3.26-3.13 (m, 2H), 2.92-2.88 (m, 1H), 2.19 (s, 3H), 2.10-1.95 (m, 2H), 1.95-1.87 (m, 2H), 1.84-1.75 (m, 1H), 1.74-1.68 (m, 1H), 1.66-1.58 (m, 1H), 1.56-1.41 (m, 3H).

### Example 8

### (S)-1'-(8-(2,3-Dichlorophenyl)-7-methylimidazo[1,2-c]pyrimidin-5-yl)-1,3-dihydrospiro [indene-2,4'-piperidin]-1 -amine

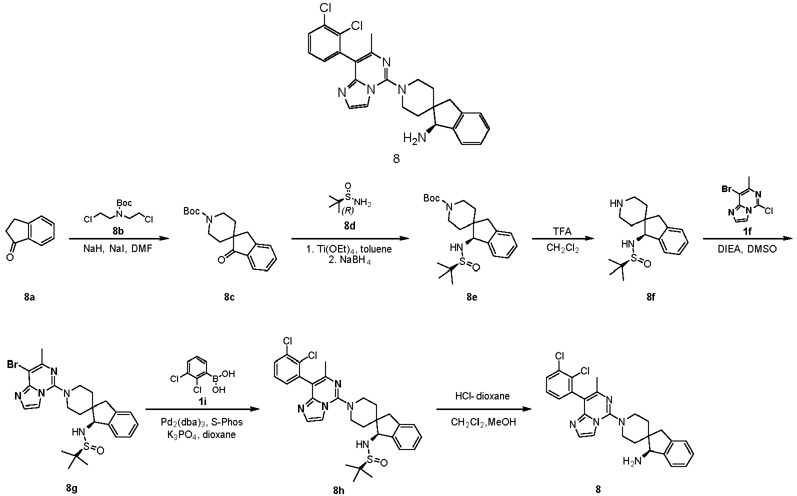

### Step 1

### Tert-butyl 1-oxo-1,3-dihydrospiro[indene-2,4'-piperidine]-1'-carboxylate 8c

Compound **8a** (10 g, 75.66 mmol) was dissolved in DMF (300 mL) under a nitrogen atmosphere, followed by the addition of sodium hydride (60% mixture with kerosene, 9.1 g, 226.98 mmol) at 10°C. The reaction system was stirred at room temperature for 30 minutes. Compound *tert*-butyl bis(2-chloroethyl)carbamate 8b (18.3 g, 75.66 mmol) and sodium hydride (22.6 g, 151.32 mmol) were added. The reaction solution was reacted at room temperature for 1 hour, and heated to 50°C for 12 hours. After the reaction was completed, saturated aqueous ammonium chloride solution (50 mL) was added, and the reaction solution was extracted with ethyl acetate (200 mL). The organic phases were combined, washed with water (80 mL×2) and saturated sodium chloride solution (80 mL×2), dried over anhydrous sodium sulfate, and concentrated under reduced pressure. The resulting crude product was purified by silica gel chromatography with petroleum ether and ethyl acetate as an eluent to obtain compound **8c** (1.9 g, yield: 8.34%) as a brown solid.
MS(ESI) m/z 246.0 [M+H-56]⁺
¹H NMR: (400 MHz, CDCl₃) δ 7.78 (d, *J* = 8.0 Hz, 1H), 7.62 (t, *J* = 7.2 Hz, 1H), 7. 48 (d, *J* = 7.2 Hz, 1H), 7.40 (t, *J* = 7.2 Hz, 1H), 4.15-4.13 (m, 2H), 3.08 (s, 2H), 3.05-2.99 (m, 2H), 1.92 (dt, *J* = 4.4 Hz, *J* = 13.2 Hz, 2H), 1.49 (s, 9H), 1.40-1.35 (m, 2H).

### Step 2

### Tert-butyl

### (S)-1-((S)-tert-butylsulfinylamino)-1,3-dihydrospiro[indene-2,4'-piperidine]-1'-carboxyl ate 8e

Compound **8c** (1.60 g, 5.31 mmol) was dissolved in anhydrous toluene (20 mL), followed by the addition of titanium tetraethoxide (2.42 g, 10.62 mmol). The reaction solution was stirred at room temperature for 20 minutes. Compound (R)-2-methylpropane-2-sulfinamide **8d** (965 mg, 7.96 mmol) was added, and the reaction system was reacted at 90°C for 15 hours. After cooling to 0°C, lithium borohydride (139 mg, 6.37 mmol) was added and the reaction solution was reacted for 30 minutes. After the reaction was completed, methanol (8 mL) was added dropwise at 0°C. Water (20 mL) and ethyl acetate (30 mL) were added, and the reaction solution was stirred for 5 minutes. Suspended matter was filtered out by diatomaceous earth, and washed with ethyl acetate (50 mL). The reaction solution was extracted with ethyl acetate (70 mL×2). The organic phases were combined, washed with saturated sodium chloride solution (30 mL×2), dried over anhydrous sodium sulfate, and concentrated under reduced pressure. The resulting crude product was purified by silica gel chromatography with petroleum ether and ethyl acetate as an eluent to obtain compound **8e** (530 mg, yield: 24.5%) as a yellow solid.
MS(ESI) m/z 307.2 [M+H-Boc]⁺
¹H NMR (400MHz, MeOH-*d*4) δ 7.31 (d, J = 5.6 Hz, 1H), 7.22-7.18 (m, 3H), 5.56 (d, J = 8.0 Hz, 1H), 4.48 (d, J = 10.4 Hz, 1H), 4.02-3.97 (m, 1H), 3.13 (d, J = 15.6 Hz, 1H), 3.08-2.95 (m, 2H), 2.73 (d, J = 16.0 Hz, 1H), 2.05-1.96 (m, 1H), 1.73-1.72 (m, 1H), 1.54-1.52 (m, 1H), 1.46 (s, 9H), 1.31 (s, 9H).

### Step 3

### (S)-N-((S)-1,3-Dihydrospiro[indene-2,4'-piperidin]-1-yl)-2-methylpropane-2-sulfinamid e 8f

Compound **8e** (460 mg, 1.13 mmol) was dissolved in dichloromethane (5 mL), followed by the addition of trifluoroacetic acid (1 mL) at 0°C. The reaction solution was stirred at room temperature for 1 hour. The reaction solution was concentrated under reduced pressure to obtain the crude product, and saturated aqueous sodium bicarbonate solution was added until pH = 7-8. The reaction solution was extracted with dichloromethane (10 mL×3). The organic phases were combined, washed with saturated sodium chloride solution (8 mL), dried over anhydrous sodium sulfate, and concentrated under reduced pressure to obtain compound **8f** (230 mg, yield: 66%) as a yellow oil.
MS(ESI) m/z 307.2 [M+H]⁺

### Step 4

### (S)-N-((S)-1'-(8-Bromo-7-methylimidazo[1,2-c]pyrimidin-5-yl)-1,3-dihydrospiro[inden e-2,4'-piperidin]-1-yl)-2-methylpropane-2-sulfinamide 8g

Compound **8f** (123 mg, 0.50 mmol) and compound **1f** (230 mg, 0.75 mmol) were dissolved in dimethyl sulfoxide (3 mL) under a nitrogen atmosphere, followed by the addition of diisopropylethylamine (129 mg, 1.0 mmol). The reaction solution was stirred at 90°C for 1 hour. Ethyl acetate (20 mL) and water (10 mL) were added, and the reaction solution was extracted with ethyl acetate (10 mL×2). The organic phases were combined, washed with water (8 mL×2) and saturated sodium chloride solution (8 mL×2), dried over anhydrous sodium sulfate, and concentrated under reduced pressure. The resulting crude product was purified by silica gel chromatography with petroleum ether and ethyl acetate as an eluent to obtain compound **8g** (250 mg, yield: 97%) as a white solid.
MS(ESI) m/z 516.1, 518.1 [M+H]⁺

### Step 5

### (S)-N-((S)-1'-(8-(2,3-Dichlorophenyl)-7-methylimidazo[1,2-c]pyrimidin-5-yl)-1,3-dihy drospiro[indene-2,4'-piperidin]-1-yl)-2-methylpropane-2-sulfinamide 8h

Compound **8g** (100 mg, 0.19 mmol) and compound **1i** (55.3 mg, 0.29 mmol) were dissolved in 1,4-dioxane (2 mL) under a nitrogen atmosphere, followed by the addition of potassium carbonate (161 mg, 0.76 mmol) at room temperature. Tris(dibenzylideneacetone)dipalladium (8.7 mg, 0.0095 mmol) and 2-dicyclohexylphosphino-2',6'-dimethoxybiphenyl (7.8 mg, 0.019 mmol) were added, and the reaction solution was heated to100°C and stirred for 1 hour. Ethyl acetate (10 mL) and water (8 mL) were added, and the reaction solution was extracted with ethyl acetate (8 mL×2). The organic phases were combined, washed with saturated sodium chloride solution (5 mL), dried over anhydrous sodium sulfate, and concentrated under reduced pressure. The resulting crude product was purified by silica gel chromatography with petroleum ether and ethyl acetate as an eluent to obtain compound **8h** (15 mg, yield: 13.5%) as a yellow solid.
MS(ESI) m/z 582.3 [M+H]⁺

### Step 6

### (S)-1'-(8-(2,3-Dichlorophenyl)-7-methylimidazo[1,2-c]pyrimidin-5-yl)-1,3-dihydrospiro [indene-2,4'-piperidin]-1-amine 8

Compound **8h** (15 mg, 0.026 mmol) was dissolved in dichloromethane (2 mL) and methanol (0.2 mL), followed by the addition of a solution of hydrogen chloride in 1,4-dioxane (0.5 mL, 4 N) at 0°C. The reaction solution was reacted at room temperature for 20 minutes. The reaction solution was concentrated under reduced pressure, and saturated aqueous sodium bicarbonate solution was added to adjust pH = 7-8. The reaction solution was extracted with dichloromethane (8 mL×3). The organic phases were combined, washed with saturated sodium chloride solution (5 mL), dried over anhydrous sodium sulfate, and concentrated under reduced pressure to obtain compound **8** (6.3 mg, yield: 50.7%).
MS(ESI) m/z 478.1 [M+H]⁺
¹H NMR (400MHz, MeOH-*d*₄) δ 7.72 (s, 1H), 7.65 (d, J = 8 Hz, 1H), 7.45-7.41 (m, 3H), 7.32-7.24 (m, 4H), 4.97-4.95 (m, 1H), 4.86-4.85 (m, 1H), 4.13 (s, 1H), 3.91-3.88 (m, 2H), 3.20 (d, J = 8 Hz, 1H), 2.93 (d, J = 8 Hz, 1H), 2.20 (s, 3H), 2.09-2.06 (m, 2H), 1.72 (d, J = 13.6 Hz, 1H), 1.62 (d, J = 13.6 Hz, 1H).

### Example 9

### (3S,4S)-8-(8-(2,3-Dichlorophenyl)-7-methyl-[1,2,4]triazolo[4,3-c]pyrimidin-5-yl)-3-me thyl-2-oxa-8-azaspiro[4.5]decan-4-amine

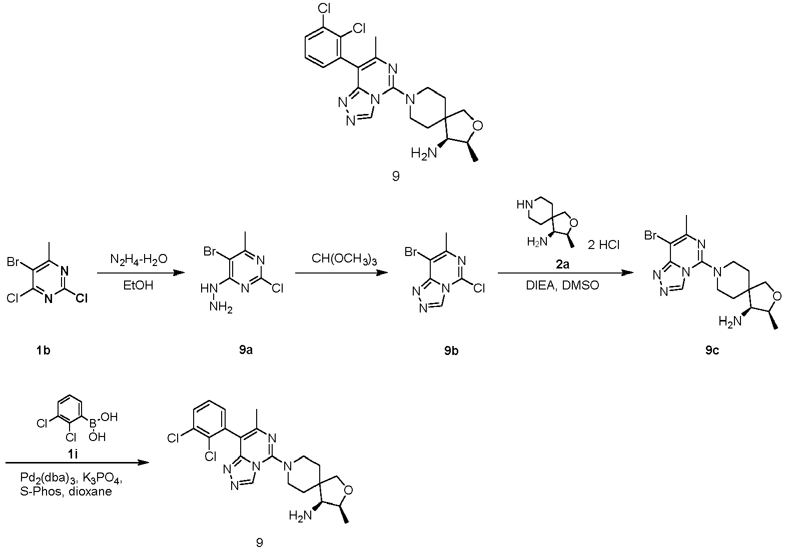

### Step 1

### 5-Bromo-2-chloro-4-hydrazineyl-6-methylpyrimidine 9a

Compound **1b** (3.0 g, 12.40 mmol) was dissolved in ethanol (30 mL), followed by the addition of hydrazine hydrate (1.86 g, 37.21 mmol). The reaction solution was stirred at room temperature for 4 hours, and then filtered. The filter cake was washed with ethanol (10 mL×3). The resulting solid was dried under vacuum to obtain compound **9a** (2.8 g, yield: 95%) as a yellow solid.
MS(ESI) m/z 236.7, 238.7 [M+H]⁺
¹H NMR (400MHz, MeOH-*d*₄) δ 2.43 (s, 3H).

### Step 2

8-Bromo-5-chloro-7-methyl-[1,2,4]triazolo[4,3-c]pyrimidine **9b** Compound **9a** (1.4 g, 5.90 mmol) was dissolved in trimethyl orthoformate (20 mL), and the reaction solution was reacted at 100°C for 2 hours. After the reaction was completed, the reaction solution was concentrated under vacuum, and the resulting crude product (1.4 g) was purified by silica gel chromatography with petroleum ether and ethyl acetate as an eluent to obtain compound **9b** (330 mg, yield: 23%) as a white solid.
MS(ESI) m/z 246.4, 248.4 [M+H]⁺

### Step 3

### (3S,4S)-8-(8-Bromo-7-methyl-[1,2,4]triazolo[4,3-c]pyrimidin-5-yl)-3-methyl-2-oxa-8-a zaspiro[4.5]decan-4-amine 9c

Compound **9b** (120 mg, 0.48 mmol) and DIEA (310 mg, 2.40 mmol) were dissolved in DMSO (4.0 mL), followed by the addition of compound **2a** (140 mg, 0.58 mmol). The reaction solution was reacted at 90°C for 1 hour. After the reaction was completed, water (50 mL) was added, and the reaction solution was extracted with ethyl acetate (25 mL×3). The organic phases were combined, washed with saturated sodium chloride solution (20 mL×3), dried over anhydrous sodium sulfate, and concentrated under reduced pressure. The resulting residue was purified by silica gel chromatography with dichloromethane and methanol as an eluent to obtain compound **9c** (110 mg, yield: 60%) as a yellow solid.
MS(ESI) m/z 381.1, 383.1 [M+H]⁺
¹H NMR (400MHz, CDCl₃) δ 8.69 (s, 1H), 4.21-4.15 (m, 1H), 3.82 (d, J = 8.8 Hz, 1H), 3.75-3.62 (m, 3H), 3.44-3.25 (m, 2H), 3.04 (d, J = 4.4 Hz, 1H), 2.53 (s, 3H), 2.10-1.98 (m, 1H), 1.95-1.74 (m, 3H), 1.24 (d, J = 6.0 Hz, 3H).

### Step 4

### (3S,4S)-8-(8-(2,3-Dichlorophenyl)-7-methyl-[1,2,4]triazolo[4,3-c]pyrimidin-5-yl)-3-me thyl-2-oxa-8-azaspiro[4.5]decan-4-amine 9

Compound **9c** (50 mg, 0.13 mmol), compound **1i** (30 mg, 0.16 mmol) and potassium phosphate (55.2 mg, 0.26 mmol) were suspended in 1,4-dioxane (2 mL) under a nitrogen atmosphere. 2-Dicyclohexylphosphino-2',6'-dimethoxy-biphenyl (S-Phos, 5.3 mg, 0.013 mmol) and tris(dibenzylideneacetone)dipalladium (Pd2(dba)3, 5.5 mg, 0.006 mmol) were added. The reaction solution was reacted under condition 10006 for 1 hour. The reaction solution was filtered, and the filtrate was concentrated under reduced pressure. The residue was purified by silica gel chromatography with ethyl acetate, methanol and ammonia as an eluent to obtain compound 9 (12.1 mg, yield: 20.6%).
MS(ESI) m/z 447.1 [M+H]⁺
¹H NMR (400MHz, CDCl₃) δ 8.70 (s, 1H), 7.57 (dd, *J* = 7.6 Hz, *J* = 1.6 Hz, 1H), 7.36-7.27 (m, 2H), 4.26-4.18 (m, 1H), 3.89-3.70 (m, 4H), 3.52-3.31 (m, 2H), 3.08 (br s, 1H), 2.23 (s, 3H), 2.11-2.01 (m, 1H), 1.98-1.77 (m, 3H), 1.27 (d, *J* = 6.4 Hz, 3H).

### Example 10

### (3S,4S)-8-(8-(2-Chloro-6-fluorophenyl)-7-methylimidazo[1,2-c]pyrimidin-5-yl)-3-meth yl-2-oxa-8-azaspiro[4.5]decan-4-amine

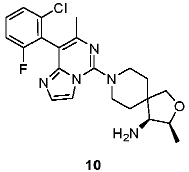

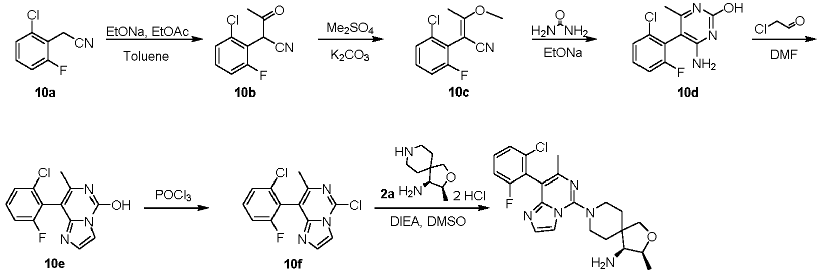

### Step 1

### 2-(2-Chloro-6-fluorophenyl)-3 -oxobutanenitrile 10b

2-(2-Chloro-6-fluorophenyl)acetonitrile **10a** (2.0 g, 11.8 mmol) was dissolved in 3.6 mL of ethyl acetate, followed by the addition of sodium ethoxide (6.8 g, 11.8 mmol). The reaction solution was stirred at 85°C for 5 hours. After the reaction was completed, 30 mL of water was added to the reaction solution, and saturated aqueous citric acid solution was added to adjust the pH to 4 to 5. The reaction solution was extracted with ethyl acetate (30 mL×3). The organic phases were combined, washed with saturated sodium chloride solution (40 mL), dried over anhydrous sodium sulfate, and filtered. The filtrate was collected, and concentrated under reduced pressure to obtain compound **10b** (2.2 g, yield: 88%) as a yellow solid.
¹H NMR: (400MHz, CDCl₃) δ 7.42-7.36 (m, 1H), 7.34-7.30 (m, 1H), 7.17-7.11 (m, 1H), 5.18 (s, 1H), 2.43 (s, 3H).

### Step 2

### (Z)-2-(2-Chloro-6-fluorophenyl)-3-methoxybut-2-enenitrile 10e

Compound **10b** (2.0 g, 9.45 mmol) was dissolved in 30 mL of tetrahydrofuran under a nitrogen atmosphere, followed by the addition of potassium carbonate (2.61 g, 18.9 mmol) and dimethyl sulfate (1.79 mL, 18.9 mmol). The reaction solution was reacted at room temperature for 10 hours. After the reaction was completed, the reaction solution was concentrated. 30 mL of water was added, and the reaction solution was extracted with ethyl acetate (30 mL). The organic phases were combined, washed with saturated sodium chloride solution (40 mL×2), dried over anhydrous sodium sulfate, and filtered. The filtrate was collected, and concentrated under reduced pressure. The residue was purified by silica gel chromatography with petroleum ether and ethyl acetate as an eluent to obtain the title compound **10c** (2.6 g, yield: 97%) as a yellow oil.
MS(ESI) m/z = 225.8 [M+H]⁺
¹H NMR: (400 MHz, MeOH-d4) δ 7.34-7.28 (m, 1H), 7.22-7.15 (m, 1H), 7.14-7.08 (m, 1H), 3.81 (s, 3H), 2.46 (s, 3H).

### Step 3

### 4-Amino-5-(2-chloro-6-fluorophenyl)-6-methylpyrimidin-2-ol 10d

Sodium ethoxide (0.90 g, 13.3 mmol) and urea (0.40 g, 6.6 mmol) were added to a solution of compound **10c** (1.0 g, 4.4 mmol) in ethanol (5 mL). The reaction solution was reacted at 80°C for 12 hours. After the reaction was completed, the reaction solution was filtered. 2M hydrochloric acid was added to the filtrate to adjust pH = 6, and the reaction solution was extracted with dichloromethane and isopropanol (20 mL×3, v/v = 3:1). The organic phases were combined, dried over anhydrous sodium sulfate, and filtered. The filtrate was collected, and concentrated under reduced pressure. The residue was purified by silica gel column chromatography with dichloromethane and methanol as an eluent to obtain compound **10d** (0.42 g, yield: 37%) as a yellow solid.
MS (ESI) m/z = 254.1 [M+H]⁺
¹H NMR: (400 MHz, MeOH-d4) δ 7.46-7.54 (m, 2H), 7.24-7.28 (m, 1H), 1.92 (s, 3H).

### Step 4

### 8-(2-Chloro-6-fluorophenyl)-7-methylimidazo[1,2-c]pyrimidin-5-ol 10e

2-Chloroacetaldehyde (0.40 mL, 2.52 mmol) was added to a solution of compound **10d** (0.32 g, 1.3 mmol) in DMF (10 mL). The reaction solution was reacted at 80°C for 2 hours. After the reaction was completed, 50 mL of water was added, and the reaction solution was extracted with ethyl acetate (50 mL). The organic phases were combined, washed with saturated sodium chloride solution (100 mL), dried over anhydrous sodium sulfate, and filtered. The filtrate was collected, and concentrated under reduced pressure. The residue was purified by silica gel chromatography with petroleum ether and ethyl acetate as an eluent to obtain the title compound **10e** (30 mg, yield: 8%).
MS(ESI) m/z 277.9 [M+H]⁺
¹H NMR: (400 MHz, CDCl₃) δ 7.77 (d, J = 1.6 Hz, 1H), 7.58-7.47 (m, 2H), 7.39-7.32 (m, 1H), 7.26 (d, J = 1.6 Hz, 1H), 6.96 (s, 1H), 2.01 (s, 3H).

### Step 5

### 5-Chloro-8-(2-chloro-6-fluorophenyl)-7-methylimidazo[1,2-c]pyrimidine 10f

Phosphorus oxychloride (1 mL) and DIEA (0.2 mL, 1.2 mmol) were added to compound **10e** (30 mg, 0.11 mmol). The reaction solution was reacted at 110°C for 2 hours. The reaction solution was concentrated under reduced pressure, dichloromethane (50 mL) and ice water (100 mL) were added, and saturated sodium bicarbonate solution was added to adjust pH = 8. The reaction solution was extracted with dichloromethane (50 mL). The organic phases were combined, washed with saturated sodium chloride solution (150 mL), dried over anhydrous sodium sulfate, and filtered. The filtrate was collected, and concentrated under reduced pressure to obtain the title compound **10f** (24 mg, yield: 75%) as a yellow solid.
MS(ESI) m/z 295.8 [M+H]⁺
1H NMR: (400 MHz, DMSO_d6) δ 8.12 (d, J = 0.8 Hz, 1H), 7.71 (d, J = 0.8 Hz, 1H), 7.66-7.60 (m, 1H), 7.58-7.54 (m, 1H), 7.44 (t, J = 8.8 Hz, 1H), 2.25 (s, 3H).

### Step 6

### (3S,4S)-8-(8-(2-Chloro-6-fluorophenyl)-7-methylimidazo[1,2-c]pyrimidin-5-yl)-3-meth yl-2-oxa-8-azaspiro[4.5]decan-4-amine 10

Compound **10f** (60 mg, 0.20 mol) was dissolved in DMSO (1.5 mL), followed by the addition of compound **2a** (49 mg, 0.20 mmol) and DIEA (0.1 mL, 0.61 mmol). The reaction solution was reacted at 90°C for 30 minutes. After the reaction was completed, water (40 mL) was added, and the reaction solution was extracted with ethyl acetate (30 mL×3). The organic phase was washed with saturated sodium chloride solution (50 mL×2), dried over anhydrous sodium sulfate, and filtered. The filtrate was collected, and concentrated under reduced pressure. The residue was purified by silica gel preparative thin layer chromatography with dichloromethane and methanol (containing 0.1% of ammonia) as an eluent to obtain the title compound **10** (32 mg, yield: 36.4%).
MS(ESI) m/z 430.2 [M+H]⁺
¹H NMR: TJN180745-173-1C3 (400MHz, MeOD_d4) δ 7.69 (d, J = 1.2 Hz, 1H), 7.52-7.46 (m, 1H), 7.45 (d, J = 1.2 Hz, 1H), 7.44-7.41 (m, 1H), 7.26-7.20 (m, 1H), 4.31-4.22 (m, 1H), 3.91 (d, J = 8.8 Hz, 1H), 3.83-3.72 (m, 3H), 3.37-3.31 (m, 1H), 3.28-3.20 (m, 1H), 3.11 (d, J = 5.2 Hz, 1H), 2.19 (s, 3H), 2.11-1.95 (m, 2H), 1.87-1.76 (m, 2H), 1.25 (d, J = 6.4 Hz, 3H).

### Example 11

### (3S,4S)-8-(8-(2,3-Dichloro-6-fluorophenyl)-7-methylimidazo[1,2-c]pyrimidin-5-yl)-3-methyl-2-oxa-8-azaspiro[4.5]decan-4-amine

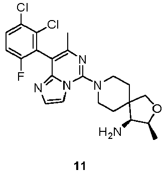

In accordance with the synthetic steps of Example 10, compound **10a** was replaced with compound 2-(2,3-dichloro-6-fluorophenyl)acetonitrile, accordingly, the compound of Example 11 was prepared.
MS(ESI) m/z 464.1 [M+H]⁺
¹H NMR: (400Hz, CD₃OD) δ 7.78-7.65 (m, 2H), 7.46 (d, J = 1.2 Hz, 1H), 7.28 (t, J = 8.4 Hz, 1H), 4.35-4.19 (m, 1H), 3.91 (d, J = 8.8 Hz, 1H), 3.87-3.73 (m, 3H), 3.42-3.33 (m, 1H), 3.30-3.19 (m, 1H), 3.12 (d, J = 4.8 Hz, 1H), 2.20 (s, 3H), 2.11-1.97 (m, 2H), 1.92-1.76 (m, 2H), 1.25 (d, J = 6.4 Hz, 3H).

### Example 12

### (3S,4S)-8-(8-(2-Bromo-3-chlorophenyl)-7-methylimidazo[1,2-c]pyrimidin-5-yl)-3-meth yl-2-oxa-8-azaspiro[4.5]decan-4-amine

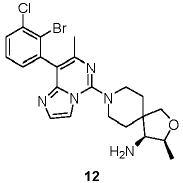

In accordance with the synthetic steps of Example 10, compound **10a** was replaced with compound 2-(2-bromo-3-chlorophenyl)acetonitrile, accordingly, the compound of Example 12 was prepared.
MS(ESI) m/z 492.3 [M+H]⁺
¹H NMR: (400MHz, CD₃OD) δ 7.70 (d, J = 1.6 Hz, 1H), 7.63 (dd, J = 1.6 Hz, 8.0 Hz, 1H), 7.49-7.43 (m, 2H), 7.27 (dd, J = 1.2 Hz, 7.6 Hz, 1H), 4.31-4.22 (m, 1H), 3.90 (d, J = 8.8 Hz, 1H), 3.80-3.71 (m, 3H), 3.37-3.32 (m, 1H), 3.27-3.19 (m, 1H), 3.10 (d, J = 4.8 Hz, 1H), 2.17 (s, 3H), 2.07-1.96 (m, 2H), 1.88-1.76 (m, 2H), 1.25 (d, J = 7.2 Hz, 3H).

### Example 13

### (3S,4S)-3-Methyl-8-(7-(trifluoromethyl)-8-((2-(trifluoromethyl)pyridin-3-yl)thio)imidaz o[1,2-c]pyrimidin-5-yl)-2-oxa-8-azaspiro[4.5]decan-4-amine

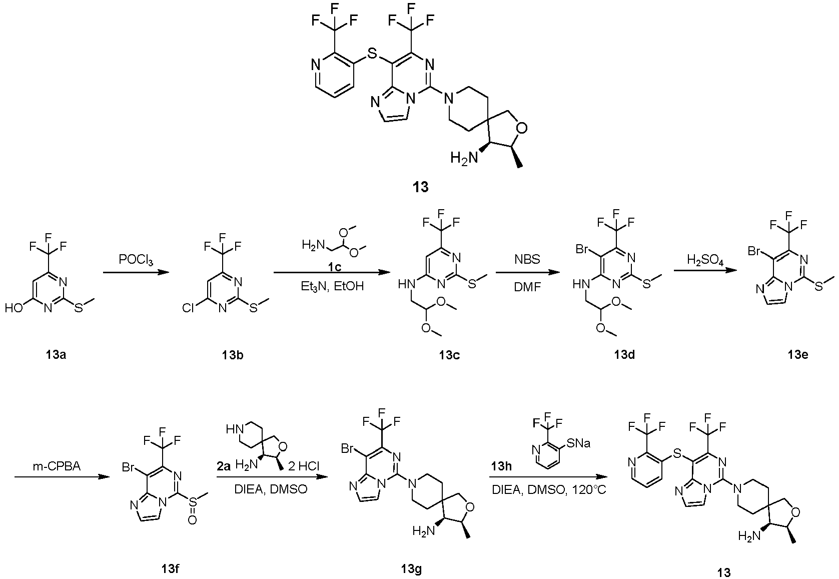

### Step 1

### 4-Chloro-2-(methylthio)-6-(trifluoromethyl)pyrimidine 13b

2-(Methylthio)-6-(trifluoromethyl)pyrimidin-4-ol **13a** (100 mg, 0.21 mmol) was dissolved in phosphorus oxychloride (20 mL). The reaction solution was reacted under a nitrogen atmosphere at 120°C for 5 hours. After the reaction was completed, the reaction solution was concentrated under reduced pressure to obtain the title compound **13b** (4 g, crude product) as a yellow solid, which was used directly in the next step without purification.

### Step 2

### N-(2,2-Dimethoxyethyl)-2-(methylthio)-6-(trifluoromethyl)pyrimidin-4-amine 13c

Compound **13b** (4 g, 17.50 mmol) was dissolved in N,N dimethylformamide (10 mL), followed by the addition of compound **1c** (2.84 mL, 26.24 mmol) and triethylamine (5.31 g, 52.49 mmol). The reaction solution was reacted at 6 to 11 °C for 12 hours. After the reaction was completed, the reaction solution was poured into 40 mL of water. Saturated aqueous sodium hydroxide solution was added to adjust the pH to 10, and the reaction solution was extracted with ethyl acetate (25 mL×3). The organic phases were combined, washed with saturated aqueous sodium chloride solution (25 mL×2), dried over anhydrous sodium sulfate, and filtered. The filtrate was concentrated under reduced pressure, and the resulting crude product was purified by silica gel chromatography with ethyl acetate and petroleum ether as an eluent to obtain the title compound **13c** (1.12 g, yield of two steps: 33%) as a yellow solid.
MS(ESI) m/z 298.2 [M+H]⁺
¹H NMR: (400MHz, CD₃OD) δ 6.52 (s, 1H), 4.54 (t, J = 5.2 Hz, 1H), 3.59 (d, J = 5.2 Hz, 2H), 3.40 (s, 6H), 2.50 (s, 3H).

### Step 3

### 5-Bromo-N-(2,2-dimethoxyethyl)-2-(methylthio)-6-(trifluoromethyl)pyrimidin-4-amine 13d

Compound **13c** (1.02 g, 3.43 mmol) was dissolved in N,N-dimethylformamide (15 mL), followed by the addition of N-bromosuccinimide (0.67 g, 3.77 mmol). The reaction solution was reacted under a nitrogen atmosphere at 70°C for 1 hour. After the reaction was completed, the reaction solution was poured into 100 mL of water, and extracted with ethyl acetate (40 mL×3). The organic phases were combined, washed with saturated aqueous sodium chloride solution (50 mL×2), dried over anhydrous sodium sulfate, and filtered. The filtrate was concentrated under reduced pressure, and the resulting crude product was purified by silica gel chromatography with ethyl acetate and petroleum ether as an eluent to obtain the title compound **13d** (1.1 g, yield: 85%) as a yellow solid.
MS(ESI) m/z 377.8 [M+H+2]⁺
¹H NMR: (400MHz, CD₃OD) δ 4.62 (t, J = 5.2 Hz, 1H), 3.65 (d, J = 5.6 Hz, 2H), 3.41 (s, 6H), 2.51 (s, 3H).

### Step 4

### 8-Bromo-5-(methylthio)-7-(trifluoromethyl)imidazo[1,2-c]pyrimidine 13e

Compound **13d** (1.05 g, 2.79 mmol) was dissolved in concentrated sulfuric acid (15 mL), and reacted at 65°C for 12 hours. After the reaction was completed, the reaction solution was diluted with 25 mL of ethyl acetate. The reaction solution was poured into 100 mL of ice water, and saturated sodium hydroxide solution was added to adjust the pH to 11 to 13. The aqueous phase was separated, and extracted with ethyl acetate (35 mL×3). The ethyl acetate phases were combined, washed with saturated aqueous sodium chloride solution (25 mL×2), dried over anhydrous sodium sulfate, and filtered. The filtrate was concentrated under reduced pressure to obtain the title compound **13e** (642 mg, yield: 74%) as a yellow solid.
MS(ESI) m/z 313.8 [M+H+2]⁺
¹H NMR: (400MHz, CD₃OD) δ 8.01 (s, 1H), 7.87 (d, J = 1.6 Hz, 1H), 2.85 (s, 3H).

### Step 5

### 8-Bromo-5-(methylsulfinyl<sulfenyl>)-7-(trifluoromethyl)imidazo[1,2-c]pyrimidine 13f

Compound **13e** (290 mg, 0.93 mmol) was dissolved in dichloromethane (8 mL), followed by the addition of *m*-chloroperoxybenzoic acid (481 mg, 2.79 mmol) at 0 to 3°C. The reaction solution was reacted at 0 to 3°C for 2 hours. After the reaction was completed, the reaction was quenched by 25 mL of saturated sodium bisulfite solution at 0 to 3°C. The reaction solution was stirred at 0 to 3°C for 10 minutes, and extracted with dichloromethane (25 mL×3). The organic phases were combined, washed with saturated sodium bicarbonate solution (30 mL×2) and saturated aqueous sodium chloride solution (30 mL) successively, dried over anhydrous sodium sulfate, and filtered. The filtrate was concentrated under reduced pressure to obtain the title compound **13f** (225 mg, yield: 74%) as a yellow solid.
MS(ESI) m/z 327.7 [M+H+2]⁺

### Step 6

### (3S,4S)-8-(8-Bromo-7-(trifluoromethyl)imidazo[1,2-c]pyrimidin-5-yl)-3-methyl-2-oxa-8-azaspiro[4.5]decan-4-amine 13g

Compound **13f** (163 mg, 0.67 mmol) was dissolved in dimethyl sulfoxide (5 mL), followed by the addition of (3S,4S)-3-methyl-2-oxa-8-azaspiro[4.5]decan-4-amine dihydrochloride **2a** (163 mg, 0.67 mmol) and N,N-diisopropylethylamine (260 mg, 2.01 mmol). The reaction solution was reacted at 60°C for 2 hours. After the reaction was completed, the reaction solution was diluted with 30 mL of water, and then extracted with a mixed solvent of chloroform and isopropanol (3/1, 30 mL×3). The organic phases were combined, dried over anhydrous sodium sulfate, and filtered. The filtrate was concentrated under reduced pressure, and the resulting crude product was purified by silica gel chromatography with methanol and dichloromethane as an eluent to obtain the title compound **13g** (115 mg, yield: 39%) as a yellow solid.
MS(ESI) m/z 435.7 [M+H+2]⁺
¹H NMR: (400MHz, CD₃OD) δ 7.78 (d, J = 1.2 Hz, 1H), 7.57 (d, J = 0.8 Hz, 1H), 4.23-4.17 (m, 1H), 3.84 (d, J = 9.2 Hz, 1H), 3.74-3.66 (m, 3H), 3.42-3.35 (m, 1H), 3.33-3.27 (m, 1H), 3.06 (d, J = 4.4 Hz, 1H), 2.09-2.00 (m, 1H), 1.95-1.88 (m, 1H), 1.86-1.78 (m, 2H), 1.26 (d, J = 6.8 Hz, 3H).

### Step 7

### (3S,4S)-3-Methyl-8-(7-(trifluoromethyl)-8-((2-(trifluoromethyl)pyridin-3-yl)thio)imidaz o[1,2-c]pyrimidin-5-yl)-2-oxa-8-azaspiro[4.5]decan-4-amine 13

Compound **13g** (100 mg, 0.23 mmol) and compound **13h** (139 mg, 0.69 mmol, prepared according to the method disclosed in the patent application "WO2016203405 A1") were dissolved in dimethyl sulfoxide (5 mL), followed by the addition of N,N-diisopropylethylamine (238 mg, 1.84 mmol). The reaction solution was reacted at 120°C for 4 hours. After the reaction was completed, the reaction solution was diluted with 30 mL of ethyl acetate, washed with saturated aqueous sodium chloride solution (20 mL×2), dried over anhydrous sodium sulfate, and filtered. The filtrate was concentrated under reduced pressure, and the resulting residue was purified by preparative high performance liquid chromatography (Welch Xtimate C18 150*30 mm*5 µm; condition: 37-67% B (A: water (containing 0.05% of ammonia), B: acetonitrile); flow rate: 25 ml/min) to obtain the title compound **13** (8.9 mg, yield: 7%).
MS(ESI) m/z 533.1 [M+H]⁺
¹H NMR: (400MHz, CD₃OD) δ 8.44-8.30 (m, 1H), 7.95 (d, J = 1.2 Hz, 1H), 7.65 (d, J = 1.2 Hz, 1H), 7.34-7.25 (m, 2H), 4.32-4.19 (m, 1H), 4.07-3.95 (m, 2H), 3.91 (d, J = 8.8 Hz, 1H), 3.76 (d, J = 8.8 Hz, 1H), 3.62-3.52 (m, 1H), 3.51-3.42 (m, 1H), 3.09 (d, J = 4.8 Hz, 1H), 2.10-1.93 (m, 2H), 1.89-1.75 (m, 2H), 1.24 (d, J = 6.4 Hz, 3H).

### Example 14

### 1-(8-((3-Chloro-2-fluoropyridin-4-yl)thio)imidazo[1,2-c]pyrimidin-5-yl)-4-methylpiperi din-4-amine

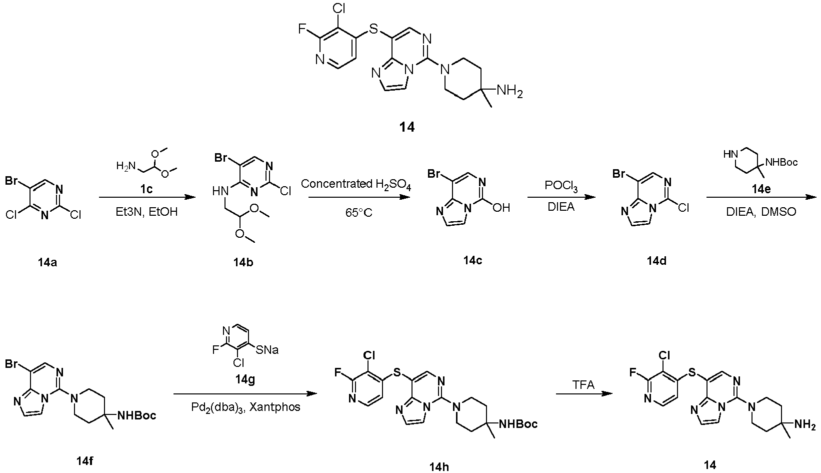

### Step 1

### 5-Bromo-2-chloro-N-(2,2-dimethoxyethyl)pyrimidin-4-amine 14b

5-Bromo-2,4-dichloropyrimidine **14a** (600 mg, 2.63 mmol) was dissolved in 15 mL of ethanol, followed by the addition of 2,2-dimethoxyethylamine **1c** (554 mg, 5.27 mmol) and triethylamine (0.73 mL, 5.27 mmol). The reaction solution was stirred at room temperature for 12 hours. After the reaction was completed, the reaction solution was concentrated. 20 mL of ice-water mixture was added, and the reaction solution was extracted with ethyl acetate (10 mL×3). The organic phases were combined, washed with saturated sodium chloride solution (5 mL), dried over anhydrous sodium sulfate, and filtered. The filtrate was collected, and concentrated under reduced pressure. The residue was purified by silica gel chromatography with petroleum ether and ethyl acetate as an eluent to obtain compound **14b** (710 mg, yield: 91%) as a yellow solid.
MS(ESI) m/z = 295.9, 297.9 [M+H]⁺
¹H NMR: (400 MHz, CDCl3) δ 8.14 (s, 1H), 5.77 (br s, 1H), 4.50 (t, J = 5.2 Hz, 1H), 3.67 (t, J = 5.2 Hz, 2H), 3.45 (s, 6H).

### Step 2

### 8-Bromo-7-imidazo[1,2-c]pyrimidin-5-ol 14c

Compound **14b** (700 mg, 2.36 mmol) was dissolved in 7 mL of concentrated sulfuric acid, and the reaction solution was reacted at 65 °C for 2 hours. After the reaction was completed, 100 mL of ice-water mixture was added, and saturated sodium hydroxide solution was added until pH=6. The reaction solution was extracted with a mixed solvent of dichloromethane and isopropanol (50 mL, volume ratio: 3:1). The organic phases were combined, washed with saturated sodium chloride solution (150 mL), dried over anhydrous sodium sulfate, and filtered. The filtrate was collected, and concentrated under reduced pressure to obtain the title compound **14c** (330 mg, yield: 65.3%) as a yellow solid.
MS(ESI) m/z = 213.7, 215.7 [M+H]⁺
¹H NMR: (400 MHz, MeOH-d4) δ 7.88 (d, J = 1.6 Hz, 1H), 7.51 (s, 1H), 7.43 (d, J = 1.6 Hz, 1H).

### Step 3

### 8-Bromo-5-chloro-imidazo[1,2-c]pyrimidine 14d

N,N-Diisopropylethylamine (6.0 mL, 36.45 mmol) was added to a suspension of compound **14c** (300 mg, 1.4 mmol) and phosphorus oxychloride (19.25 g). The reaction solution was reacted at 110°C for 3 hours. After the reaction was completed, the reaction solution was concentrated under reduced pressure. 50 mL of saturated sodium bicarbonate solution was added, and the reaction solution was extracted with ethyl acetate (50 mL). The organic phases were combined, washed with saturated sodium chloride solution (80 mL), dried over anhydrous sodium sulfate, and filtered. The filtrate was collected, and concentrated under reduced pressure to obtain the title compound **14d** (230 mg, yield: 55%) as a yellow solid.
MS (ESI) m/z = 231.8, 233.8 [M+H]⁺

### Step 4

### Tert-butyl (1-(8-bromoimidazo[1,2-c]pyrimidin-5-yl)-4-methylpiperidin-4-yl)carbamate 14f

Compound **14d** (220 mg, 0.95 mmol), *tert*-butyl (4-methylpiperidin-4-yl)carbamate **14e** (203 mg, 0.95 mmol) and N,N-diisopropylethylamine (0.47 mL, 2.84 mmol) were dissolved in 5 mL of dimethyl sulfoxide. The reaction solution was reacted at 90°C for 1.5 hours. After the reaction was completed, 20 mL of ethyl acetate and 40 mL of water were added, and the reaction solution was extracted with ethyl acetate (20 mL×3). The organic phases were combined, washed with saturated sodium chloride solution (100 mL), dried over anhydrous sodium sulfate, and filtered. The filtrate was collected, and concentrated under reduced pressure. The residue was purified by silica gel chromatography with dichloromethane and methanol as an eluent to obtain the title compound **14f** (220 mg, yield: 54.3%) as a yellow solid.
MS(ESI) m/z 409.9 [M+H]⁺
¹H NMR: (400 MHz, CDCl3) δ 7.88 (s, 1H), 7.66 (d, J = 1.6 Hz, 1H), 7.49 (d, J = 1.6 Hz, 1H), 4.45 (br s, 1H), 3.57-3.49 (m, 2H), 3.37-3.27 (m, 2H), 2.26-2.20 (m, 2H), 1.85-1.77 (m, 2H), 1.45 (s, 12H).

### Step 5

### Tert-butyl

### (1-(8-((3-chloro-2-fluoropyridin-4-yl)thio)imidazo[1,2-c]pyrimidin-5-yl)-4-methylpiper idin-4-yl)carbamate 14h

Compound **14f** (210 mg, 0.51 mmol), compound **14g** (237 mg, 0.77 mmol), N,N-diisopropylethylamine (0.17 mL, 1.02 mmol), tris(dibenzylideneacetone)dipalladium (23.4 mg, 0.026 mmol) and 4,5-bis(diphenylphosphino)-9,9-dimethylxanthene (29.6 mg, 0.051 mmol) were dissolved successively in 6 mL of 1,4-dioxane under a nitrogen atmosphere. The reaction solution was reacted at 100°C for 15 hours, and warmed up to 120°C for 3 hours. The reaction solution was cooled to room temperature. 10 mL of ethyl acetate and 10 mL of water were added, and the reaction solution was extracted with ethyl acetate (10 mL). The organic phases were combined, washed with saturated sodium chloride solution (50 mL), dried over anhydrous sodium sulfate, and filtered. The filtrate was collected, and concentrated under reduced pressure. The residue was purified by silica gel chromatography with petroleum ether and ethyl acetate as an eluent to obtain the title compound **14h** (25 mg, yield: 9.9%) as a yellow solid.
MS(ESI) m/z 493.1 [M+H]⁺
¹H NMR: (400 MHz, CDCl3) δ 8.03 (s, 1H), 7.72 (d, J = 5.2 Hz, 1H), 7.63 (d, J = 1.2 Hz, 1H), 7.52 (d, J = 1.2 Hz, 1H), 6.41 (d, J = 5.2 Hz, 1H), 4.48 (br s, 1H), 3.78-3.70 (m, 2H), 3.53-3.44 (m, 2H), 2.32-2.25 (m, 2H), 1.88-1.79 (m, 2H), 1.48-1.44 (m, 12H).

### Step 6

### 1-(8-((3-Chloro-2-fluoropyridin-4-yl)thio)imidazo[1,2-c]pyrimidin-5-yl)-4-methylpiperi din-4-amine 14

Compound **14h** (25 mg, 0.051 mol) was dissolved in 3 mL of dichloromethane, followed by the addition of 1 mL of trifluoroacetic acid at 0°C. The reaction solution was reacted at 25°C for 2 hours. Saturated sodium bicarbonate solution was added until pH = 9, and the reaction solution was extracted with chloroform (20 mL×2). The organic phase was washed with saturated sodium chloride solution (20 mL×2), dried over anhydrous sodium sulfate, and filtered. The filtrate was collected, and concentrated under reduced pressure to obtain the title compound **14** (3.7 mg, yield: 18.6%).
MS(ESI) m/z 393.1 [M+H]⁺
¹H NMR: (400 MHz, CDCl3) δ 8.04 (s, 1H), 7.72 (d, J = 5.2 Hz, 1H), 7.62 (d, J = 1.6 Hz, 1H), 7.51 (d, J = 1.6 Hz, 1H), 6.43 (d, J = 5.2 Hz, 1H), 3.71-3.64 (m, 4H), 1.87-1.77 (m, 2H), 1.73-1.66 (m, 2H), 1.30 (s, 3H).

### Example 15

### (R)-8-(8-((3-Chloro-2-fluoropyridin-4-yl)thio)imidazo[1,2-c]pyrimidin-5-yl)-8-azaspiro [4.5]decan-1-amine

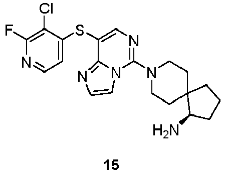

In accordance with the synthetic steps of Example 14, compound **14e** was replaced with compound **1g**, accordingly, the compound of Example 15 was prepared.
MS(ESI) m/z 433.1 [M+H]⁺
¹H NMR: (400 MHz, CDCl3) δ 8.03 (s, 1H), 7.72 (d, J= 5.2 Hz, 1H), 7.62 (s, 1H), 7.53 (s, 1H), 6.43 (d, J = 4.4 Hz, 1H), 4.02-3.91 (m, 2H), 3.33-3.19 (m, 2H),2.94 (t, J = 7.2 Hz, 1H), 2.13-2.00 (m, 1H), 1.92-1.84 (m, 3H),1.80-1.73 (m, 1H), 1.70-1.65 (m, 1H), 1.51-1.46 (m, 2H), 1.45-1.37 (s, 2H).

### Example 16

### (R)-8-(8-((2-(Trifluoromethyl)pyridin-3-yl)thio)imidazo[1,2-c]pyrimidin-5-yl)-8-azaspir o[4.5]decan-1-amine

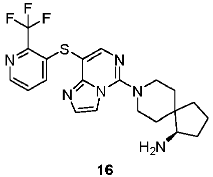

In accordance with the synthetic steps of Example 14, compound **14e** was replaced with compound **1g**, and compound **14g** was replaced with compound **13h,** accordingly, the compound of Example 16 was prepared.
MS(ESI) m/z 449.1 [M+H]+
¹H NMR: (400 MHz, CDCl3) δ 8.44 (s, 1H), 7.98 (s, 1H), 7.62 (s, 1H), 7.57-7.47 (m, 2H), 7.25-7.18 (m, 1H), 3.96-3.84 (m, 2H), 3.22 (q, J= 8.0Hz, 2H), 2.93 (t, J= 7.2Hz, 1H), 2.12-2.00 (m, 1H), 1.92-1.83 (m, 3H),1.79-1.74 (m, 1H), 1.66-1.61 (m, 1H), 1.54-1.48 (m, 2H), 1.44-1.38 (m, 2H).

### Example 17

### (3S,4S)-3-Methyl-8-(8-((2-(trifluoromethyl)pyridin-3-yl)thio)imidazo[1,2-c]pyrimidin-5-yl)-2-oxa-8-azaspiro[4.5]decan-4-amine

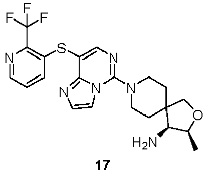

In accordance with the synthetic steps of Example 14, compound **14e** was replaced with compound **2a,** and compound **14g** was replaced with compound **13h,** accordingly, the compound of Example 17 was prepared.
MS(ESI) m/z 479.0 [M+H]⁺
¹H NMR: (400MHz, CD₃OD) δ 8.36 (d, J = 4.4 Hz, 1H), 7.75 (d, J = 1.2 Hz, 1H), 7.49 (d, J = 1.6 Hz, 1H), 7.31 (dd, J = 4.4, 8.4 Hz, 1H), 7.24 (d, J = 8.4 Hz, 1H), 4.30-4.22 (m, 1H), 3.94-3.83 (m, 3H), 3.76 (d, J = 8.8 Hz, 1H), 3.46-3.38 (m, 1H), 3.37-3.32 (m, 1H), 3.09 (d, J = 4.8 Hz, 1H), 2.53 (s, 3H), 2.07-1.95 (m, 2H), 1.86-1.76 (m, 2H), 1.24 (d, J = 6.4 Hz, 3H).

### Example 18

### (3S,4S)-3-Methyl-8-(8-((3-(trifluoromethyl)pyridin-4-yl)thio)imidazo[1,2-c]pyrimidin-5-yl)-2-oxa-8-azaspiro[4.5]decan-4-amine

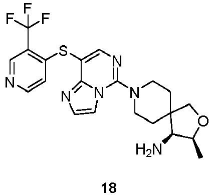

In accordance with the synthetic steps of Example 14, compound **13e** was replaced with compound **2a,** and compound **14g** was replaced with compound sodium 3-(trifluoromethyl)pyridine-4-thiolate, accordingly, the compound of Example 18 was prepared.
MS(ESI) m/z 465.1 [M+H]⁺
¹H NMR: (400 MHz, CD₃OD) δ 8.77-8.63 (m, 1H), 8.36-8.24 (m, 1H), 8.14-8.04 (m, 1H), 7.91-7.80 (m, 1H), 7.61-7.51 (m, 1H), 6.94-6.83(m, 1H), 4.33-4.20 (m, 1H), 4.00-3.84 (m, 3H), 3.80-3.70 (m, 1H), 3.53-3.35 (m, 2H), 3.14-3.05 (m, 1H), 2.11-1.93 (m, 2H), 1.90-1.74 (m, 2H), 1.24 (t, J = 6.0 Hz, 3H).

### Example 19

### 1-(8-((3-Chloro-2-fluoropyridin-4-yl)thio)-[1,2,4]triazolo[4,3-c]pyrimidin-5-yl)-4-meth ylpiperidin-4-amine

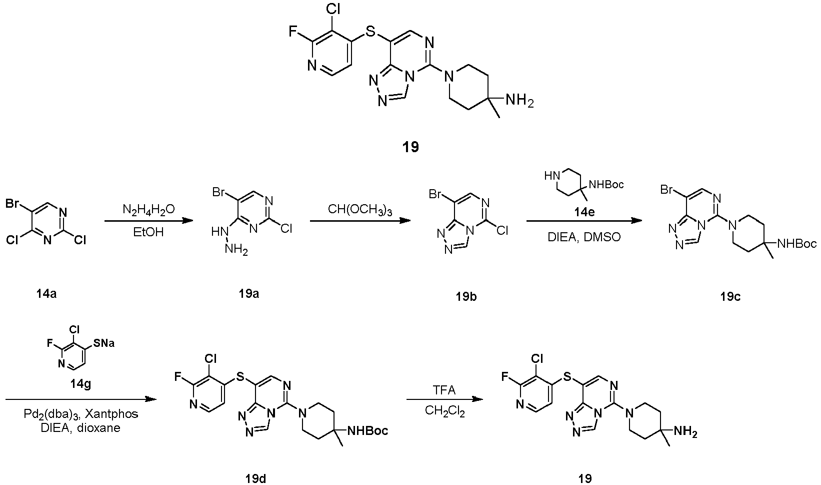

### Step 1

### 5 -Bromo-2-chloro-4-hydrazineylpyrimidine 19a

Compound **14a** (5.0 g, 22.0 mmol) was dissolved in ethanol (55 mL), followed by the addition of hydrazine hydrate (1.4 g, 26.4 mmol). The reaction solution was stirred at room temperature for 4 hours, and then filtered. The filter cake was rinsed with *n*-hexane (30 mL). The resulting solid was dried under vacuum to obtain compound **19a** (3.4 g, yield: 69%) as a yellow solid.
MS(ESI) m/z 222.9, 224.9 [M+H]⁺
¹H NMR (400 MHz, DMSO-*d*₆) δ 9.03 (br s, 1H), 8.15 (s, 1H), 4.62 (br s, 2H).

### Step 2

### 8-Bromo-5-chloro-[1,2,4]triazolo[4,3-c]pyrimidine 19b

Compound **19a** (3.2 g, 14.35 mmol) was dissolved in trimethyl orthoformate (32 mL), and the reaction solution was reacted at 90°C for 2 hours. After filtering the reaction solution, the filtrate was concentrated under vacuum to, and the resulting crude product was purified by silica gel chromatography with petroleum ether and ethyl acetate as an eluent to obtain the title compound (840 mg, yield: 25%) as a yellow solid.
MS(ESI) m/z 234.7 [M+H]⁺
¹H NMR: (400 MHz, CDCl3) δ 9.02 (s, 1H), 8.04 (s, 1H).

### Step 3

### Tert-butyl

### (1-(8-bromo-[1,2,4]triazolo[4,3-c]pyrimidin-5-yl)-4-methylpiperidin-4-yl)carbamate 19c

Compound **19b** (200 mg, 0.856 mmol), compound **14e** (220 mg, 1.03 mmol) and DIEA (221 mg, 1.71 mmol) were dissolved in DMSO (4.0 mL). The reaction solution was reacted at 28°C for 2 hours. Water (150 mL) was slowly added dropwise at 0°C, and the reaction solution was filtered. The filter cake was dried under vacuum to obtain compound **19c** (400 mg, yield: 90%) as a yellow solid.
MS(ESI) m/z 410.8, 412.8 [M+H]⁺
¹H NMR: (400 MHz, CDCl3) δ 8.76 (s, 1H), 7.82 (s, 1H), 4.46 (br s, 1H), 3.69-3.60 (m, 2H), 3.51-3.39 (m, 2H), 2.32-2.22 (m, 2H), 1.84-1.76 (m, 2H), 1.45 (s, 12H).

### Step 4

### Tert-butyl

### (1-(8-((3-chloro-2-fluoropyridin-4-yl)thio)-[1,2,4]triazolo[4,3-c]pyrimidin-5-yl)-4-meth ylpiperidin-4-yl)carbamate 19d

Compound **19c** (200 mg, 0.486 mmol), compound **14g** (108 mg, 0.583 mmol) and DIEA (188 mg, 1.46 mmol) were added to 1,4-dioxane (4 mL) under a nitrogen atmosphere. Tris(dibenzylideneacetone)dipalladium (44 mg, 0.048 mmol) and 4,5-bis(diphenylphosphino)-9,9-dimethylxanthene (56 mg, 0.097 mmol) were added. The reaction solution was reacted at 100°C for 16 hours. After the reaction was completed, water (30 mL) was added, and the reaction solution was extracted with ethyl acetate (30 mL×3). The organic phases were combined, washed with saturated sodium chloride solution (30 mL×3), and dried over anhydrous sodium sulfate. The product was purified by silica gel chromatography with petroleum ether and ethyl acetate as an eluent to obtain the title compound **19d** (129 mg, yield: 54%) as a yellow solid.
MS(ESI) m/z 494.0 [M+H]⁺
¹H NMR: (400 MHz, CDCl3) δ 8.82 (s, 1H), 7.99 (s, 1H), 7.75 (d, *J* = 5.6 Hz, 1H), 6.49 (d, *J* = 5.6 Hz, 1H), 4.48 (s, 1H), 3.94-3.85 (m, 2H), 3.68-3.59 (m, 2H), 2.38-2.27 (m, 2H), 1.87-1.77 (m, 2H), 1.46 (s, 12H).

### Step 5

### 1-(8-((3-Chloro-2-fluoropyridin-4-yl)thio)-[1,2,4]triazolo[4,3-c]pyrimidin-5-yl)-4-meth ylpiperidin-4-amine 19

Compound **19d** (129 mg, 0.26 mmol) was dissolved in dichloromethane (3.0 mL), followed by the addition of trifluoroacetic acid (1.0 mL). The reaction solution was reacted at room temperature for 1 hour. After the reaction was completed, IN aqueous NaOH solution was added to adjust the pH to 10. The reaction solution was extracted with chloroform (15 mL×3). The organic phases were combined, washed with saturated sodium chloride solution (15 mL×3), dried over anhydrous sodium sulfate, and concentrated under reduced pressure to obtain compound **19** (34.5 mg, yield: 33%).
MS(ESI) m/z 393.9 [M+H]⁺
¹H NMR: (400 MHz, CDCl3) δ 8.81 (s, 1H), 7.97 (s, 1H), 7.75 (d, *J* = 5.6 Hz, 1H), 6.49 (d, *J* = 5.6 Hz, 1H), 3.87-3.78 (m, 4H), 1.84-1.77 (m, 2H), 1.69-1.60 (m, 2H), 1.30 (s, 3H).

### Example 20

### (R)-8-(7-Amino-8-(2,3-dichlorophenyl)imidazo[1,2-c]pyrimidin-5-yl)-8-azaspiro[4.5]de can-1-amine

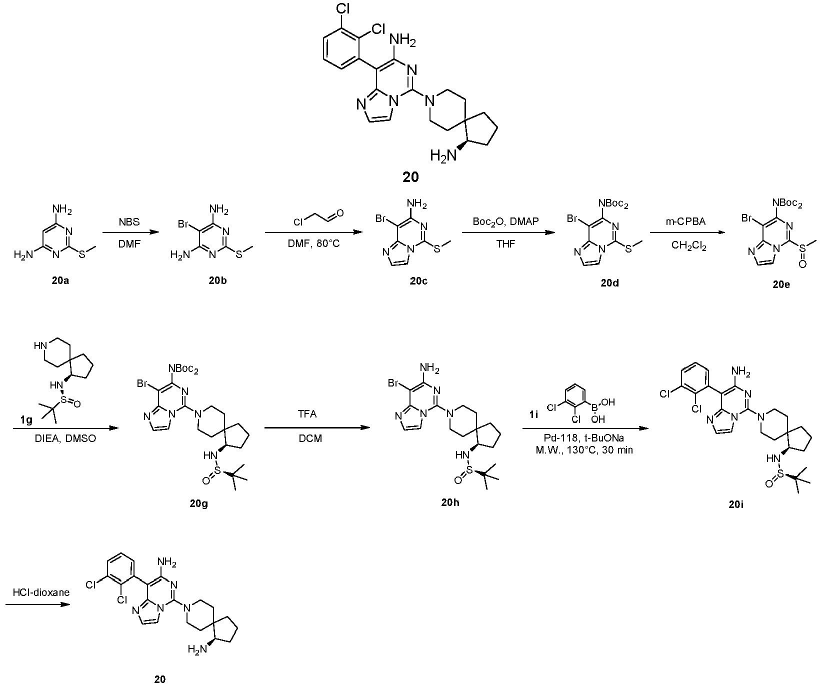

### Step 1

### 5-Bromo-2-(methylthio)pyrimidine-4,6-diamine 20a

Compound **20a** (10 g, 64 mmol) was dissolved in DMF (100 mL), followed by the addition of N-bromosuccinimide (13.7 g, 76.8 mmol). The reaction solution was reacted at 3 to 7°C for 12 hours. After the reaction was completed, 500 mL of water was added, and 12 mL of saturated sodium hydroxide solution was added to adjust the pH to 10. The reaction solution was extracted with ethyl acetate (200 mL×5). The organic phases were combined, and concentrated under reduced pressure. The crude residue was purified by silica gel chromatography with methanol and dichloroethane as an eluent to obtain the title compound **20b** (12 g, yield: 80%) as a yellow solid.
MS(ESI) m/z 236.7 [M+H]⁺
¹H NMR: (400MHz, CDCl3) δ 5.12 (br s, 4H), 2.45 (s, 3H).

### Step 2

### 8-Bromo-5-(methylthio)imidazo[1,2-c]pyrimidin-7-amine 20c

Compound **20b** (5.7 g, 24 mmol) was dissolved in DMF (70 mL), followed by the addition of 2-chloroacetaldehyde (7.1 g, 36 mmol). The reaction solution was reacted at 80°C for 2 hours. After the reaction was completed, 500 mL of water was added, and saturated sodium hydroxide solution was added to adjust the pH to 10. The reaction solution was extracted with ethyl acetate (200 mL×5). The organic phases were combined, and concentrated under reduced pressure. The crude residue was purified by silica gel chromatography with petroleum ether and ethyl acetate as an eluent to obtain the title compound **20c** (3.6 g, yield: 57%) as a yellow solid.
MS(ESI) m/z 260.8 [M+H]⁺
¹H NMR: (400MHz, CDCl3) δ 7.45 (d, J = 1.2 Hz, 1H), 7.35 (d, J = 1.6 Hz, 1H), 4.81 (br s, 2H), 2.71 (s, 3H).

### Step 3

### Di-tert-butyl (8-bromo-5-(methylthio)imidazo[1,2-c]pyrimidin-7-yl)aminodicarboxylate 20d

Compound **20c** (3.8 g, 15 mmol) was dissolved in THF (40 mL), followed by the addition of di-*tert*-butyl dicarbonate (9.6 g, 44 mmol) and DMAP (0.34 g, 2.9 mmol). The reaction solution was reacted at 20°C for 12 hours. After the reaction was completed, 100 mL of water was added, and the reaction solution was extracted with ethyl acetate (70 mL×3). The organic phases were combined, and concentrated under reduced pressure. The crude residue was purified by silica gel chromatography with petroleum ether and ethyl acetate as an eluent to obtain the title compound **20d** (3.2 g, yield: 48%) as a yellow solid.
MS(ESI) m/z 460.9 [M+H]⁺
¹H NMR: (400MHz, CDCl3) δ 7.74 (d, J = 1.6 Hz, 1H), 7.60 (d, J = 1.6 Hz, 1H), 2.75 (s, 3H), 1.45 (s, 18H).

### Step 4

### Di-tert-butyl

### (8-bromo-5-(methylsulfinyl)imidazo[1,2-c]pyrimidin-7-yl)aminodicarboxylate 20e

Compound **20d** (0.40 g, 0.87 mmol) was dissolved in DCM (10 mL), followed by the addition of *m*-chloroperoxybenzoic acid (0.53 g, 2.6 mmol). The reaction solution was reacted at 0°C for 1 hour. After the reaction was completed, 15 mL of saturated aqueous sodium bisulfite solution was added, and the reaction solution was stirred for 15 minutes. 30 mL water was added, and the reaction solution was extract with dichloromethane (25 mL×3). The organic phases were combined, washed with saturated aqueous sodium bicarbonate solution (20 mL) and saturated aqueous sodium chloride solution (20 mL) successively, and concentrated under reduced pressure to obtain the title compound **20e** (0.4 g, yield: 97%) as a yellow solid.
MS(ESI) m/z 476.7 [M+H]⁺
¹H NMR: (400MHz, CDCl3) δ 8.77 (d, J = 1.2 Hz, 1H), 7.88 (d, J = 1.2 Hz, 1H), 3.18 (s, 3H), 1.45 (s, 18H).

### Step 5

### Di-tert-butyl

### (8-bromo-5-((R)-1-(((R)-tert-butylsulfinyl)amino)-8-azaspiro[4.5]decan-8-yl)imidazo[1, 2-c]pyrimidin-7-yl)aminodicarboxylate 20g

Compound **20e** (1.9 g, 4.0 mmol) and compound **1g** (0.58 g, 2.3 mmol) were dissolved in DMSO (20 mL), followed by the addition of DIEA (0.87 g, 6.8 mmol). The reaction solution was reacted at 50°C for 2 hours. After the reaction was completed, 50 mL of water was added, and the reaction solution was extracted with ethyl acetate (50 mL×3). The organic phases were combined, washed with saturated aqueous sodium chloride solution (50 mL×3), dried over anhydrous sodium sulfate, and filtered. The filtrate was concentrated under reduced pressure, and the crude residue was purified by C-18 chromatography with ammonia (10 mM) and methanol as an eluent to obtain the title compound **20g** (1.0 g, yield: 37%) as a yellow solid.
MS(ESI) m/z 669.3 [M+H]⁺
¹H NMR: (400MHz, CDCl3) δ 7.65 (s, 1H), 7.55 - 7.50 (m, 1H), 3.83 - 3.64 (m, 2H), 3.41 (q, J=6.8 Hz, 1H), 3.25 (d, J=6.0 Hz, 1H), 3.16 - 2.97 (m, 2H), 2.19 - 2.04 (m, 2H), 1.92 - 1.82 (m, 2H), 1.80 - 1.64 (m, 4H), 1.63 - 1.53 (m, 2H), 1.45 (s, 18H), 1.25 (s, 9H).

### Step 6

### (R)-N-((R)-8-(7-Amino-8-bromoimidazo[1,2-c]pyrimidin-5-yl)-8-azaspiro[4.5]decan-1-yl)-2-methylpropane-2-sulfinamide 20h

Compound **20g** (100 mg, 0.15 mmol) was dissolved in DCM (3 mL), followed by the addition of TFA (1 mL). The reaction solution was reacted at 5 to 8°C for 1 hour. After the reaction was completed, the reaction solution was concentrated under reduced pressure. 20 mL of water was added, and saturated sodium hydroxide solution was added to adjust pH = 13. The reaction solution was extracted successively with ethyl acetate (10 mL×3) and dichloromethane (20 mL×3). The organic phases were combined, dried over anhydrous sodium sulfate, and filtered. The filtrate was concentrated under reduced pressure to obtain the title compound **20h** (37 mg, yield: 38%) as a yellow solid.
MS(ESI) m/z 469.0 [M+H]⁺
¹H NMR: (400MHz, CDCl3) δ 7.42 - 7.36 (m, 1H), 7.29 - 7.27 (m, 1H), 4.68 (s, 2H), 3.79 - 3.73 (m, 1H), 3.45 - 3.33 (m, 1H), 3.24 (d, J=5.2 Hz, 1H), 3.10 - 2.97 (m, 2H), 2.19 - 2.06 (m, 1H), 2.05 - 1.96 (m, 1H), 1.90 - 1.65 (m, 6H), 1.48 - 1.42 (m, 2H), 1.24 (s, 9H).

### Step 7

### (R)-N-((R)-8-(7-Amino-8-(2,3-dichlorophenyl)imidazo[1,2-c]pyrimidin-5-yl)-8-azaspir o[4.5]decan-1-yl)-2-methylpropane-2-sulfinamide 20i

Compound **20h** (100 mg, 0.21 mmol) and 2,3-dichlorophenylboronic acid **1i** (203 mg, 1.07 mmol) were dissolved in dioxane (4 mL) under a nitrogen atmosphere, followed by the addition of Pd-118 (42 mg, 0.064 mmol) and sodium *tert*-butoxide (82 mg, 0.85 mmol). The reaction solution was reacted under microwave at 130°C for 30 minutes. After the reaction was completed, the reaction solution was concentrated under reduced pressure, and the resulting residue was purified by silica gel chromatography with methanol and dichloroethane as an eluent to obtain the yellow title compound **20i** (85 mg, yield: 43%).
MS(ESI) m/z 535.0 [M+H]⁺

### Step 8

### (R)-8-(7-Amino-8-(2,3-dichlorophenyl)imidazo[1,2-c]pyrimidin-5-yl)-8-azaspiro[4.5]de can-1-amine 20

Compound **20i** (80 mg, 0.085 mmol) was dissolved in dioxane (3 mL), followed by the addition of a solution (1 mL, 4 mol/L) of hydrogen chloride in dioxane. The reaction solution was stirred at 0 to 7°C for 30 minutes. After the reaction was completed, water (25 mL) was added, and the reaction solution was washed with ethyl acetate (25 mL×3). Saturated sodium hydroxide solution was added to the aqueous phase to adjust the pH to 8 to 9, and the aqueous phase was extracted with dichloromethane (25 mL×3). The organic phases were combined, washed with saturated aqueous sodium chloride solution (30 mL), dried over sodium sulfate, and filtered. The filtrate was concentrated under reduced pressure, and the resulting crude product was purified by preparative high performance liquid chromatography (Phenomenex Gemini-NX 150*30mm*5 µm; condition: 33-63% B (A: water (containing 0.04% of ammonia + 10 mM ammonium bicarbonate), B: acetonitrile); flow rate: 30 ml/min) to obtain the title compound **20** (19.3 mg, yield: 53%).
MS(ESI) m/z 431.1 [M+H]⁺
¹H NMR: (400MHz, CD₃OD) δ 7.59 (dd, J = 1.6, 8.0 Hz, 1H), 7.42-7.37 (m, 2H), 7.34 (dd, J = 1.6, 8.0 Hz, 1H), 7.18 (d, J = 2.0 Hz, 1H), 3.92-3.75 (m, 2H), 3.24-3.07 (m, 2H), 2.95 (t, I = 7.2 Hz, 1H), 2.14-2.03 (m, 1H), 1.97-1.88 (m, 2H), 1.86-1.77 (m, 2H), 1.73-1.63 (m, 2H), 1.55-1.43 (m, 3H).

### Example 21

### (3S,4S)-8-(7-Amino-8-(2,3-dichlorophenyl)imidazo[1,2-c]pyrimidin-5-yl)-3-methyl-2-o xa-8-azaspiro[4.5]decan-4-amine

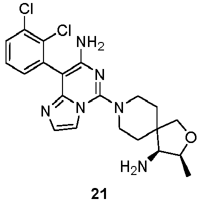

In accordance with the synthetic steps of Example **20,** compound **1g** was replaced with compound **2a,** accordingly, the compound of Example **21** was prepared.
MS(ESI) m/z 477.2 [M+H]⁺
¹H NMR: (400MHz, CD₃OD) δ 7.60 (dd, J = 2.0 Hz, 8.0 Hz, 1H), 7.43 (d, J = 2.0 Hz, 1H), 7.40 (t, J = 7.6 Hz, 1H), 7.34 (dd, J = 2.0 Hz, 7.6 Hz, 1H), 7.19 (d, J = 1.6 Hz, 1H), 4.30-4.23 (m, 1H), 3.90 (d, J = 8.8 Hz, 1H), 3.79-3.70 (m, 3H), 3.28-3.22 (m, 1H), 3.21-3.15 (m, 1H), 3.14-3.10 (m, 1H), 2.09-1.93 (m, 2H), 1.88-1.72 (m, 2H), 1.25 (d, J = 6.4 Hz, 3H).

### Example 22

### (R)-8-(7-Amino-8-((3-chloro-2-fluoropyridin-4-yl)thio)imidazo[1,2-c]pyrimidin-5-yl)-8 -azaspiro[4.5]decan-1-amine

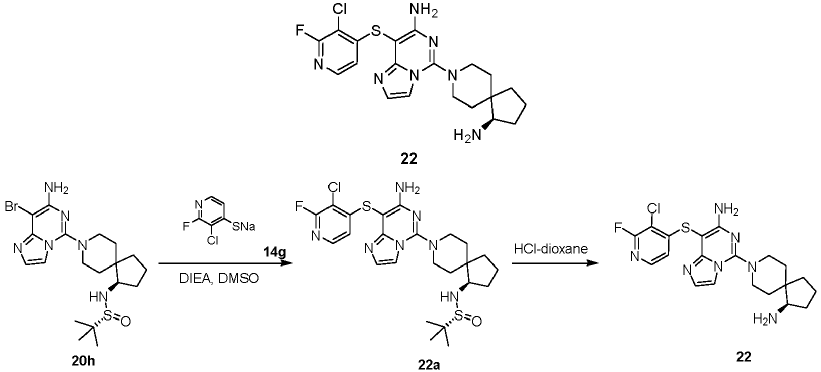

### Step 1

### (R)-N-((R)-8-(7-Amino-8-((3-chloro-2-fluoropyridin-4-yl)thio)imidazo[1,2-c]pyrimidin -5-yl)-8-azaspiro[4.5]decan-1-yl)-2-methylpropane-2-sulfinamide 22a

Compound **20h** (50 mg, 0.11 mmol) and sodium 3-chloro-2-fluoropyridine-4-thiolate **14g** (49 mg, 0.27 mmol) were dissolved in dimethyl sulfoxide (4.0 mL), followed by the addition of N,N-diisopropylethylamine (76 mg, 0.59 mmol). The reaction solution was reacted at 90°C for 20 hours. After the reaction was completed, ethyl acetate (20 mL) was added. The reaction solution was washed with 15 mL of saturated aqueous sodium chloride solution three times, dried over sodium sulfate, and filtered. The filtrate was concentrated under reduced pressure, and the resulting crude product was purified by silica gel chromatography with dichloromethane and methanol as an eluent to obtain compound **22a** (36 mg, yield: 54%) as a yellow solid.
MS(ESI) m/z 552.0 [M+H]⁺
¹H NMR: (400MHz, CDCl3) δ 7.74 (d, J = 5.6 Hz, 1H), 7.29 (s, 1H), 7.27 (s, 1H), 6.52 (d, J = 5.2 Hz, 1H), 5.10 (br s, 2H), 4.05-3.85 (m, 2H), 3.40 (q, J = 6.8 Hz, 1H), 3.31-3.24 (m, 1H), 3.23-3.07 (m, 2H), 2.14-2.03 (m, 2H), 1.91-1.84 (m, 2H), 1.78-1.56 (m, 4H), 1.52-1.43 (m, 2H), 1.23 (s, 9H).

### Step 2

### (R)-8-(7-Amino-8-((3-chloro-2-fluoropyridin-4-yl)thio)imidazo[1,2-c]pyrimidin-5-yl)-8 -azaspiro[4.5]decan-1-amine 22

Compound **22a** (33 mg, 0.052 mmol) was dissolved in dioxane (3.0 mL), followed by the addition of hydrochloric acid in methanol (0.5 mL). The reaction solution was reacted at 6 to 9°C for 0.5 hour. After the reaction was completed, 25 mL of water was added, and the reaction solution was washed with 20 mL of ethyl acetate. Saturated aqueous sodium bicarbonate solution was added to the aqueous phase to adjust the pH to 8 to 9, and the aqueous phase was extracted with dichloromethane (20 mL). The organic phases were combined, washed with 20 mL of saturated aqueous sodium chloride solution, dried over sodium sulfate, and filtered. The filtrate was concentrated under reduced pressure, and the resulting crude product was purified by preparative high performance liquid chromatography (Phenomenex Gemini-NX 150*30mm*5 µm; condition: 31-61% B (A: water (containing 0.04% of ammonia + 10 mM ammonium bicarbonate), B: acetonitrile); flow rate: 30 ml/min) to obtain the title compound **22** (10 mg, yield: 38%).
MS(ESI) m/z 448.1 [M+H]⁺
¹H NMR: (400MHz, CDCl3) δ 7.75 (d, J = 5 .2 Hz, 1H), 7.33 (s, 1H), 7.29-7.27 (m, 1H), 6.54 (d, J = 5.6 Hz, 1H), 5.06 (br s, 2H), 4.06-3.83 (m, 2H), 3.28-3.12 (m, 2H), 2.94 (t, J = 7.2 Hz, 1H), 2.11-1.99 (m, 1H), 1.90-1.82 (m, 3H), 1.79-1.75 (m, 1H), 1.73-1.63 (m, 2H), 1.58-1.52 (m, 1H), 1.49-1.42 (m, 2H).

### Example 23

### (R)-8-(7-Amino-8-((2-(trifluoromethyl)pyridin-3-yl)thio)imidazo[1,2-c]pyrimidin-5-yl)-8-azaspiro[4.5]decan-1-amine

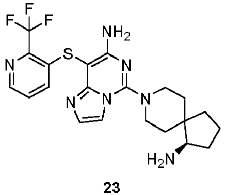

In accordance with the synthetic steps of Example **22,** compound **14g** was replaced with compound **13h,** accordingly, the compound of Example **23** was prepared.
MS(ESI) m/z 464.2 [M+H]⁺
¹H NMR: (400MHz, CD₃OD) δ 8.39-8.29 (m, 1H), 7.47 (s, 1H), 7.34 (d, J = 2.4 Hz, 2H), 7.19 (s, 1H), 3.97 (t, J = 13.2 Hz, 2H), 3.29-3.16 (m, 2H), 2.94 (t, J = 7.2 Hz, 1H), 2.15-2.04 (m, 1H), 1.97-1.87 (m, 2H), 1.86-1.75 (m, 2H), 1.75-1.61 (m, 2H), 1.59-1.48 (m, 2H), 1.47-1.39 (m, 1H).

### Example 24

### (3S,4S)-8-(7-Amino-8-((2-(trifluoromethyl)pyridin-3-yl)thio)imidazo[1,2-c]pyrimidin-5 -yl)-3-methyl-2-oxa-8-azaspiro[4.5]decan-4-amine

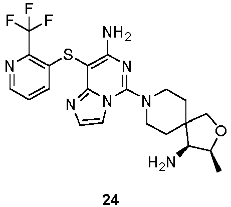

In accordance with the synthetic steps of Example **22,** compound **1g** was replaced with compound **2a,** and compound **14g** was replaced with compound **13h,** accordingly, the compound of Example **24** was prepared.
MS(ESI) m/z 480.2 [M+H]⁺
¹H NMR: (400MHz, CD₃OD) δ 8.34 (t, J = 2.8 Hz, 1H), 7.48 (d, J = 1.6 Hz, 1H), 7.38-7.31 (m, 2H), 7.19 (d, J = 1.6 Hz, 1H), 4.30-4.19 (m, 1H), 3.88 (d, J = 8.8 Hz, 1H), 3.87-3.78 (m, 2H), 3.74 (d, J = 8.8 Hz, 1H), 3.41-3.33 (m, 1H), 3.30-3.23 (m, 1H), 3.07 (d, J = 5.2 Hz, 1H), 2.04-1.88 (m, 2H), 1.84-1.72 (m, 2H), 1.23 (d, J = 6.4 Hz, 3H).

### Example 25

### (3S,4S)-8-(7-Amino-8-((3-chloro-2-methylpyridin-4-yl)thio)imidazo[1,2-c]pyrimidin-5-yl)-3-methyl-2-oxa-8-azaspiro[4.5]decan-4-amine

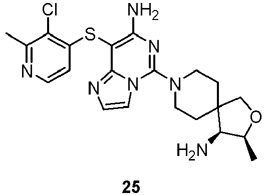

In accordance with the synthetic steps of Example **22,** compound **1g** was replaced with compound **2a,** and compound **14g** was replaced with compound sodium 3-chloro-2-methylpyridine-4-thiolate, accordingly, the compound of Example **25** was prepared.
MS(ESI) m/z 460.1 [M+H]⁺
¹H NMR: (400MHz, CD₃OD) δ 7.95 (d, J = 5.6 Hz, 1H), 7.48 (s, 1H), 7.18 (d, J = 1.6 Hz, 1H), 6.56 (d, J = 5.2 Hz, 1H), 4.28-4.22 (m, 1H), 3.91-3.81 (m, 3H), 3.78-3.73 (m, 1H), 3.41-3.33 (m, 1H), 3.27-3.11 (m, 1H), 3.10-3.06 (m, 1H), 2.03-1.94 (m, 2H), 1.83-1.74 (m, 2H), 1.24 (d, J = 6.4 Hz, 3H).

### Example 26

### (3S,4S)-8-(7-Amino-8-((3-chloro-2-fluoropyridin-4-yl)thio)imidazo[1,2-c]pyrimidin-5-yl)-3-methyl-2-oxa-8-azaspiro[4.5]decan-4-amine

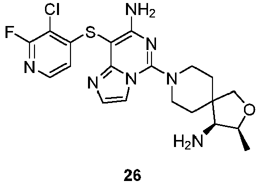

In accordance with the synthetic steps of Example **22,** compound **1g** was replaced with compound **2a,** accordingly, the compound of Example **26** was prepared.
MS(ESI) m/z 464.2 [M+H]⁺
¹H NMR: (400MHz, CD₃OD) δ 7.74 (d, J = 5.6 Hz, 1H), 7.49 (d, J = 2.0 Hz, 1H), 7.18 (d, J = 1.2 Hz, 1H), 6.60 (d, J = 5.2 Hz, 1H), 4.29-4.21 (m, 1H), 3.91-3.81 (m, 3H), 3.75 (d, J = 8.8 Hz, 1H), 3.43-3.34 (m, 1H), 3.29-3.24 (m, 1H), 3.07 (d, J = 4.8 Hz, 1H), 2.03-1.91 (m, 2H), 1.85-1.72 (m, 2H), 1.24 (d, J = 6.4 Hz, 3H).

### Example 27

### (S)-1'-(8-((2-Amino-3-chloropyridin-4-yl)thio)imidazo[1,2-c]pyrimidin-5-yl)-5,7-dihyd rospiro[cyclopenta[b]pyridine-6,4'-piperidin]-5-amine

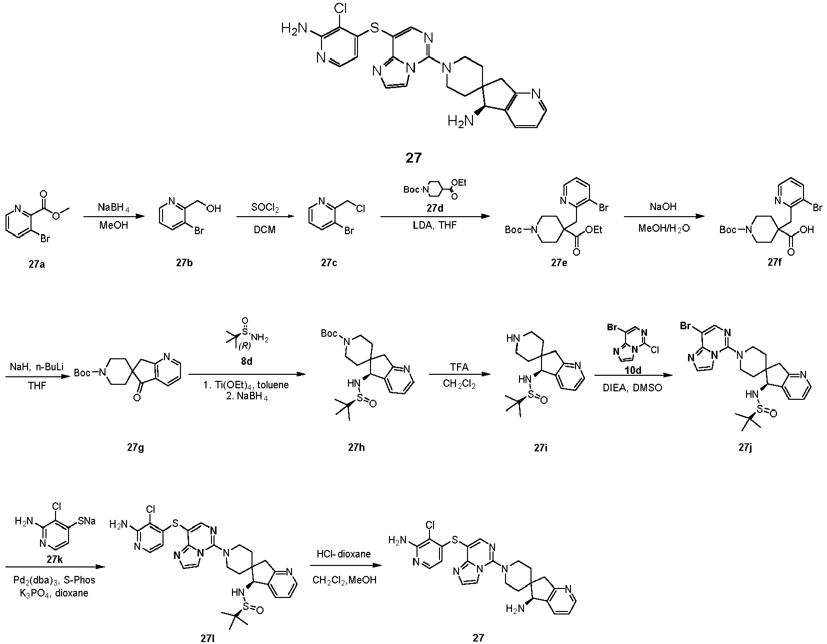

### Step 1

### (3-Bromopyridin-2-yl)methanol 27b

Compound **27a** (17.2 g, 79.6 mmol) was dissolved in methanol (50 mL), followed by the addition of sodium borohydride (15.1 g, 398 mmol) at 0°C. The reaction system was stirred at room temperature for 12 hours. After the reaction was completed, saturated aqueous ammonium chloride solution (600 mL) was added, and the reaction solution was extracted with ethyl acetate (200 mL×3). The organic phases were combined, washed with saturated sodium chloride solution (200 mL×2), dried over anhydrous sodium sulfate, and concentrated under reduced pressure to obtain compound **27b** (9.7 g, yield: 64.8%) as a white solid.
MS(ESI) m/z 187.8 [M+H]⁺
¹H NMR: (400MHz, MeOD-*d*₄) δ = 8.52 (d, J = 4.8 Hz, 1H), 8.01 (dd, J = 1.2, 8.0 Hz, 1H), 7.26 (dd, J = 4.4, 6.4 Hz, 1H), 4.77 (s, 2H).

### Step 2

### 3-Bromo-2-(chloromethyl)pyridine 27c

Compound **27b** (9.70 g, 51.6 mmol) was dissolved in dichloromethane (20 mL), followed by the addition of thionyl chloride (7.48 mL, 103 mmol) at room temperature. The reaction solution was stirred at room temperature for 3 hours. After the reaction was completed, saturated aqueous sodium bicarbonate solution (300 mL) was added at 0°C, and the reaction solution was extract with dichloromethane (80 mL×3). The organic phases were combined, washed with saturated sodium chloride solution (100 mL), dried over anhydrous sodium sulfate, and concentrated under reduced pressure to obtain compound **27c** (10.3 g, yield: 96.9%) as a pink oil.
MS(ESI) m/z 207.7 [M+H]⁺
¹H NMR (400MHz, MeOH-d4) δ = 8.55-8.45 (m, 1H), 8.12-7.99 (m, 1H), 7.37-7.21 (m, 1H), 4.84-4.80 (m, 2H).

### Step 3

### 1-(Tert-butyl) 4-ethyl 4-((3-bromopyridin-2-yl)methyl)piperidine-1,4-dicarboxylate 27e

Compound **27c** (9.97 g, 38.7 mmol) was dissolved in tetrahydrofuran (80 mL) under a nitrogen atmosphere, and LDA (13.5 mL, 2M solution in tetrahydrofuran and *n*-hexane) was added dropwise at -78°C. After completion of the addition, the reaction solution was stirred at -78°C for 1 hour. Compound **27d** (8.8 g, 35.07 mmol) was added dropwise at -78°C, and the reaction solution was stirred at -78°C for 9 hours. After the reaction was completed, saturated aqueous ammonium chloride solution (400 mL) was added, and the reaction solution was extracted with ethyl acetate (100 mL×3). The organic phases were combined, washed with saturated sodium chloride solution (100 mL×2), and dried over anhydrous sodium sulfate. The organic phase was concentrated under vacuum, the resulting crude product was purified by silica gel chromatography with petroleum ether and ethyl acetate as an eluent to obtain compound **27e** (14.8 g, yield: 89.4%) as a yellow oil.
MS(ESI) m/z 429.0 [M+H]⁺

### Step 4

### 4-((3-Bromopyridin-2-yl)methyl)-1-(tert-butoxycarbonyl)piperidine-4-carboxylic acid 27f

Compound **27e** (14.8 g, 34.6 mmol) was dissolved in methanol (3 mL), followed by the addition of aqueous sodium hydroxide solution (13.8 g, 346 mmol, dissolved in 40 mL of water) at 0°C. The reaction solution was stirred at 80°C for 12 hours. After the reaction was completed, the reaction solution was concentrated, to which ethyl acetate (300 mL) and water (300 mL) were added. Saturated aqueous sodium hydroxide solution (10 mL) was added to adjust the pH to 12. The aqueous phase was separated, and washed with ethyl acetate (80 mL×2). 2N hydrochloric acid (25 mL) was added to the resulting aqueous phase to adjust the pH to 3, and the aqueous phase was extracted with ethyl acetate (100 mL×3). The organic phases were combined, washed with saturated sodium chloride solution (150 mL), dried over anhydrous sodium sulfate, and concentrated under reduced pressure to obtain compound **27f** (11.4 g, yield: 82.4%) as a white solid.
MS(ESI) m/z 344.0 [M-56+H] ⁺

### Step 5

### Tert-butyl 5-oxo-5,7-dihydrospiro[cyclopenta[b]pyridine-6,4'-piperidine]-1'-carboxylate 27g

Sodium hydride (60% mixture with kerosene, 1.32 g, 33.1 mmol) was added to a solution of compound **27f** (11.0 g, 27.6 mmol) in tetrahydrofuran (100 mL) under a nitrogen atmosphere at -15°C. The reaction solution was stirred at -15°C for 1 hour. The reaction solution was cooled to -78°C, and 2.5 M solution (16.5 mL, 41.3 mmol) of n-butyllithium in *n*-hexane was added dropwise. The reaction solution was stirred at -78°C for 1 hour. After the reaction was completed, saturated aqueous ammonium chloride solution (400 mL) was added at 0°C, and the reaction solution was extract with ethyl acetate (100 mL×3). The organic phases were combined, washed with saturated sodium chloride solution (100 mL×2), dried over anhydrous sodium sulfate, and concentrated under vacuum. The resulting crude product was purified by silica gel chromatography with dichloromethane and methanol as an eluent to obtain compound **27g** (4.60 g, yield: 55.2%) as a white solid.
MS(ESI) m/z 246.9 [M-56+H]⁺
¹H NMR (400MHz, MeOH-d4) δ = 8.82 (dd, J = 1.6, 4.8 Hz, 1H), 8.12 (dd, J = 1.6, 7.6 Hz, 1H), 7.50 (dd, J = 4.8, 7.6 Hz, 1H), 4.08 (td, J = 3.6, 13.6 Hz, 2H), 3.25 (s, 2H), 3.12 (br s, 2H), 1.88-1.77 (m, 2H), 1.51 (br s, 2H), 1.49 (s, 9H).

### Step 6

### Tert-butyl

### (S)-5-((S)-tert-butylsulfinylamino)-5,7-dihydrospiro[cyclopenta[b]pyridine-6,4'-piperidi ne]-1'-carboxylate 27h

Tetraethyl titanate (9.4 mL, 44.6 mmol) was added to a solution of compound **27g** (4.50 g, 14.9 mmol) in anhydrous toluene (80 mL) under a nitrogen atmosphere. The reaction solution was stirred at room temperature for 10 minutes. Compound **8d** (5.4 g, 44.6 mmol) was added, and the reaction solution was reacted at 120°C for 5 hours. After cooling to 0°C, lithium borohydride (1.58 g, 89.2 mmol) was added, and the reaction solution was reacted for 30 minutes. The reaction solution was warmed up to room temperature and stirred for 1 hour. After the reaction was completed, methanol (20 mL) was added dropwise at 0°C. Water (100 mL) and ethyl acetate (100 mL) were added, and the reaction solution was stirred for 5 minutes. Suspended matter was filtered out by diatomaceous earth, and washed with ethyl acetate (300 mL) and water (300 mL). The organic phases were combined, washed with saturated sodium chloride solution (500 mL), dried over anhydrous sodium sulfate, and concentrated under reduced pressure. The resulting crude product was purified by silica gel chromatography with petroleum ether and ethyl acetate as an eluent to obtain compound **27h** (4.40 g, yield: 72.6%) as a yellow solid.
MS(ESI) m/z 408.1 [M+H]⁺

### Step 7

### (S)-N-((S)-5,7-Dihydrospiro[cyclopenta[b]pyridine-6,4'-piperidin]-5-yl)-2-methylpropa ne-2-sulfinamide 27i

Compound **27g** (4.40 g, 10.8 mmol) was dissolved in dichloromethane (15 mL), followed by the addition of trifluoroacetic acid (5 mL) at 0°C. The reaction solution was stirred at 0°C for 1 hour. The reaction solution was concentrated under reduced pressure to obtain the crude product, and 4M aqueous sodium hydroxide solution was added until pH = 11. The reaction solution was extracted with chloroform and isopropanol (volume ratio: 3:1). The organic phases were combined, dried over anhydrous sodium sulfate, and concentrated under reduced pressure to obtain the final product **27i** (3.32 g, yield: 100%) as a yellow oil.
MS(ESI) m/z 307.9 [M+H]⁺

### Step 8

### (S)-N-((S)-1'-(8-Bromoimidazo[1,2-c]pyrimidin-5-yl)-5,7-dihydrospiro[cyclopenta[b]p yridine-6,4'-piperidin]-5-yl)-2-methylpropane-2-sulfinamide 27j

Compound **27i** (3.30 mg, 10.7 mmol) and compound **10d** (2.50 g, 10.7 mmol) were dissolved in dimethyl sulfoxide (40 mL) under a nitrogen atmosphere, followed by the addition of diisopropylethylamine (7.7 g, 59.8 mmol). The reaction solution was stirred at 90°C for 2 hours. Ethyl acetate (50 mL) and water (100 mL) were added, and the reaction solution was extracted with ethyl acetate (50 mL×2). The organic phases were combined, washed with saturated sodium chloride solution (50 mL×3), dried over anhydrous sodium sulfate, and concentrated under reduced pressure. The resulting crude product was purified by silica gel chromatography with dichloromethane and methanol as an eluent to obtain compound **27j** (2.96 g, yield: 54.6%).
MS(ESI) m/z 503.1 [M+H]⁺
¹H NMR (400MHz, MeOH-d4) δ = 8.41 (d, *J*=4.8 Hz, 1H), 7.97 (s, 1H), 7.92 (d, *J*=1.5 Hz, 1H), 7.81 (d, *J*=7.5 Hz, 1H), 7.66 (d, *J*=1.5 Hz, 1H), 7.32 (dd, *J*=5.0, 7.5 Hz, 1H), 4.61 (br s, 2H), 3.95 - 3.83 (m, 2H), 3.30 - 3.21 (m, 2H), 2.99 (d, *J*=16.6 Hz, 1H), 2.40 (dt, *J*=4.0, 12.7 Hz, 1H), 2.14 (dt, *J*=3.6, 12.4 Hz, 1H), 1.82 (br d, *J*=13.3 Hz, 1H), 1.54 (br d, *J*=12.3 Hz, 1H), 1.36 (s, 9H).

### Step 9

### (S)-N-((S)-1'-(8-((2-Amino-3-chloropyridin-4-yl)thio)imidazo[1,2-c]pyrimidin-5-yl)-5, 7-dihydrospiro[cyclopenta[b]pyridine-6,4'-piperidin]-5-yl)-2-methylpropane-2-sulfinam ide 271

Compound **27j** (70 mg, 0.14 mmol) and compound **27k** (33 mg, 0.21 mmol, prepared according to the method disclosed in the patent application " WO2016203405 A1") were dissolved in 1,4-dioxane (1 mL) under a nitrogen atmosphere, followed by the addition of diisopropylethylamine (54 mg, 0.42 mmol) at room temperature. Tris(dibenzylideneacetone)dipalladium (13 mg, 0.014 mmol) and 2-dicyclohexylphosphino-2',6'-dimethoxybiphenyl (14 mg, 0.028 mmol) were added. The reaction solution was heated to 110°C and stirred for 12 hours. After the reaction was completed, the reaction solution was filtered. The filtrate was concentrated, and the residue was purified by C-18 reversed chromatography with water and methanol as an eluent to obtain compound **271** (45 mg, yield: 55.1%) as a brown oil.
MS(ESI) m/z 583.1 [M+H]⁺

### Step 10

### (S)-1'-(8-((2-Amino-3-chloropyridin-4-yl)thio)imidazo[1,2-c]pyrimidin-5-yl)-5,7-dihyd rospiro[cyclopenta[b]pyridine-6,4'-piperidin]-5-amine 27

Compound **271** (25 mg, 0.035 mmol) was dissolved in 1,4-dioxane, followed by the addition of a solution of hydrogen chloride in 1,4-dioxane (0.2 mL, 4 N) at 0°C. The reaction solution was reacted at 2 to 7°C for 1 hour. After the reaction was completed, water (30mL) was added, and the reaction solution was extract with ethyl acetate (15 mL×2). The organic phases were combined, washed with saturated sodium chloride solution (20 mL), dried over anhydrous sodium sulfate, and concentrated under reduced pressure. The residue was purified by C-18 reversed chromatography to obtain compound **27** (3.9 mg, yield: 19.0%).
MS(ESI) m/z 479.1 [M+H]⁺
¹H NMR: (400 MHz, MeOD-d4) δ = 8.38 (d, J= 4.8 Hz, 1H), 8.06 (s, 1H), 7.90-7.84 (m, 2H), 7.57 (s, 1H), 7.50 (d, J = 5.2 Hz, 1H), 7.30 (dd, J = 5.6, 7.6 Hz, 1H), 5.90 (d, J = 6.0 Hz, 1H), 4.16 (s, 1H), 4.06 (br d, J = 13.6 Hz, 2H), 3.48-3.36 (m, 2H), 3.30-3.24(m, 1H), 3.01(br d, J = 16.4 Hz, 1H), 2.20-2.01 (m, 2H), 1.80-1.71 (m, 1H), 1.61-1.53 (m, 1H).

### Example 28

### (S)-1'-(7-Amino-8-((2-(trifluoromethyl)pyridin-3-yl)thio)imidazo[1,2-c]pyrimidin-5-yl) -5,7-dihydrospiro[cyclopenta[b]pyridine-6,4'-piperidin]-5-amine

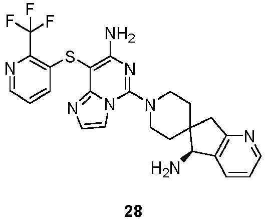

In accordance with the synthetic steps of Example **22,** compound **1g** was replaced with compound **27i,** and compound **14g** was replaced with compound **13h,** accordingly, the compound of Example **28** was prepared.
MS(ESI) m/z 513.2 [M+H]⁺
¹H NMR: (400MHz, MeOD-d4) δ 8.39-8.31 (m, 2H), 7.85 (d, J = 7.6 Hz, 1H), 7.51 (d, J = 1.6 Hz, 1H), 7.40-7.31 (m, 2H), 7.28 (dd, J = 5.2, 7.6 Hz, 1H), 7.20 (d, J = 1.2 Hz, 1H), 4.09 (s, 1H), 4.07-3.94 (m, 2H), 3.42-3.32 (m, 2H), 3.25 (d, J = 16.4 Hz, 1H), 2.95 (d, J = 16.8 Hz, 1H), 2.15-2.00 (m, 2H), 1.76-1.66 (m, 1H), 1.59-1.43 (m, 1H).

### Example 29

### (S)-1'-(7-Amino-8-((2-amino-3-chloropyridin-4-yl)thio)imidazo[1,2-c]pyrimidin-5-yl)-1 ,3-dihydrospiro[indene-2,4'-piperidin]-1-amine

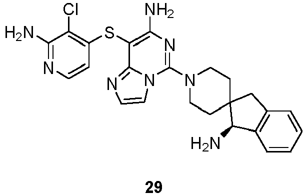

In accordance with the synthetic steps of Example **22,** compound **1g** was replaced with compound **8f,** and compound **14g** was replaced with compound **27k,** accordingly, the compound of Example **29** was prepared.
MS(ESI) m/z 493.1 [M+H]⁺
¹HNMR: (400MHz, CD₃OD) δ 7.71-7.13 (m, 7H), 5.98 (br s, 1H), 4.16-3.88 (m, 3H), 3.52-3.36 (m, 2H), 3.22-3.09 (m, 1H), 2.95-2.78 (m, 1H), 2.22-1.90 (m, 2H), 1.82-1.40 (m, 2H).

### Example 30

### (S)-1'-(7-Amino-8-((2-(trifluoromethyl)pyridin-3-yl)thio)imidazo[1,2-c]pyrimidin-5-yl) -1,3-dihydrospiro[indene-2,4'-piperidin]-1-amine

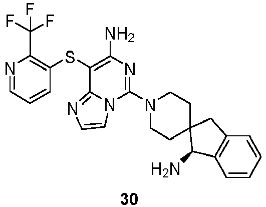

In accordance with the synthetic steps of Example **22,** compound **1g** was replaced with compound **8f,** and compound **14g** was replaced with compound **13h,** accordingly, the compound of Example **30** was prepared.
MS(ESI) m/z 512.2 [M+H]⁺
¹HNMR: (400MHz, CD₃OD) δ 8.34 (dd, J = 1.6, 4.0 Hz, 1H), 7.49 (d, J = 2.0 Hz, 1H), 7.41-7.37 (m, 1H), 7.36-7.31 (m, 2H), 7.25-7.17 (m, 4H), 4.04-3.92 (m, 3H), 3.39-3.33 (m, 1H), 3.31-3.28 (m, 1H), 3.16 (d, J = 15.6 Hz, 1H), 2.81 (d, J = 15.6 Hz, 1H), 2.12-1.92 (m, 2H), 1.66 (d, J = 13.2 Hz, 1H), 1.49 (d, J = 13.2 Hz, 1H).

### Example 31

### (S)-1'-(8-((2-(Trifluoromethyl)pyridin-3-yl)thio)imidazo[1,2-c]pyrimidin-5-yl)-1,3-dihy drospiro[indene-2,4'-piperidin]-1-amine

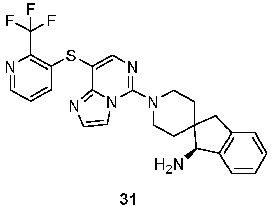

In accordance with the synthetic steps of Example **27,** compound **27i** was replaced with compound **8f,** and compound **27k** was replaced with compound **13h,** accordingly, the compound of Example **31** was prepared.
MS(ESI) m/z 497.1 [M+H]⁺
¹H NMR (400MHz, MeOH-d4) δ 8.39 (s, 1H), 8.05 (d, *J=* 5.6 Hz, 1H), 7.86 (d, *J* = 5.2 Hz, 1H), 7.56 (d, *J* = 5.2 Hz, 1H), 7.47-7.30 (m, 3H), 7.28-7.16 (m, 3H), 4.12-3.95 (m, 3H), 3.51-3.37 (m, 2H), 3.19 (dd, *J* = 5.2, 15.2 Hz, 1H), 2.86 (dd, *J* = 5.2, 15.2 Hz, 1H), 2.16-1.96 (m, 2H),1.77-1.66 (m, 1H), 1.62-1.49 (m, 1H).

### Example 32

### (S)-1'-(8-((2-Amino-3-chloropyridin-4-yl)thio)imidazo[1,2-c]pyrimidin-5-yl)-1,3-dihyd rospiro[indene-2,4'-piperidin]-1-amine

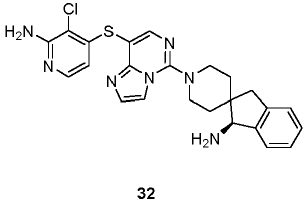

In accordance with the synthetic steps of Example **27,** compound **27i** was replaced with compound **8f,** accordingly, the compound of Example **32** was prepared.
MS(ESI) m/z 478.1 [M+H]⁺
¹H NMR (400MHz, MeOH-d4) δ 8.06 (s, 1H), 7.85 (d, *J* = 1.2 Hz, 1H), 7.56 (d, *J* = 1.6 Hz, 1H), 7.50 (d, *J* = 5.2 Hz, 1H), 7.44-7.40 (m, 1H), 7.28-7.23 (m, 3H), 5.89 (d, *J* = 5.6 Hz, 1H), 4.11 (s, 1H), 4.06-4.00 (m, 2H), 3.45-3.38 (m, 2H), 3.20 (d, *J* = 16.0 Hz, 1H), 2.92 (d, *J* = 16.0 Hz, 1H), 2.08-2.01 (m, 2H), 1.75-1.68 (m, 1H), 1.63-1.57 (m, 1H).

### Example 33

### (S)-1'-(8-(2,3-Dichlorophenyl)-7-methylimidazo[1,2-c]pyrimidin-5-yl)-5,7-dihydrospiro [cyclopenta[b]pyridine-6,4'-piperidin]-5-amine

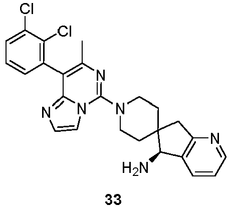

In accordance with the synthetic steps of Example **8,** compound **8f** was replaced with compound **27i,** accordingly, the compound of Example **33** was prepared.
MS(ESI) m/z 479.1 [M+H] ⁺
¹H NMR: (400MHz, CD₃OD) δ 8.35 (d, J = 4.8 Hz, 1H), 7.86 (d, J = 7.6 Hz, 1H), 7.72 (s, 1H), 7.64 (d, J = 8.0 Hz, 1H), 7.47-7.39 (m, 2H), 7.35-7.25 (m, 2H), 4.11 (s, 1H), 3.91 (d, J = 11.2 Hz, 2H), 3.39-3.31 (m, 1H), 3.29-3.23 (m, 1H), 2.95 (d, J = 16.4 Hz, 1H), 2.19 (s, 3H), 2.16-2.02 (m, 2H), 1.74 (d, J = 13.2 Hz, 1H), 1.53 (d, J = 13.2 Hz, 1H).

### Example 34

### (S)-1'-(8-((2-(Trifluoromethyl)pyridin-3-yl)thio)imidazo[1,2-c]pyrimidin-5-yl)-5,7-dihy drospiro[cyclopenta[b]pyridine-6,4'-piperidin]-5-amine

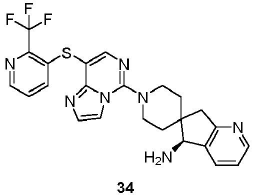

In accordance with the synthetic steps of Example **27,** compound **27k** was replaced with compound **13h,** accordingly, the compound of Example **34** was prepared.
MS(ESI) m/z 498.1 [M+H] ⁺
¹H NMR (400MHz, MeOH-d4) δ 8.39 (d, *J* = 4.8 Hz, 1H), 8.36 (d, *J* = 4.0 Hz, 1H), 8.05 (s, 1H), 7.88-7.83 (m, 1H), 7.56 (d, *J=* 1.6 Hz, 1H), 7.44 (d, *J=* 8.0 Hz, 1H), 7.36-7.26 (m, 2H), 4.11 (s, 1H), 4.08-4.01 (m, 2H), 3.47-3.36 (m, 2H), 3.27 (d, *J* = 16.4 Hz, 1H), 2.97 (d, *J* = 16.4 Hz, 1H), 2.18-2.02 (m, 2H), 1.75 (d, *J* = 12.8 Hz, 1H), 1.53 (d, *J* = 14.0 Hz, 1H)

### Example 35

### (S)-1'-(8-((3-Chloro-2-methoxypyridin-4-yl)thio)imidazo[1,2-c]pyrimidin-5-yl)-5,7-dih ydrospiro[cyclopenta[b]pyridine-6,4'-piperidin]-5-amine

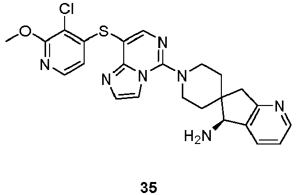

In accordance with the synthetic steps of Example **27,** compound **27k** was replaced with compound sodium 3-chloro-2-methoxypyridine-4-thiolate, accordingly, the compound of Example **35** was prepared.
MS(ESI) m/z 494.1 [M+H] ⁺
¹H NMR (400MHz, MeOH-d4) δ 8.38 (d, J=4.4 Hz, 1H), 8.06 (s, 1H), 7.90 - 7.83 (m, 2H), 7.67 (d, J=5.6 Hz, 1H), 7.55 (d, J=1.2 Hz, 1H), 7.30 (dd, J=5.2, 7.6 Hz, 1H), 6.21 (d, J=5.2 Hz, 1H), 4.17 (s, 1H), 4.07 (br d, J=13.2 Hz, 2H), 3.96 (s, 3H), 3.49-3.38 (m, 2H), 3.26 (d, J=16.8 Hz, 1H), 3.01 (d, J=16.8 Hz, 1H), 2.17 - 2.04 (m, 2H), 1.75 (d, J=13.6 Hz, 1H), 1.58 (d, J=13.6 Hz, 1H)

### Example 36

### (S)-1'-(8-((3-Chloro-2-(methylamino)pyridin-4-yl)thio)imidazo[1,2-c]pyrimidin-5-yl)-5, 7-dihydrospiro[cyclopenta[b]pyridine-6,4'-piperidin]-5-amine

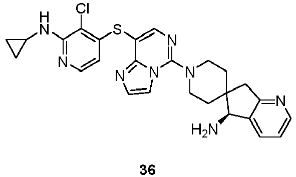

In accordance with the synthetic steps of Example **27,** compound **27k** was replaced with compound sodium 3-chloro-2-(cyclopropylamino)pyridine-4-thiolate, accordingly, the compound of Example **36** was prepared.
MS(ESI) m/z 519.3 [M+H] ⁺
¹H NMR: (400MHz, MeOD_d4) δ 8.36 (d, J = 4.4 Hz, 1H), 8.04 (s, 1H), 7.88-7.83 (m, 2H), 7.62 (d, J = 5.6 Hz, 1H), 7.56 (d, J = 1.6 Hz, 1H), 7.29 (dd, J = 5.2, 7.6 Hz, 1H), 5.91 (d, J = 5.6 Hz, 1H), 4.13-4.00 (m, 3H), 3.50-3.37 (m, 2H), 3.27-3.19 (m, 1H), 2.70-2.63 (m, 1H), 2.18-2.03 (m, 2H), 1.75 (br d, J = 14.0 Hz, 1H), 1.54 (br d, J = 13.2 Hz, 1H), 0.82-0.76 (m, 2H), 0.58-0.52 (m, 2H).

### Example 37

### (S)-1'-(8-((3-Fluoro-2-(methylamino)pyridin-4-yl)thio)imidazo[1,2-c]pyrimidin-5-yl)-5, 7-dihydrospiro[cyclopenta[b]pyridine-6,4'-piperidin]-5-amine

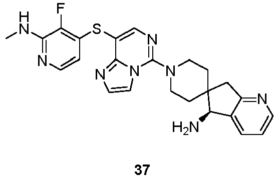

In accordance with the synthetic steps of Example **27,** compound **27k** was replaced with compound sodium 3-fluoro-2-(methylamino)pyridine-4-thiolate, accordingly, the compound of Example **37** was prepared.
MS(ESI) m/z 477.3 [M+H] ⁺
¹H NMR: (400MHz, MeOD_d4) δ 8.37 (d, J = 4.8 Hz, 1H), 8.04 (s, 1H), 7.90-7.82 (m, 2H), 7.57 (d, J = 1.6 Hz, 1H), 7.47 (d, J = 5.6 Hz, 1H), 7.30 (dd, J = 4.8, 7.2 Hz, 1H), 5.90 (t, J = 5.2 Hz, 1H), 4.14 (s, 1H), 4.03 (br d, J = 13.6 Hz, 2H), 3.47-3.35 (m, 2H), 3.27 (d, J = 16.8 Hz, 1H), 2.99 (d, J = 16.8 Hz, 1H), 2.92 (s, 3H), 2.14-2.01 (m, 2H), 1.74 (br d, J = 13.2 Hz 1H), 1.55 (br d, J = 14.0 Hz, 1H).

### Example 38

### (S)-1'-(8-((2-(Methylamino)pyridin-3-yl)thio)imidazo[1,2-c]pyrimidin-5-yl)-5,7-dihydr ospiro[cyclopenta[b]pyridine-6,4'-piperidin]-5-amine

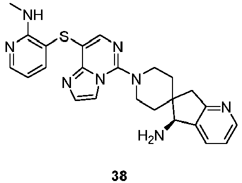

In accordance with the synthetic steps of Example **27,** compound **27k** was replaced with compound sodium 2-(methylamino)pyridine-3-thiolate, accordingly, the compound of Example **38** was prepared.
MS(ESI) m/z 459.2 [M+H] ⁺
¹H NMR: (400MHz, MeOD_d4) δ 8.34 (d, J = 4.0 Hz, 1H), 8.04 (dd, J = 1.6, 5.2 Hz, 1H), 7.85-7.74 (m, 3H), 7.64 (d, J = 1.6 Hz, 1H), 7.54 (s, 1H), 7.27 (dd, J = 5.2, 7.6 Hz, 1H), 6.58 (dd, J = 4.8, 7.6 Hz, 1H), 4.08 (s, 1H), 3.84 (br d, J = 13.2 Hz, 2H), 3.30-3.18 (m, 3H), 2.95-2.89 (m, 4H), 2.13-1.98 (m, 2H), 1.69 (br d, J = 13.6 Hz, 1H), 1.48 (br d, J = 14.0 Hz, 1H)

### Example 39

### (S)-1-(4-((5-(5-Amino-5,7-dihydrospiro[cyclopenta[b]pyridine-6,4'-piperidin]-1'-yl)imi dazo[1,2-c]pyrimidin-8-yl)thio)-3-chloropyridin-2-yl)-3-methylazetidin-3-ol

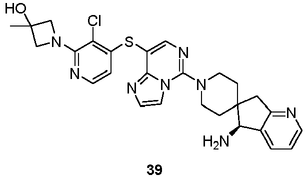

In accordance with the synthetic steps of Example **27,** compound **27k** was replaced with compound 1-(3-chloro-4-thiolpyridin-2-yl)-3-methylazetidin-3-ol, accordingly, the compound of Example **39** was prepared.
MS(ESI) m/z 549.1 [M+H] ⁺
¹H NMR: (400MHz, MeOD_d4) δ 8.36 (d, J = 4.0 Hz, 1H), 8.05 (s, 1H), 7.88-7.82 (m, 2H), 7.62 (d, J = 5.2 Hz, 1H), 7.56 (d, J = 1.6 Hz, 1H), 7.29 (dd, J = 5.2, 7.6 Hz, 1H), 5.98 (d, J = 5.6 Hz, 1H), 4.16-4.12 (m, 2H), 4.12-4.02 (m, 5H), 3.50-3.36 (m, 2H), 3.27 (br d, J = 16.8 Hz, 1H), 2.97 (d, J = 16.4 Hz, 1H), 2.18-2.02 (m, 2H), 1.75 (br d, J = 13.2 Hz, 1H), 1.55 (s, 4H).

### Example 40

### (S)-1-(4-((5-(5-Amino-5,7-dihydrospiro[cyclopenta[b]pyridine-6,4'-piperidin]-1'-yl)imi dazo[1,2-c]pyrimidin-8-yl)thio)-3-chloropyridin-2-yl)-3-methylazetidin-3-ol

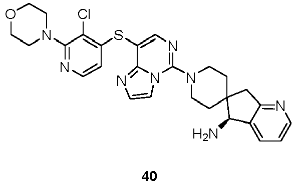

In accordance with the synthetic steps of Example **27,** compound **27k** was replaced with compound sodium 3-chloro-2-morpholinopyridine-4-thiolate, accordingly, the compound of Example **40** was prepared.
MS(ESI) m/z 549.2 [M+H] ⁺
¹H NMR: (400MHz, MeOD_d4) δ 8.36 (d, J =5.2 Hz, 1H), 8.07 (s, 1H), 7.83-7.89 (m, 2H), 7.79 (d, J =5.2 Hz, 1H), 7.56 (s, 1H), 7.29 (dd, J =5.2, 7.6 Hz, 1H), 6.27 (d, J = 5.6 Hz, 1H), 4.02-4.14 (m, 3H), 3.80-3.88 (m, 4H), 3.32-3.50 (m, 4H), 3.24-3.29 (m, 3H), 2.97 (br d, J = 16.0 Hz, 1H), 2.04-2.18 (m, 2H), 1.75 (br d, J = 12.8 Hz, 1H), 1.54 (br d, J = 12.8 Hz, 1H).

### Example 41

### (S)-1'-(8-((3-Chloro-2-(methylamino)pyridin-4-yl)thio)-7-methylimidazo[1,2-c]pyrimid in-5-yl)-5,7-dihydrospiro[cyclopenta[b]pyridine-6,4'-piperidin]-5-amine

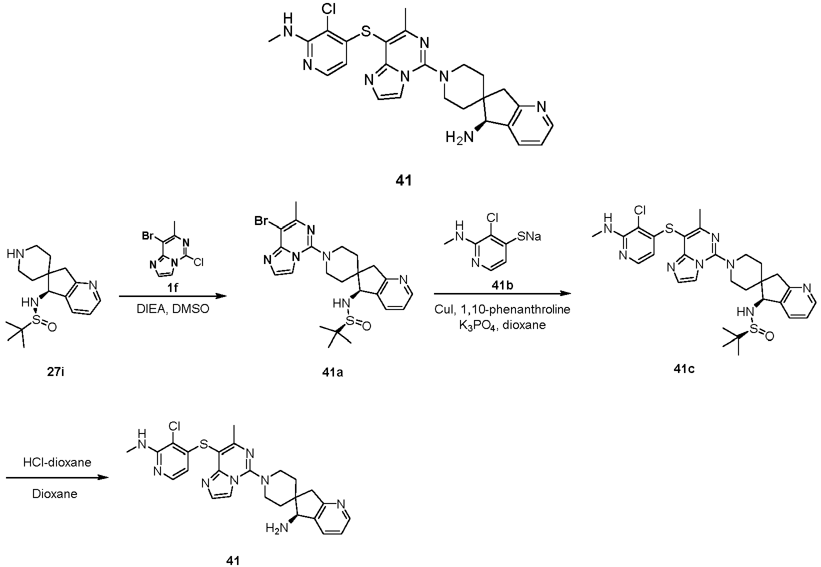

### Step 1

### (S)-N-((S)-1'-(8-Bromo-7-methylimidazo[1,2-c]pyrimidin-5-yl)-5,7-dihydrospiro[cyclo penta[b]pyridine-6,4'-piperidin]-5-yl)-2-methylpropane-2-sulfinamide 41a

Compound **27i** (260 mg, 0.85 mmol) and compound **1f** (271 mg, 1.10 mmol) were dissolved in dimethyl sulfoxide (3 mL), followed by the addition of diisopropylethylamine (547 mg, 4.23 mmol). The reaction solution was stirred at 90°C for 1 hour. Water (30 mL) was added, and the reaction solution was extracted with ethyl acetate (30 mL×3). The organic phases were combined, washed with saturated sodium chloride solution (50 mL×2), dried over anhydrous sodium sulfate, and filtered. The filtrate was concentrated under reduced pressure, and the resulting crude product was purified by silica gel chromatography with methanol and dichloromethane as an eluent to obtain compound **41a** (370 mg, yield: 84.5%) as a yellow solid.
MS(ESI) m/z 518.8 [M+H] ⁺
¹H NMR (400MHz, MeOH-d4) δ 8.40 (d, J = 4.8 Hz, 1H), 7.87 (d, J = 1.2 Hz, 1H), 7.82 (d, J = 7.6 Hz, 1H), 7.63 (d, J = 2.0 Hz, 1H), 7.33 (dd, J = 5.2, 7.6 Hz, 1H), 4.72-4.66 (m, 1H), 3.95-3.84 (m, 2H), 3.31-3.19 (m, 3H), 3.01-2.94 (m, 1H), 2.58 (s, 3H), 2.37 (dt, J = 4.0, 12.8 Hz, 1H), 2.13 (dt, J = 4.0, 12.8 Hz, 1H), 1.80 (d, J = 12.8 Hz, 1H), 1.52 (d, J = 14.0 Hz, 1H), 1.34 (s, 9H).

### Step 2

### (S)-N-((S)-1'-(8-((3-Chloro-2-(methylamino)pyridin-4-yl)thio)-7-methylimidazo[1,2-c] pyrimidin-5-yl)-5,7-dihydrospiro[cyclopenta[b]pyridine-6,4'-piperidin]-5-yl)-2-methylp ropane-2-sulfinamide 41c

Compound **41a** (50 mg, 0.10 mmol), compound **41b** (77 mg, 0.39 mmol, prepared according to the method disclosed in the patent application "WO2018013597 A1") and potassium phosphate (41 mg, 0.19 mmol) were dissolved in 1,4-dioxane (1 mL). The reaction solution was purged with nitrogen three times under stirring. 1,10-Phenanthroline (3.5 mg, 0.02 mmol) and cuprous iodide (1.8 mg, 0.01 mmol) were added rapidly under a nitrogen atmosphere. The reaction solution was purged with nitrogen three times, heated to 130°C and stirred for 10 hours. Water (50 mL) was added, and the reaction solution was extracted with ethyl acetate (40 mL×3). The organic phases were combined, washed with saturated sodium chloride solution (70 mL×2), dried over anhydrous sodium sulfate, and filtered. The filtrate was concentrated under reduced pressure, and the resulting crude product was purified by silica gel chromatography with dichloromethane and methanol as an eluent to obtain compound 41c (36 mg, yield: 58.5%) as a white solid.
MS(ESI) m/z 611.1 [M+H] ⁺

### Step 3

### (S)-1'-(8-((3-Chloro-2-(methylamino)pyridin-4-yl)thio)-7-methylimidazo[1,2-c]pyrimid in-5-yl)-5,7-dihydrospiro[cyclopenta[b]pyridine-6,4'-piperidin]-5-amine 41

Compound **41c** (36 mg, 0.059 mmol) was dissolved in dry dioxane (1 mL), followed by the dropwise addition of a solution (1 mL, 4 N) of hydrogen chloride in 1,4-dioxane at 10°C. The reaction solution was reacted at 10°C for 15 minutes. Water (30 mL) was added to the turbid reaction solution, which was then extracted with ethyl acetate (30 × 3). Saturated aqueous sodium bicarbonate solution was added to the aqueous phase to adjust pH = 8, and aqueous phase was extracted with chloroform (40 mL×4). All organic phases were combined, dried over anhydrous sodium sulfate, and filtered. The filtrate was concentrated under reduced pressure, and the resulting crude product was purified by high performance liquid chromatography to obtain compound **41** (2.3 mg, yield: 7.7%).
MS(ESI) m/z 507.3 [M+H] ⁺
1H NMR (400MHz, MeOH-d4) δ = 8.35 (d, *J* = 4.4 Hz, 1H), 7.85 (d, *J* = 7.6 Hz, 1H), 7.76 (d, *J* = 1.6 Hz, 1H), 7.58 (d, *J* = 5.6 Hz, 1H), 7.48 (d, *J* = 1.6 Hz, 1H), 7.29 (dd, *J* = 5.2 Hz, 7.6 Hz, 1H), 5.75 (d, *J* = 6.0 Hz, 1H), 4.12-4.00 (m, 3H), 3.46-3.34 (m, 2H), 3.29-3.23 (m, 1H), 3.01-2.92 (m, 4H), 2.55 (s, 3H), 2.17-2.01 (m, 2H), 1.74 (d, *J* = 13.6 Hz, 1H), 1.53 (d, *J* = 13.6 Hz, 1H).

### Example 42

### (S)-1'-(8-((2-Amino-3-chloropyridin-4-yl)thio)-7-methylimidazo[1,2-c]pyrimidin-5-yl)-5,7-dihydrospiro[cyclopenta[b]pyridine-6,4'-piperidin]-5-amine

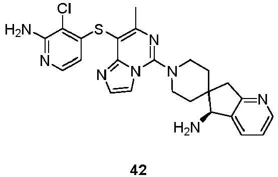

In accordance with the synthetic steps of Example **41,** compound **41b** was replaced with compound **27k,** accordingly, the compound of Example **42** was prepared.
MS(ESI) m/z 493.1 [M+H] ⁺
¹H NMR: (400MHz, CDCl3) δ 8.45 (d, J=4.4 Hz, 1H), 7.67 (d, J=7.6 Hz, 1H), 7.61 (d, J=5.2 Hz, 1H), 7.56 (d, J=1.2 Hz, 1H), 7.44 (d, J=1.2 Hz, 1H), 7.18 (dd, J=5.2, 7.6 Hz, 1H), 5.86 (d, J=5.2 Hz, 1H), 4.89 (s, 2H), 4.11 (s, 1H), 4.05 - 3.93 (m, 2H), 3.39 - 3.28 (m, 2H), 3.25 (d, J=16.4 Hz, 1H), 2.93 (d, J=16.4 Hz, 1H), 2.58 (s, 3H), 2.15 - 2.06 (m, 1H), 2.05 - 1.98 (m, 1H), 1.82 - 1.73 (m, 1H), 1.54 - 1.45 (m, 1H).

### Example 43

### (S)-1'-(8-((2-Amino-3-chloropyridin-4-yl)thio)imidazo[1,2-c]pyrimidin-5-yl)-5,7-dihyd rospiro[cyclopenta[b]pyridine-6,4'-piperidin]-5-amine

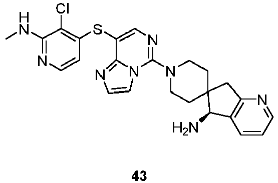

In accordance with the synthetic steps of Example **27,** compound **27k** was replaced with compound **41b,** accordingly, the compound of Example **43** was prepared.
MS(ESI) m/z 493.1 [M+H] ⁺
¹H NMR (400MHz, MeOH-d4) δ 8.36 (d, J=4.8 Hz, 1H), 8.04 (s, 1H), 7.88 - 7.81 (m, 2H), 7.60 - 7.52 (m, 2H), 7.29 (dd, J=5.2, 7.2 Hz, 1H), 5.83 (d, J=5.2 Hz, 1H), 4.12 (s, 1H), 4.05 (d, J=13.6 Hz, 2H), 3.48-3.36 (m, 2H), 3.29-3.23 (m, 1H), 3.01-2.95 (m, 1H), 2.94 (s, 3H), 2.18 - 2.03 (m, 2H), 1.74 (br d, J=13.2 Hz, 1H), 1.54 (br d, J=13.2 Hz, 1H).

### Example 44

### (S)-1'-(8-((3-Chloro-2-(methylamino)pyridin-4-yl)thio)imidazo[1,2-c]pyrimidin-5-yl)-2 -methyl-5,7-dihydrospiro[cyclopenta[b]pyridine-6,4'-piperidin]-5-amine

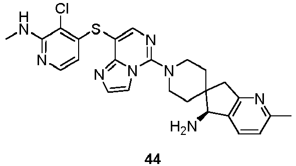

In accordance with the synthetic steps of Example **27,** compound **27a** was replaced with compound methyl 3-bromo-6-methylpicolinate, and compound **27k** was replaced with compound **41b,** accordingly, the compound of Example **44** was prepared.
MS(ESI) m/z 507.2 [M+H] ⁺
¹HNMR: (400MHz, CD₃OD) δ 8.04 (s, 1H), 7.85 (d, J = 1.2 Hz, 1H), 7.76 (d, J = 7.6 Hz, 1H), 7.59-7.55 (m, 2H), 7.18 (d, J = 8.0 Hz, 1H), 5.83 (d, J = 5.6 Hz, 1H), 4.15 (s, 1H), 4.12-3.96 (m, 2H), 3.48-3.37 (m, 2H), 3.23 (d, J = 16.8 Hz, 1H), 2.99 (d, J = 16.8 Hz, 1H), 2.94 (s, 3H), 2.53 (s, 3H), 2.14-2.04 (m, 2H), 1.73 (br d, J = 13.2 Hz, 1H), 1.62 (br d, J = 13.2 Hz, 1H).

### Biological Assay

The present invention will be further described with reference to the following test examples, but the examples should not be considered as limiting the scope of the present invention.

### Test Example 1. Determination of the in vitro activity of the compound of the present invention on SHP2 wild-type phosphatase

### 1. Experimental materials and instruments

| Instrument name | Manufacturer | Model |
|---|---|---|
| Thermostatic shaker | IMB | MB-1002A |
| Microplate reader | MDSpectraMax | M5 |

| Reagent name | Supplier | Art. No. |
|---|---|---|
| Shp2 | Genscript | N/A |
| Activated polypeptide | Genscript | N/A |
| DMSO | Sigma | C34557 |
| 1M HEPES | Thermofisher | 15630080 |
| 5M NaCl | Thermofisher | AM9760G |
| 2M KCl | Thermofisher | AM9640G |
| 1M DTT | Thermofisher | P2325 |
| 10% SDS | Thermofisher | AM9822 |
| 30% Brij™-35 | Thermofisher | 20150 |
| EDTA | Sigma | EDS-500G |
| Difmup | Invitrogen | TM 6567 |

### 2. Experimental procedures

0.2 nM recombinantly expressed full-length SHP2 (aa 1-593), 0.5 nM activated polypeptide IRS1 with double phosphorylation sites (sequence: H2N-LN(pY)IDLDLY(dPEG8)LST(pY)ASINFQK-amide) and a series of concentrations of the test compound (final concentrations were 1 µM, 0.3 µM, 0.1 µM, 0.03 µM, 0.01 µM, 0.003 µM, 0.001 µM, 0.0003 µM, 0.0001 µM, 0.00003 µM) were added to the phosphatase reaction solution (60 mM HEPES, pH 7.5 0.005% Brij-35, 75 mM NaCl, 75 mM KCl, 1 mM EDTA, 5 mM DTT). The reaction solution was shaked (350 rpm) at room temperature for 30 minutes. The reaction substrate DiFMUP with a final concentration of 30 µM was added, and the reaction solution was reacted at room temperature for 30 minutes. The phosphatase reaction was stopped by adding 5 µL of stop solution (60 mM HEPES, pH 7.5, 0.2% SDS). Ex358nm/Em455 fluorescence value was read on the fluorescence plate reader MD SpectraMax.

The IC₅₀ value of the compound was calculated using the four-parameter logit method. In the following formula, x represents the logarithmic form of the compound concentration, and F(x) represents the effect value (the inhibition rate of cell proliferation under the given concentration condition): F(x) = ((A-D)/(1+ ((x/C) ^B))) + D. A, B, C and D are four parameters. Different concentrations correspond to different inhibition rates, based on which an inverse curve was plotted, and the IC₅₀ of the inhibitor was calculated from the curve. The IC₅₀ of the compound was calculated with Primer premier 6.0.

The *in vitro* activity of the compound of the present invention on SHP2 was determined by the above test. SHP2 inhibitors SHP099 and RMC4550 having an oral activity were selected as positive drugs. The structure of compound SHP099 is published in the literature J. Med. Chem. 2016, 59, 7773-7782, and the compound was purchased from Shanghai Haoyuan Chemexpress Co., Ltd. The structure of compound RMC4550 is published in the literature Nature Cell Biology, 2018, 20, 1064-1073, and the compound was purchased from Shanghai AppTec Co., Ltd.

The resulting IC₅₀ values are shown in Table 1.

**Table 1. IC₅₀ of the compound of the present invention on SHP2 phosphatase**

| Example No. | IC₅₀ (nM) | Example No. | IC₅₀ (nM) |
|---|---|---|---|
| SHP099 | 79 | RMC4550 | 3.0 |
| 1 | 4.5 | 2 | 1.1 |
| The atropisomer with a RT of 1.495 minutes in Examples 3 and 4 | 0.7 | The atropisomer with a RT of 2.716 minutes in Examples 3 and 4 | 61.7 |
| 5 | 2.9 | 6 | 26.9 |
| 7 | 42.2 | 8 | 5.0 |
| 9 | 3.2 | 10 | 111 |
| 11 | 41.1 | 13 | 818 |
| 14 | 232 | 15 | 5.6 |
| 16 | 6.8 | 17 | 4.2 |
| 18 | 8.6 | 19 | 555 |
| 20 | 2.0 | 21 | 3.8 |
| 22 | 2.1 | 23 | 2.3 |
| 24 | 4.4 | 25 | 5.2 |
| 26 | 2.7 | 27 | 1.7 |
| 28 | 2.6 | 29 | 1.3 |
| 30 | 1.4 | 31 | 3.4 |
| 32 | 1.0 | 33 | 1.2 |
| 34 | 2.8 | 35 | 1.5 |
| 36 | 2.6 | 37 | 4.0 |
| 38 | 10.9 | 39 | 1.0 |
| 40 | 1.4 | 41 | 2.1 |
| 42 | 1.9 | 43 | 2.0 |

### Test Example 2. Determination of the in vitro activity of the compound of the present invention on SHP2 mutant E67K and E69K phosphatases

### 1. Experimental materials and instruments: see the above determination of the in vitro activity on wild-type phosphatase

### 2. Experimental procedures

Since SHP2E69K and E76K mutant proteins themselves have a background enzyme activity that does not depend on the activation of phosphorylated polypeptide, the inhibition of the compound on the enzyme activity of the mutant was determined in the presence and absence of the activated polypeptide.

0.2 nM recombinantly expressed full-length SHP2 (aa 1-593) with E69K and E76K (porduced by Novoprotein Scientific Inc.), 0.5 nM activated polypeptide IRS1 with double phosphorylation sites (sequence: H2N-LN(pY)IDLDLY(dPEG8)LST(pY)ASINFQK-amide) (added or not added) and a series of concentrations of the test compound (final concentrations were 1 µM, 0.3 µM, 0.1 µM, 0.03 µM, 0.01 µM, 0.003 µM, 0.001 µM, 0.0003 µM, 0.0001 µM, 0.00003 µM) were added to the phosphatase reaction solution (60 mM HEPES, pH 7.5 0.005% Brij-35, 75 mM NaCl, 75 mM KCl, 1 mM EDTA, 5 mM DTT). The reaction solution was shaked (350 rpm) at room temperature for 30 minutes. The reaction substrate DiFMUP with a final concentration of 30 µM was added, and the reaction solution was reacted at room temperature for 30 minutes. The phosphatase reaction was stopped by adding 5 µL of stop solution (60 mM HEPES, pH 7.5, 0.2% SDS). Ex358nm/Em455 fluorescence value was read on the fluorescence plate reader MD SpectraMax. The IC₅₀ value of the compound on inhibiting the enzyme activity of the mutant was calculated using the four-parameter logit method with reference to Test Example 1.

**Table 2. IC₅₀ of the compound of the present invention on SHP2 mutant E67K and E69K phosphatases**

| Example No. | SHP2 E69K the activated polypeptide was added IC₅₀ (nM) | SHP2 E69K the activated polypeptide was not added IC₅₀ (nM) | SHP2 E76K the activated polypeptide was added IC₅₀ (nM) | SHP2 E76K the activated polypeptide was not added IC₅₀ (nM) |
|---|---|---|---|---|
| SHP099 | > 10000 | 34 | > 10000 | 1540 |
| RMC4550 | 295 | 1.59 | > 10000 | 16.8 |
| 41 | 8.5 | 0.91 | 134 | 5.4 |
| 43 | 7.1 | 0.78 | 78 | 4.2 |

### Test Example 3. Determination of p-ERK in KYSE-520 cells

### 1. Experimental materials and instruments

| Instrument name | Manufacturer | Model |
|---|---|---|
| Cell counter | Applitech | NC200 |
| Biological safety cabinet of Class II | ESCO | AC2-6S1 |
| CO₂ incubator | Thermo | 160i |
| Centrifuge | Eppendorf | 5810R |
| Microplate reader | SpectraMax | M5 |

| Reagent name | Supplier | Item No. |
|---|---|---|
| RPMI 1640 | Gibco | A10491 |
| FBS | Gibco | 10099-141 |
| Trypsin-EDTA | Invitrogen | 12605-010 |
| DMSO | Sigma | C34557 |
| Phospho-ERK kit | Cisbio | 64ERKPEG |

### 2. Experimental procedures

KYSE-520 cells (Nanjing Cobioer Biosciences CO., Ltd.) in the logarithmic growth phase were inoculated (30,000 cells/well) in 1640 medium containing 10% of FBS, and adhered in a 96-well plate overnight (5% CO₂, 37°C). A series of concentrations of the test compound (10 µM, 3 µM, 1 µM, 0.3 µM, 0.1 µM, 0.03 µM, 0.01 µM, 0.003 µM, 0.001 µM) were added, and the reaction solution was reacted at 37°C, 5% CO₂ for 2 hours. The cell culture was stopped with cell lysate. The level of phosphorylated ERK in KYSE-520 cells was determined by a method based on homogeneous time-resolved fluorescence HTRF (Cisbio, 64ERKPEG). The fluorescence values (Ex337nm/Em625/665nm) were read on the compatible HTRF reader (MD SpectraMax). The IC₅₀ value of the compound on inhibiting intracellular phosphorylated ERK was calculated using the four-parameter logit method. In the following formula, x represents the logarithmic form of the compound concentration, and F(x) represents the effect value (the inhibition rate of cell proliferation under the given concentration condition): F(x) = ((A-D)/(1+ ((x/C) ^B))) + D. A, B, C and D are four parameters. Different concentrations correspond to different inhibition rates, based on which an inverse curve was plotted, and the IC₅₀ of the inhibitor was calculated from the curve. The IC₅₀ of the compound was calculated with Primer premier 6.0.

**Table 3. IC₅₀ of the compound of the present invention on p-ERK in KYSE-520 cells**

| Example No. | IC₅₀ (nM) | Example No. | IC₅₀ (nM) |
|---|---|---|---|
| RMC4550 | 34.6 | 1 | 383 |
| 2 | 40.5 | The atropisomer with a RT of 1.495 minutes in Examples 3 and 4 | 39.0 |
| 8 | 370 | 9 | 285 |
| 12 | 59.2 | 15 | 197 |
| 16 | 166 | 17 | 32.3 |
| 20 | 85.9 | 21 | 26.6 |
| 22 | 24.5 | 23 | 23.2 |
| 24 | 11.0 | 25 | 29.1 |
| 26 | 11.6 | 27 | 8.0 |
| 28 | 8.6 | 39 | 7.9 |
| 30 | 6.1 | 31 | 6.5 |
| 32 | 2.7 | 34 | 5.4 |
| 36 | 8.0 | 37 | 8.6 |
| 39 | 10.3 | 40 | 6.5 |
| 41 | 14.0 | 42 | 20.2 |
| 43 | 4.8 | | |

### Test Example 4. KYSE-520 cell proliferation experiment

### 1. Experimental materials and instruments

| Instrument name | Manufacturer | Model |
|---|---|---|
| Biological safety cabinet of Class II | Thermo | 1389 |
| Cell counter | Nexcelom | Cello meter |
| CO₂ incubator | Thermo | 3111 |
| Centrifuge | Eppendorf | 5810R |
| Microplate reader | PerkinElmer | 2105 |

| Reagent name | Supplier | Item No. |
|---|---|---|
| RPMI 1640 | Gibco | A10491 |
| FBS | Gibco | 10099-141 |
| Trypsin-EDTA (0.25%) | Invitrogen | 25200056 |
| DMSO | Sigma | C34557 |
| CellTiter-Glo | Promega | G7573 |

### 2. Experimental procedures

KYSE-520 cells in the logarithmic growth phase were adhered (600 cells/well) in 1640 medium containing 10% of FBS in a 96-well plate overnight, and then treated with a series of concentrations of the test compound (10 µM, 3 µM, 1 µM, 0.3 µM, 0.1 µM, 0.03 µM, 0.01 µM, 0.003 µM, 0.001 µM). The treated cell plate was incubated at 37°C, 5% CO₂ for 7 days. CellTiter-Glo (Promega, G7573) was used to determine the number of viable cells in each well of the treated plate. 100 µL of the detection reagent was added to each well, and the plate was incubated at room temperature for 10 minutes. Then, the fluorescence signal in each well was measured with Envision plate reader (PerkinElmer). The IC₅₀ value of KYSE-520 proliferation inhibition was calculated using the four-parameter logit method. In the following formula, x represents the logarithmic form of the compound concentration, and F(x) represents the effect value (the inhibition rate of cell proliferation under the given concentration condition): F(x) = ((A-D)/(1+ ((x/C) ^B))) + D. A, B, C and D are four parameters. The IC₅₀ value was further calculated as the compound concentration required for 50% proliferation inhibition in the best-fit curve with Primer premier 6.0.

**Table 4. IC₅₀ of the compound of the present invention on KYSE-520 cell proliferation**

| Example No. | IC₅₀ (nM) | Example No. | IC₅₀ (nM) |
|---|---|---|---|
| RMC4550 | 201 | 1 | 723 |
| 2 | 186 | The atropisomer with a RT of 1.495 minutes in Examples 3 and 4 | 126 |
| 8 | 151 | 9 | 785 |
| 12 | 346 | 15 | 312 |
| 16 | 548 | 17 | 179 |
| 20 | 178 | 21 | 111 |
| 22 | 102 | 23 | 51.6 |
| 24 | 75.9 | 26 | 62.0 |
| 27 | 31.5 | 28 | 21.6 |
| 29 | 14.0 | 30 | 8.9 |
| 31 | 52.0 | 32 | 12.9 |
| 34 | 92.5 | 36 | 40.5 |
| 37 | 37.0 | 39 | 24.1 |
| 40 | 27.3 | 41 | 58.3 |
| 42 | 65.1 | 43 | 30.4 |

### Test Example 5. hERG current inhibition experiment

### 1. Experimental materials and instruments

| Instrument name | Manufacturer | Model |
|---|---|---|
| Manual patch clamp system | HEKA | EPC-10 |

| | | |
|---|---|---|
| Reagent name | Supplier | Item No. |
| NaCl | Sigma | S1679-1KG |
| KCl | Sigma | 31248-100G |
| CaCl2 (1M solution) | Sigma | 21114-1L |
| MgCl2·6H2O | Sigma | M7304-100G |
| HEPES | Sigma | H3375-1KG |
| Glucose | Sigma | G8270-1KG |
| EGTA | Sigma | 03777-50G |
| Na2-ATP | Sigma | A-7699-5G |
| NaOH (2M solution) | Sigma | 35254-1L |
| KOH | Sigma | 232041-50G |

### 2. Experimental procedures

In this experiment, whole-cell current recording was performed using a manual patch clamp system (HEKA EPC-10 signal amplification and digital conversion system, purchased from HEKA Electronic, Germany). The round glass slide of which surface CHO hERG cells (provided by Sophion Bioscience Inc., Denmark, the cell generation number was P21) were grown on was placed in an electrophysiological recording slot under an inverted microscope. The recording slot was continuously perfused with extracellular fluid (approximately 1 mL per minute). Conventional whole-cell patch clamp current recording technique was used in the experiment. The experiments were performed at normal room temperature (∼25 °C). The cells were clamped at a voltage of -80 mV. Cell patch clamp voltage was depolarized to +20 mV to activate hERG potassium channel, and to -50 mV after 5 seconds to eliminate inactivation and generate tail currents. The tail current peak was used as a value of hERG current. When the hERG potassium current recorded in the above steps became steady under continuous perfusion of the extracellular fluid in the recording slot, the drug to be tested could be added to the perfusion, until the inhibition effect of the drug on hERG current reached a steady state. Generally, the overlapping of most recent three consecutive current recording lines was used as a criterion to determine whether the state was stable. After reaching the steady state, the recording slot was perfused with extracellular fluid until the hERG current returned to the value before the drug adding. The test data was analyzed by HEKA Patchmaster (V2x73.2), Microsoft Excel and the data analysis software provided by Graphpad Prism 5.0.

**Table 5. IC₅₀ of the compound of the present invention on CHO cell hERG**

| Example No. | hERG IC₅₀ (µM) |
|---|---|
| 1 | 2.13 |
| 2 | 2.61 |
| 9 | 1.91 |
| 17 | 12.3 |
| 24 | > 30 |
| 36 | 3.97 |
| 41 | 10.2 |
| 42 | > 30 |
| 43 | 10.3 |

### Pharmacokinetics Evaluation

### Test Example 6. Pharmacokinetics assay of the compound of the present invention

### 1. Abstract

Rats were used as test animals. The drug concentration in plasma at different time points was determined by LC/MS/MS method after intravenous administration or intragastrical administration of the compound of the present invention to rats. The pharmacokinetic behavior of the compound of the present invention was studied and evaluated in rats.

### 2. Test protocol

### 2.1 Test compounds

The atropisomer with a RT of 1.495 minutes in Examples 3 and 4, compounds of Example 17, Example 41 and Example 43.

### 2.2 Test animals

Healthy male SD rats (6-8 weeks old), 3 rats per group.

### 2.3 Preparation of the test compound

Intravenous administration: A certain amount of the test compound was weighed, to which 10% by volume of N,N-dimethylacetamide, 33% by volume of triethylene glycol and 57% by volume of water were added to prepare a 1 mg/mL colorless, clear and transparent solution;

Intragastrical administration: A certain amount of the test compound was weighed, to which 0.5% by mass of hypromellose, 0.1% by volume of Tween 80 and 99.4% by volume of water were added to prepare a 1 mg/mL white suspension.

### 2.4 Administration

After an overnight fast, the SD rats were intravenously administered the test compound at an administration dose of 1 mg/kg, or intragastrically administered the test compound at an administration dose of 5 mg/kg.

### 3. Process

The rats were intravenously administered the compound of the present invention. 0.2 ml of blood was taken from the jugular vein at 0.083, 0.25, 0.5, 1, 2, 4, 8 and 24 hours after the administration. The samples were placed in tubes containing EDTA-K2, and centrifuged at 4000 rpm and 4°C for 5 minutes to separate the blood plasma. The plasma samples were stored at -75°C.

Or, the rats were intragastrically administered the compound of the present invention. 0.2 ml of blood was taken from the jugular vein at 0.25, 0.5, 1, 2, 4, 8 and 24 hours after the administration. The samples were placed in tubes containing EDTA-K2, and centrifuged at 4000 rpm and 4°C for 5 minutes to separate the blood plasma. The plasma samples were stored at -75°C.

The content of the test compound in the plasma of rat after intragastrical administration of the test compound at different concentrations was determined: 50 µL of rat plasma at each time after administration was taken, to which 200 µL of a solution (50 ng/mL) of internal standard dexamethasone in acetonitrile was added. The plasma was vortex-mixed for 30 seconds, and centrifuged at 4700 rpm and 4°C for 15 minutes. The supernatant was taken from the plasma samples, and a three-fold dilution was carried out by adding water. 2.0 µL of the supernatant was used for LC/MS/MS analysis.

### 4. Results of pharmacokinetic parameters

Pharmacokinetic parameters of the compounds of the present invention are shown below:

| No. | | Pharmacokinetics assay | | | | | |
|---|---|---|---|---|---|---|---|
| | | Maximum plasma concentrati on | Area under curve | Half-life | Clearance | Apparent distributio n volume | Bioavailabi lity |
| | | Cmax (ng /mL) | AUC (ng /mL*h) | T_{1/2} (h) | CL_obs (ml/min/kg) | Vss_obs (mL/kg) | F (%) |
| The atropisomer with a RT of 1.495 minutes in Examples 3 and 4 | IV 1 mg/kg | - | 3132±300 | 5.50±0.59 | 5.13±0.59 | 2030±150 | 82.6±17.8 |
| | PO 5 mg/kg | 1223+220 | 12527+2468 | 6.18±0.61 | - | - | |
| Example 17 | IV 1 mg/kg | - | 764±196 | 2.83±0.93 | 21.5±5.2 | 3800±100 | 80.3+8.5 |
| | PO 5 mg/kg | 408±77 | 3211±351 | 3.42±0.37 | - | - | |
| Example 41 | IV 1 mg/kg | - | 134±210 | 5.33±0.11 | 14.6±2.6 | 4750+720 | 67.6±10.9 |
| | PO 5 mg/kg | 339±88 | 3881±631 | 4.22±0.11 | - | - | |
| Example 43 | IV 1 mg/kg | - | 6642±1359 | 4.71±0.27 | 2.52±0.52 | 765+82 | |
| | PO 5 mg/kg | 2513±405 | 25155+2504 | 4.17±0.14 | - | - | 75.4±7.5 |

Conclusion: The compounds of the present invention are well absorbed, and have a pharmacokinetic advantage.

## Claims

1. A compound of formula (I) 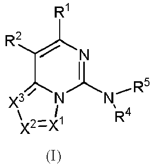 or a tautomer, mesomer, racemate, enantiomer, diastereomer, atropisomer thereof, or mixture thereof, or a pharmaceutically acceptable salt thereof,
wherein:
R¹ is selected from the group consisting of hydrogen atom, deuterium atom, hydroxy, cyano, nitro, halogen, carboxy, alkyl, alkoxy, haloalkyl, haloalkoxy, amino, alkenyl and hydroxyalkyl;
R² is 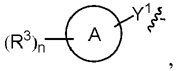
Y¹ is selected from the group consisting of -S-, -NH-, -S(O)₂-, -S(O)₂-NH-, -C(=CH₂)-, -S(O)- and a bond;
ring A is selected from the group consisting of cycloalkyl, heterocyclyl, aryl and heteroaryl, wherein the cycloalkyl, heterocyclyl, aryl and heteroaryl are each independently a 5 to 12 membered monocycle or polycycle;
each R³ is independently selected from the group consisting of hydrogen atom, deuterium atom, halogen, alkyl, alkoxy, cyano, amino, nitro, carboxy, hydroxy, hydroxyalkyl, C₃₋₈ cycloalkyl, 3 to 12 membered heterocyclyl, aryl, heteroaryl, C₂₋₆ alkenyl, C₄₋₈ cycloalkenyl, C₂₋₆ alkynyl, -CHR^{a}R^{b}, -NR^{a}R^{b}, -alkenyl-NR^{a}R^{b}, -alkenyl-O-R^{a}, -alkenyl-C(O)₂R^{a}, -alkenyl-R^{a}, -alkenyl-CO-NR^{a}R^{b}, -alkenyl-NR^{a}-CO-NR^{a}R^{b}, -alkenyl-NR^{a}-C(O)R^{b}, -C(O)NR^{a}R^{b}, -C(O)R^{a}, -CO-alkenyl-NR^{a}R^{b}, -NR^{a}C(O)R^{b}, -C(O)2R^{a}, -O-alkenyl-CO-OR^{a}, -O-alkenyl-CO-NR^{a}R^{b}, -O-alkenyl-NR^{a}R^{b}, -OR^{a}, -SR^{a}, -NR^{a}-CO-NR^{a}R^{b}, -NR^{a}-alkenyl-NR^{a}R^{b}, -NR^{a}-alkenyl-R^{b}, -NR^{a}S(O)2R^{b}, -NR^{a}S(O)R^{b}, -NR^{a}S(O)₂NR^{a}R^{b}, -NR^{a}S(O)NR^{a}R^{b}, -S(O)₂NR^{a}R^{b}, -S(O)NR^{a}R^{b}, -S(O)R^{a}, -S(O)₂R ^{a}, -P(O)R^{a}R^{b}, -N(S(O)R^{a}R^{b}) and -S(O)(NR^{a})R^{b}, wherein the alkyl, alkoxy, aryl and heteroaryl are each independently optionally further substituted by one or more substituents selected from the group consisting of halogen, hydrogen atom, deuterium atom, cyano, amino, nitro, carboxy, hydroxy, hydroxyalkyl, alkyl, alkoxy, haloalkyl and haloalkoxy;
n is selected from the group consisting of 0, 1, 2, 3, 4 and 5;
X¹, X² and X³ are each independently selected from the group consisting of CR^{c} and N, wherein at least one of them is N, and preferably X¹ is CR^{c};
R^{c} is selected from the group consisting of hydrogen atom, deuterium atom, C₁₋₆ alkyl, C₁₋₆ alkoxy, C₁₋₆ alkylthio, amino, nitro, hydroxy, carbonyl, carboxy, halogen and cyano;
R⁴ is selected from the group consisting of hydrogen, C₁₋₆ alkyl, 3 to 12 membered monocyclic heterocyclyl or polycyclic heterocyclyl and C₃₋₈ cycloalkyl, wherein the alkyl, heterocyclyl and cycloalkyl are each independently optionally substituted by one or more substituents selected from the group consisting of halogen, hydroxy, C₁₋₃ alkyl, amino, alkylamino, hydroxyalkyl and alkoxy;
R⁵ is selected from the group consisting of hydrogen, hydroxy, C₁₋₆ alkyl and C₃₋₈ cycloalkyl, wherein the alkyl or cycloalkyl is optionally substituted by one or more amino; or
R⁴ and R⁵ together with the nitrogen atom to which they are attached form a 3 to 12 membered monocyclic heterocycle or polycyclic heterocycle, wherein the monocyclic heterocycle or polycyclic heterocycle is optionally substituted by one or more substituents selected from the group consisting of halogen, hydroxy, halogen-substituted or unsubstituted C₁₋₆ alkyl, amino, alkoxy, hydroxyalkyl, aryl, heteroaryl, heterocyclyl, alkylamino, halogen-substituted or unsubstituted alkoxy and -NR^{a}S(O)NR^{a}R^{b}; or
R⁴ and R⁵ together with the nitrogen atom to which they are attached form a structure of 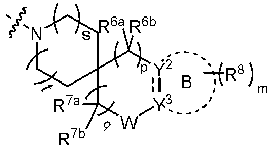
wherein s and t are each independently selected from the group consisting of 0 and 1;
R^{6a} and R^{6b} are each independently selected from the group consisting of hydrogen atom, deuterium atom, fluorine atom, amino, hydroxy, cyano, nitro, carboxy, fluorine-substituted or unsubstituted alkyl and fluorine-substituted or unsubstituted alkoxy; or R^{6a} and R^{6b} together with the carbon atom to which they are attached form a CO, C=NH, C=N-OH, 3 to 12 membered heterocyclyl or C₃₋₈ cycloalkyl;
p is selected from the group consisting of 0, 1, 2, 3 and 4;
R^{7a} and R^{7b} are each independently selected from the group consisting of hydrogen atom, deuterium atom, fluorine atom, amino, hydroxy, cyano, nitro, carboxy, fluorine-substituted or unsubstituted alkyl, fluorine-substituted or unsubstituted alkoxy and -NR^{a}S(O)NR^{a}R^{b};
or R^{7a} and R^{7b} together with the carbon atom to which they are attached form a 3 to 12 membered heterocyclyl, 5 to 10 membered heteroaryl, C₃₋₈ cycloalkyl and C=NR^{7c}, wherein the rings are optionally substituted;
R^{7c} is selected from the group consisting of hydrogen atom, deuterium atom and C₁₋₆ alkyl;
q is selected from the group consisting of 0, 1, 2, 3 and 4;
W is absent or is selected from the group consisting of -O-, -S- and -NR^{w}-;
R^{w} is selected from the group consisting of hydrogen atom, halogen, amino, hydroxy, cyano, nitro, carboxy, -C(O)C₁₋₆ alkyl, -C(O)₂C₁₋₆ alkyl, C₁₋₆ alkyl ether, halogen-substituted or unsubstituted C₁₋₆ alkyl and halogen-substituted or unsubstituted C₁₋₆ alkoxy;
ring B is absent or is a 3 to 10 membered ring;
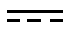 is a single bond or double bond;
when ring B is absent, then Y² is CR^{2a}R^{2b}, NR^{2a} or O, Y³ is CR^{3a}R^{3b}, NR^{3a} or O;
when ring B is a 3 to 10 membered ring, then
3) Y² is CR^{2a} or N, Y³ is CR^{3a} or N, 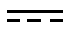 is a single bond; or
4) Y² is C and Y³ is C, 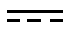 is a double bond;
R^{2a}, R^{2b}, R^{3a} and R^{3b} are each independently selected from the group consisting of hydrogen atom, deuterium atom, halogen, cyano, amino, nitro, carboxy, hydroxy, hydroxyalkyl, C₃₋₈ cycloalkyl, 3 to 12 membered heterocyclyl, aryl, heteroaryl, C₂₋₆ alkenyl, C₄₋₈ cycloalkenyl, C₂₋₆ alkynyl, -NR^{a}R^{b}, -alkenyl-NR^{a}R^{b}, -alkenyl-O-R^{a}, -alkenyl-C(OhR^{a}, -alkenyl-R^{a}, -alkenyl-CO-NR^{a}R^{b}, -alkenyl-NR^{a}-CO-NR^{a}R^{b}, -alkenyl-NR^{a}-C(O)R^{b}, -C(O)NR^{a}R^{b}, -C(O)R^{a}, -CO-alkenyl-NR^{a}R^{b}, -NR^{a}C(O)R^{b}, -C(O)₂R^{a}, -O-alkenyl-CO-OR^{a}, -O-alkenyl-CO-NR^{a}R^{b}, -O-alkenyl-NR^{a}R^{b}, -OR^{a}, -SR^{a}, -NR^{a}-CO-NR^{a}R^{b}, -NR^{a}-alkenyl-NR^{a}R^{b}, -NR^{a}-alkenyl-R^{b}, -NR^{a}S(O)₂R^{b}, -NR^{a}S(O)R^{b}, -NR^{a}S(O)₂NR^{a}R^{b}, -NR^{a}S(O)NR^{a}R^{b}, -S(O)₂NR^{a}R^{b}, -S(O)NR^{a}R^{b}, -S(O)R^{a}, -S(O)₂R^{a}, -P(O)R^{a}R^{b}, -N(S(O)R^{a}R^{b}) and -S(O)(NR^{a})R^{b}, wherein the aryl and heteroaryl are each independently optionally further substituted by one or more substituents selected from the group consisting of halogen, hydrogen atom, deuterium atom, cyano, amino, nitro, carboxy, hydroxy, hydroxyalkyl, alkyl, alkoxy, haloalkyl and haloalkoxy;
R^{a} and R^{b} are each independently selected from the group consisting of hydrogen, deuterium atom, halogen, amino, hydroxy, cyano, nitro, carboxy, alkyl, alkoxy, haloalkyl, haloalkoxy, C₃₋₈ cycloalkyl, 5 to 10 membered heteroaryl and aryl, wherein the aryl and heteroaryl are each independently optionally further substituted by one or more substituents selected from the group consisting of halogen, hydrogen atom, deuterium atom, cyano, amino, nitro, carboxy, hydroxy, hydroxyalkyl, alkyl, alkoxy, haloalkyl and haloalkoxy;
m is selected from the group consisting of 0, 1, 2, 3 and 4; and
each R⁸ is independently selected from the group consisting of hydrogen atom, deuterium atom, halogen, amino, hydroxy, cyano, nitro, carboxy, C₁₋₆ alkyl and C₁₋₆ alkoxy;
or two R⁸ are attached together to form a phenyl, 5 membered heteroaryl, 6 membered heteroaryl or 3 to 6 membered heterocyclyl, wherein each ring is optionally substituted by one or more substituents selected from the group consisting of halogen, amino, hydroxy, cyano, nitro and C₁₋₆ alkyl.

2. The compound according to claim 1, wherein R⁴ and R⁵ together with the nitrogen atom to which they are attached form a structure of 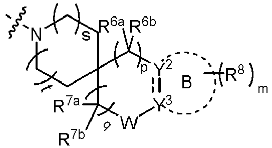
wherein s and t are each independently selected from the group consisting of 0 and 1;
R^{6a} and R^{6b} are each independently selected from the group consisting of hydrogen atom, deuterium atom, C₁₋₆ alkyl and C₁₋₆ alkoxy; or R^{6a} and R^{6b} together with the carbon atom to which they are attached form a 3 to 12 membered heterocyclyl or C₃₋₈ cycloalkyl;
p is selected from the group consisting of 0, 1 and 2;
R^{7a} and R^{7b} are each independently selected from the group consisting of hydrogen atom, deuterium atom, amino, C₁₋₆ alkyl and -NR^{a}S(O)NR^{a}R^{b}, wherein R^{a} and R^{b} are as defined in claim 1;
q is 1 or 2;
W is absent;
ring B is absent or is a 3 to 10 membered ring;
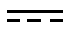 is a single bond or double bond;
when ring B is absent, then Y² is CR^{2a}R^{2b} or O, Y³ is CR^{3a}R^{3b}; or
when ring B is a 3 to 10 membered ring, then
Y² is CR^{2a} or N, Y³ is CR^{3a} or N, 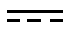 is a single bond; or
Y² is C and Y³ is C, 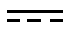 is a double bond;
R^{2a}, R^{2b} and R^{3a} are each independently selected from the group consisting of hydrogen atom, deuterium atom and C₁₋₆ alkyl;
m is selected from the group consisting of 0, 1, 2, 3 and 4; and
each R⁸ is independently selected from the group consisting of hydrogen atom, deuterium atom, halogen, amino, hydroxy, cyano, nitro, carboxy, C₁₋₆ alkyl and C₁₋₆ alkoxy;
or two R⁸ are attached together to form a phenyl, 5 membered heteroaryl, 6 membered heteroaryl or 3 to 6 membered heterocyclyl, wherein each ring is optionally substituted by one or more substituents selected from the group consisting of halogen, amino, hydroxy, cyano, nitro and C₁₋₆ alkyl.

3. The compound according to claim 1 or 2, wherein R⁴ and R⁵ together with the nitrogen atom to which they are attached form a structure of 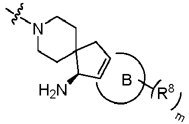 wherein:
ring B is selected from the group consisting of benzene ring, 5 membered heteroaromatic ring and 6 membered heteroaromatic ring, preferably a benzene ring or pyridine ring;
each R⁸ is independently selected from the group consisting of hydrogen atom, deuterium atom, halogen, cyano, C₁₋₆ alkyl and C₁₋₆ alkoxy; and
m is selected from the group consisting of 0, 1, 2, 3 and 4.

4. The compound according to claim 1, wherein R⁴ and R⁵ together with the nitrogen atom to which they are attached form a structure of 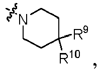
wherein R⁹ and R¹⁰ are each independently selected from the group consisting of hydrogen atom, deuterium atom, hydroxy, C₁₋₆ alkyl, C₁₋₆ alkoxy, halogen, C₁₋₆ hydroxyalkyl, aryl, heteroaryl, heterocyclyl, amino, C₁₋₆ alkylamino and -NR^{a}S(O)NR^{a}R^{b}, preferably selected from the group consisting of hydrogen atom, deuterium atom, C₁₋₆ alkyl, amino and -NR^{a}S(O)NR^{a}R^{b}; or
R^{a} and R^{b} are as defined in claim 1.

5. The compound according to any one of claims 1 to 4, wherein
Y¹ is -S- or a bond;
ring A is an aryl or heteroaryl;
each R³ is independently selected from the group consisting of hydrogen atom, deuterium atom, halogen, C₁₋₆ alkyl, haloC₁₋₆ alkyl, haloC₁₋₆ alkoxy, C₁₋₆ alkoxy, cyano, amino, nitro, carboxy, hydroxy and phenyl, wherein the phenyl is optionally further substituted by one or more substituents selected from the group consisting of halogen, hydrogen atom, deuterium atom, cyano, amino, nitro, carboxy, hydroxy, hydroxyalkyl, alkyl, alkoxy, haloalkyl and haloalkoxy; each R³ is preferably selected from the group consisting of hydrogen atom, deuterium atom, halogen, haloC₁₋₆ alkyl, C₁₋₆ alkyl, C₁₋₆ alkoxy, haloC₁₋₆ alkoxy and phenyl, wherein the phenyl is optionally further substituted by one or more substituents selected from the group consisting of halogen, hydrogen atom, deuterium atom, cyano, amino, nitro, carboxy, hydroxy, hydroxyalkyl, alkyl, alkoxy, haloalkyl and haloalkoxy; and
n is selected from the group consisting of 0, 1, 2, 3, 4 and 5.

6. The compound according to any one of claims 1 to 5, wherein X¹, X² and X³ are each independently selected from the group consisting of CR^{c} and N, wherein at least one of them is N, preferably X¹ is CR^{c}, and R^{c} is a hydrogen atom.

7. The compound according to claim 6, wherein X¹ and X² are both CR^{c} and X³ is N, or X¹ is CR^{c} and X² and X³ are both N, and R^{c} is a hydrogen atom.

8. The compound according to any one of claims 1 to 7, wherein R¹ is selected from the group consisting of hydrogen atom, deuterium atom, C₁₋₆ alkyl, C₁₋₆ alkoxy, amino and hydroxy.

9. The compound according to claim 1 or 4, wherein
R¹ is selected from the group consisting of hydrogen atom, deuterium atom, C₁₋₆ alkyl and amino;
Y¹ is -S- or a bond;
ring A is an aryl or heteroaryl;
each R³ is independently selected from the group consisting of hydrogen atom, deuterium atom, halogen, haloC₁₋₆ alkyl, C₁₋₆ alkyl, C₁₋₆ alkoxy, haloC₁₋₆ alkoxy and substituted phenyl;
n is selected from the group consisting of 0, 1, 2, 3, 4 and 5;
X¹, X² and X³ are each independently selected from the group consisting of CR^{c} and N, wherein at least one of them is N, preferably X¹ is CR^{c}, and R^{c} is a hydrogen atom;
R⁴ and R⁵ together with the nitrogen atom to which they are attached form a structure of 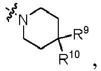 and
R⁹ and R¹⁰ are each independently selected from the group consisting of hydrogen atom, deuterium atom, C₁₋₆ alkyl, amino and -NR^{a}S(O)NR^{a}R^{b}, wherein R^{a} and R^{b} are as defined in claim 1.

10. The compound according to claim 1 or 2, wherein
R¹ is selected from the group consisting of hydrogen atom, deuterium atom, C₁₋₆ alkyl and amino;
Y¹ is -S- or a bond;
ring A is an aryl or heteroaryl;
each R³ is independently selected from the group consisting of hydrogen atom, deuterium atom, halogen, haloC₁₋₆ alkyl, C₁₋₆ alkyl, C₁₋₆ alkoxy, haloC₁₋₆ alkoxy and substituted phenyl;
n is selected from the group consisting of 0, 1, 2, 3, 4 and 5;
X¹, X² and X³ are each independently selected from the group consisting of CR^{c} and N, wherein at least one of them is N, preferably X¹ is CR^{c}, and R^{c} is a hydrogen atom;
R^{6a} and R^{6b} are each independently selected from the group consisting of hydrogen atom, deuterium atom, C₁₋₆ alkyl and C₁₋₆ alkoxy; or R^{6a} and R^{6b} together with the carbon atom to which they are attached form a 3 to 12 membered heterocyclyl or C₃₋₈ cycloalkyl;
p is 1 or 2;
R^{7a} and R^{7b} are each independently selected from the group consisting of hydrogen atom, deuterium atom, amino, C₁₋₆ alkyl and -NR^{a}S(O)NR^{a}R^{b}, wherein R^{a} and R^{b} are as defined in claim 1;
q is 1 or 2;
W is absent;
ring B is absent, Y² is CR^{2a}R^{2b} or O, Y³ is CR^{3a}R3^{b}; and
R^{2a}, R^{2b}, R^{3a} and R^{3b} are each independently selected from the group consisting of hydrogen atom, deuterium atom and C₁₋₆ alkyl.

11. The compound according to any one of claims 1 to 3, 5, 6 and 8, wherein
R¹ is selected from the group consisting of hydrogen atom, deuterium atom, C₁₋₆ alkyl and amino;
Y¹ is -S- or a bond;
ring A is an aryl or heteroaryl;
each R³ is independently selected from the group consisting of hydrogen atom, deuterium atom, halogen, haloC₁₋₆ alkyl, C₁₋₆ alkyl, C₁₋₆ alkoxy, haloC₁₋₆ alkoxy and substituted phenyl;
n is selected from the group consisting of 0, 1, 2, 3, 4 and 5;
X¹, X² and X³ are each independently selected from the group consisting of CR^{c} and N, wherein at least one of them is N, preferably X¹ is CR^{c}, and R^{c} is a hydrogen atom;
R^{6a} and R^{6b} are each independently selected from the group consisting of hydrogen atom, deuterium atom, C₁₋₆ alkyl and C₁₋₆ alkoxy;
p is 1 or 2;
R^{7a} and R^{7b} are each independently selected from the group consisting of hydrogen atom, deuterium atom, amino, C₁₋₆ alkyl and -NR^{a}S(O)NR^{a}R^{b}, wherein R^{a} and R^{b} are as defined in claim 1;
q is 1 or 2;
W is absent;
ring B is selected from the group consisting of phenyl, 5 membered heteroaryl and 6 membered heteroaryl;
Y² is C and Y³ is C, 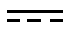 is a double bond;
each R⁸ is independently selected from the group consisting of hydrogen atom, deuterium atom, halogen, amino, hydroxy, cyano, nitro, carboxy, C₁₋₆ alkyl and C₁₋₆ alkoxy; and
m is selected from the group consisting of 0, 1, 2, 3 and 4.

12. The compound according to claim 1 or 2, wherein R⁴ and R⁵ together with the nitrogen atom to which they are attached form a structure of 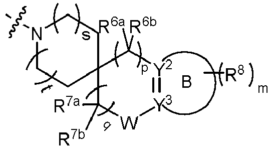
R¹ is selected from the group consisting of hydrogen atom, C₁₋₆ alkyl and amino;
Y¹ is -S- or a bond;
ring A is an aryl or heteroaryl, preferably phenyl or pyridyl;
each R³ is independently selected from the group consisting of hydrogen atom, deuterium atom, halogen, cyano, amino, haloC₁₋₆ alkyl, C₁₋₆ alkyl, C₁₋₆ alkoxy, haloC₁₋₆ alkoxy, C₁₋₆ alkylamino, haloC₁₋₆ alkylamino, C₃₋₈ cycloalkyl, 3 to 12 membered heterocyclyl, -OR^{a}, -CHR^{a}R^{b} and -NR^{a}R;
R^{a} and R^{b} are each independently selected from the group consisting of hydrogen, deuterium atom, hydroxy, C₁₋₆ alkyl and C₃₋₈ cycloalkyl, wherein the alkyl, heterocyclyl and cycloalkyl are each independently optionally further substituted by one or more substituents selected from the group consisting of halogen, deuterium atom, cyano, amino and hydroxy;
n is selected from the group consisting of 0, 1, 2, 3, 4 and 5;
X³ is N, X¹ and X² are each independently CR^{c}, and R^{c} is a hydrogen atom;
s and t are each independently selected from the group consisting of 0 and 1;
R^{6a} and R^{6b} are each independently selected from the group consisting of hydrogen atom, deuterium atom, C₁₋₆ alkyl and C₁₋₆ alkoxy;
p is 1;
R^{7a} and R^{7b} are each independently selected from the group consisting of hydrogen atom, deuterium atom, amino and C₁₋₆ alkyl;
q is 1;
W is absent;
ring B is selected from the group consisting of benzene ring, 5 membered heteroaromatic ring and 6 membered heteroaromatic ring, preferably a benzene ring or pyridine ring;
Y² is C and Y³ is C;
each R⁸ is independently selected from the group consisting of hydrogen atom, deuterium atom, halogen, amino, hydroxy, cyano, nitro, carboxy, C₁₋₆ alkyl and C₁₋₆ alkoxy; and
m is selected from the group consisting of 0, 1, 2, 3 and 4.

13. The compound according to any one of claims 1 to 3, 5 to 8 and 12, being a compound of formula (II): 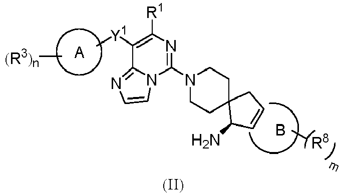 or a tautomer, mesomer, racemate, enantiomer, diastereomer, atropisomer thereof, or mixture thereof, or a pharmaceutically acceptable salt thereof,
wherein:
R¹ is selected from the group consisting of hydrogen atom, C₁₋₆ alkyl, haloalkyl and amino;
Y¹ is -S- or a bond;
ring A is an aryl or heteroaryl, preferably phenyl or pyridyl;
each R³ is independently selected from the group consisting of hydrogen atom, deuterium atom, halogen, cyano, amino, C₁₋₆ alkyl, C₁₋₆ alkoxy, haloC₁₋₆ alkyl, haloC₁₋₆ alkoxy, C₃₋₈ cycloalkyl, 3 to 12 membered heterocyclyl, -OR^{a}, -CHR^{a}R^{b} and -NR^{a}R^{b};
R^{a} and R^{b} are each independently selected from the group consisting of hydrogen, deuterium atom, hydroxy, C₁₋₆ alkyl and C₃₋₈ cycloalkyl, wherein the alkyl, heterocyclyl and cycloalkyl are each independently optionally further substituted by one or more substituents selected from the group consisting of halogen, deuterium atom, cyano, amino and hydroxy;
ring B is selected from the group consisting of benzene ring, 5 membered heteroaromatic ring and 6 membered heteroaromatic ring, preferably a benzene ring or pyridine ring;
each R⁸ is independently selected from the group consisting of hydrogen atom, deuterium atom, halogen, cyano, C₁₋₆ alkyl and C₁₋₆ alkoxy;
m is selected from the group consisting of 0, 1, 2, 3 and 4; and
n is selected from the group consisting of 1, 2, 3 and 4.

14. The compound according to any one of claims 1 to 13, being

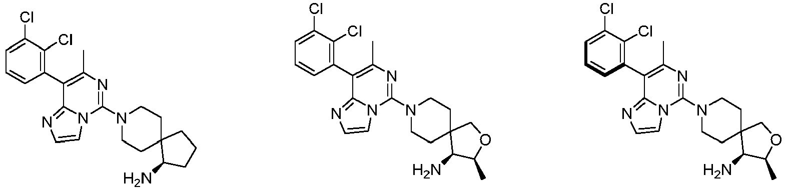

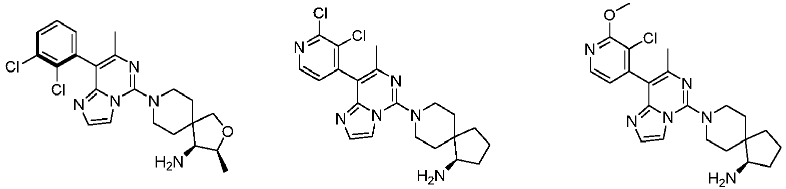

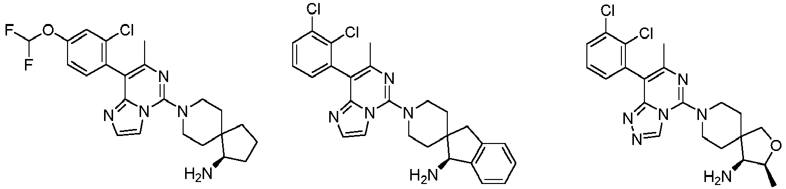

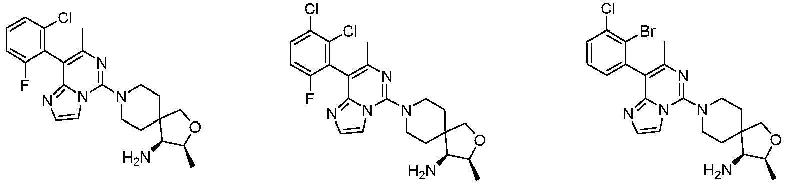

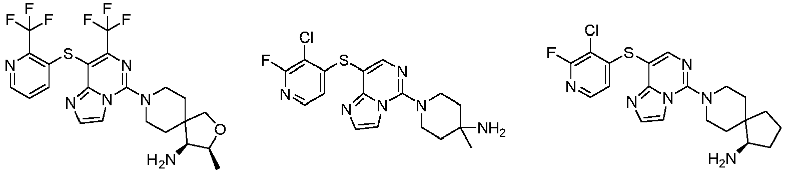

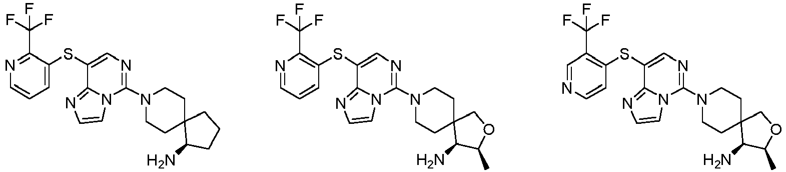

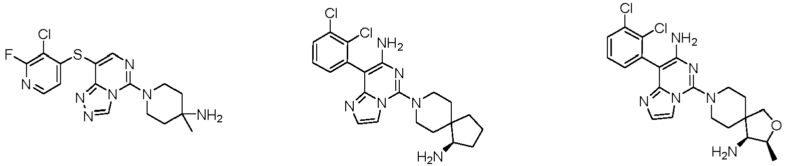

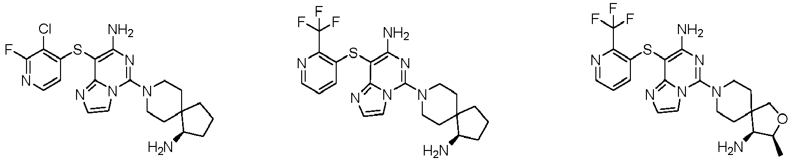

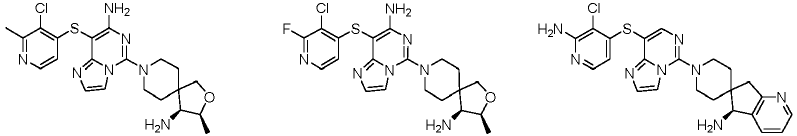

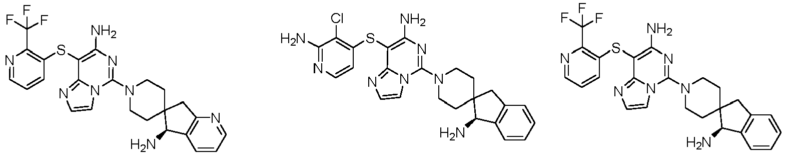

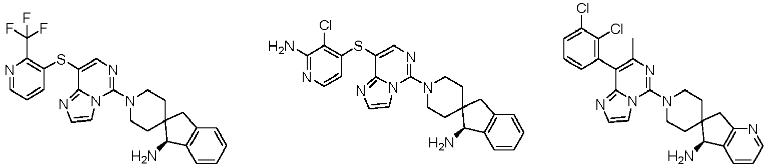

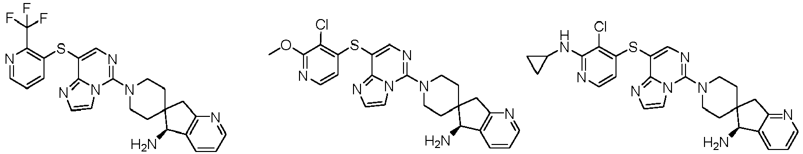

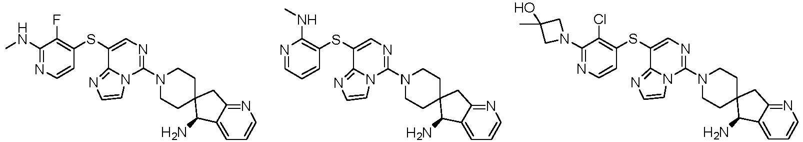

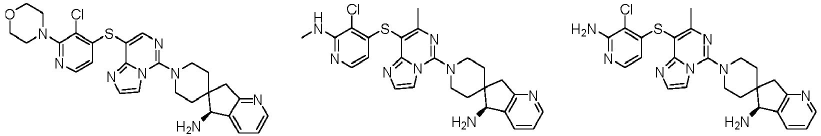

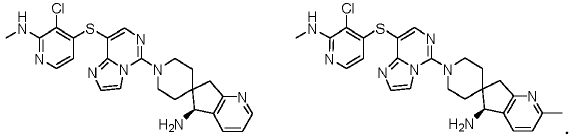

15. A method for preparing the compound according to any one of claims 1 to 12 and 14, wherein the compound of formula (I) is a compound of formula (I-A) or a compound of formula (I-B), wherein 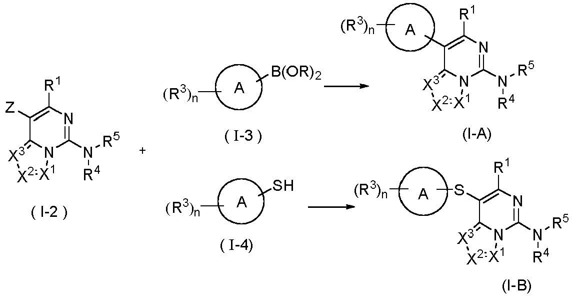
subjecting a compound of formula (I-2) and a compound of formula (I-3) to a Suzuki coupling reaction under an alkaline condition in the presence of a catalyst to obtain the compound of formula (I-A), wherein the catalyst is selected from the group consisting of palladium on carbon, Raney nickel, tetrakis(triphenylphosphine)palladium, palladium dichloride, palladium acetate, [1,1'-bis(diphenylphosphino)ferrocene]palladium (II) dichloride, 1,1'-bis(dibenzylphosphino)dichloroferrocene palladium (II), tris(dibenzylideneacetone)dipalladium and 2-dicyclohexylphosphino-2',6'-dimethoxybiphenyl, and preferably [1,1'-bis(diphenylphosphino)ferrocene]palladium (II) dichloride and 2-dicyclohexylphosphino-2',6'-dimethoxybiphenyl; or
subjecting a compound of formula (I-2) and a compound of formula (I-4) to a C-S coupling reaction under an alkaline condition to obtain the compound of formula (I-B);
wherein the reagent that provides an alkaline condition includes organic bases and inorganic bases; the organic base is selected from the group consisting of triethylamine, *N,N*-diisopropylethylamine, n-butyllithium, lithium diisopropylamide, lithium bistrimethylsilylamide, potassium acetate, sodium *tert*-butoxide and potassium *tert*-butoxide; the inorganic base is selected from the group consisting of sodium hydride, potassium phosphate, sodium carbonate, potassium carbonate, potassium acetate, cesium carbonate, sodium hydroxide and lithium hydroxide;
B(OR)₂ is a borate or boric acid;
Z is a halogen or sulfonyl; and
ring A, R¹, X¹, X², X³, R³, R⁴, R⁵ and n are as defined in claim 1.

16. A method for preparing the compound according to claim 13, wherein the compound of formula (II) is a compound of formula (II-A) or a compound of formula (II-B), comprising the following steps of: 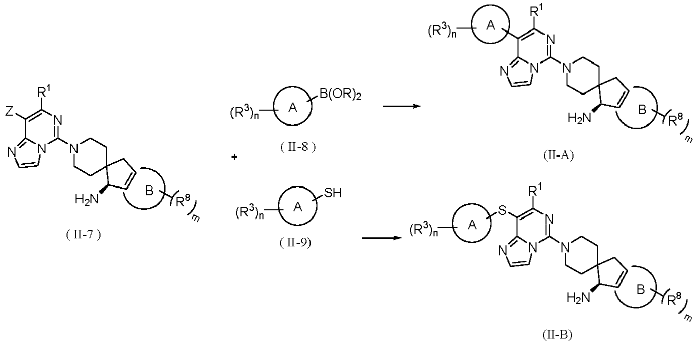
subjecting a compound of formula (II-7) and a compound of formula (II-8) to a Suzuki coupling reaction under an alkaline condition in the presence of a catalyst to obtain the compound of formula (II-A);
or subjecting a compound of formula (II-7) and a compound of formula (II-9) to a C-S coupling reaction under an alkaline condition to obtain the compound of formula (II-B);
wherein the catalyst is selected from the group consisting of palladium on carbon, Raney nickel, tetrakis(triphenylphosphine)palladium, palladium dichloride, palladium acetate, [1,1'-bis(diphenylphosphino)ferrocene]palladium (II) dichloride, 1,1'-bis(dibenzylphosphino)dichloroferrocene palladium (II), tris(dibenzylideneacetone)dipalladium and 2-dicyclohexylphosphino-2',6'-dimethoxybiphenyl, and preferably [1,1'-bis(diphenylphosphino)ferrocene]palladium (II) dichloride and 2-dicyclohexylphosphino-2',6'-dimethoxybiphenyl;
the reagent that provides an alkaline condition includes organic bases and inorganic bases; the organic base is selected from the group consisting of triethylamine, *N,N*-diisopropylethylamine, n-butyllithium, lithium diisopropylamide, lithium bistrimethylsilylamide, potassium acetate, sodium *tert*-butoxide and potassium *tert*-butoxide; the inorganic base is selected from the group consisting of sodium hydride, potassium phosphate, sodium carbonate, potassium carbonate, potassium acetate, cesium carbonate, sodium hydroxide and lithium hydroxide;
B(OR)₂ is a borate or boric acid;
Z is selected from the group consisting of halogen, sulfonyl and sulfinyl;
ring A, ring B, R¹, R³, R⁸, B, m and n are as defined in claim 13.

17. The method according to claim 16, further comprising a step of reacting a compound of formula (II-5) with a compound of formula (II-6) under an alkaline condition to obtain the compound of formula (II-7), 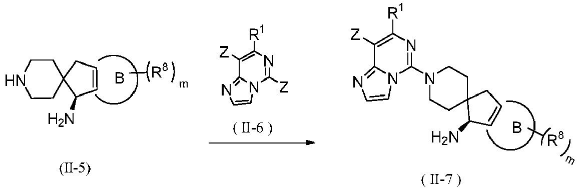 wherein the reagent that provides an alkaline condition includes organic bases and inorganic bases; the organic base is selected from the group consisting of triethylamine, *N,N*-diisopropylethylamine, n-butyllithium, lithium diisopropylamide, lithium bistrimethylsilylamide, potassium acetate, sodium *tert*-butoxide and potassium *tert*-butoxide; the inorganic base is selected from the group consisting of sodium hydride, potassium phosphate, sodium carbonate, potassium carbonate, potassium acetate, cesium carbonate, sodium hydroxide and lithium hydroxide.

18. A compound of formula (I-2) or a pharmaceutically acceptable salt thereof, 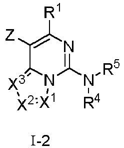
wherein R¹, X¹, X², X³, R⁴ and R⁵ are as defined in claim 1;
Z is a halogen or sulfonyl.

19. A compound of formula (I-1) or a pharmaceutically acceptable salt thereof 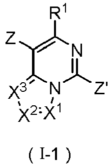
wherein R¹, X¹, X² and X³ are as defined in claim 1;
Z and Z' are each independently selected from the group consisting of halogen and sulfonyl.

20. A method for preparing the compound of formula (I) from the compound of formula (I-2) or the pharmaceutically acceptable salt thereof or the compound of formula (I-1) or the pharmaceutically acceptable salt thereof.

21. A pharmaceutical composition, comprising the compound or the tautomer, mesomer, racemate, enantiomer, diastereomer, atropisomer thereof, or mixture thereof, or the pharmaceutically acceptable salt thereof according to any one of claims 1 to 14, and one or more pharmaceutically acceptable carrier, diluent or excipient.

22. Use of the compound or the tautomer, mesomer, racemate, enantiomer, diastereomer, atropisomer thereof, or mixture thereof, or the pharmaceutically acceptable salt thereof according to any one of claims 1 to 14, or the pharmaceutical composition according to claim 21 in the preparation of a medicament for preventing or treating a disease or condition mediated by SHP2 activity.

23. Use of the compound or the tautomer, mesomer, racemate, enantiomer, diastereomer, atropisomer thereof, or mixture thereof, or the pharmaceutically acceptable salt thereof according to any one of claims 1 to 14, or the pharmaceutical composition according to claim 21 as a SHP2 inhibitor in the preparation of a medicament for preventing and/or treating tumor or cancer.

24. Use of the compound or the tautomer, mesomer, racemate, enantiomer, diastereomer, atropisomer thereof, or mixture thereof, or the pharmaceutically acceptable salt thereof according to any one of claims 1 to 14, or the pharmaceutical composition according to claim 21 in the preparation of a medicament for preventing or treating Noonan syndrome, Leopard syndrome, juvenile myelomonocytic leukemia, neuroblastoma, melanoma, acute myelogenous leukemia, breast cancer, esophageal cancer, lung cancer, colon cancer, head cancer, pancreatic cancer, head and neck squamous cell carcinoma, stomach cancer, liver cancer, anaplastic large cell lymphoma or glioblastoma.
